# EUROPÄISCHE PATENTSCHRIFT

(11) **EP 3 577 101 B1**
(45) Veröffentlichungstag und Bekanntmachung des Hinweises auf die Patenterteilung: **03.03.2021**
(21) Anmeldenummer: 18701493.1
(22) Anmeldetag: 30.01.2018
(51) Int. Cl.: C07C 211/61, C07D 405/04, C07D 405/12, C07D 407/12, C07D 471/04, C07D 307/91, C09K 11/06, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 02.02.2017 EP 17154481
(43) Veröffentlichungstag der Anmeldung: 11.12.2019
(73) Patentinhaber: Merck Patent GmbH, 64293 Darmstadt (DE)
(72) Erfinder: STOESSEL, Philipp, 60389 Frankfurt am Main (DE); JOOSTEN, Dominik, 60487 Frankfurt am Main (DE); MUJICA-FERNAUD, Teresa, 64283 Darmstadt (DE)
(86) Internationale Anmeldenummer: PCT/EP2018/052196
(87) Internationale Veröffentlichungsnummer: WO 2018/141706

(56) Entgegenhaltungen:
- WO-A1-2015/086108
- WO-A1-2016/150544
- DE-A1-102006 025 846

## Beschreibung

Die vorliegende Anmeldung betrifft Propellan-Verbindungen gemäß den weiter unten definierten Formeln, die sich zur Verwendung in elektronischen Vorrichtungen eignen. Weiterhin betrifft die vorliegende Anmeldung Verfahren zur Herstellung der genannten Verbindungen, sowie elektronische Vorrichtungen enthaltend die genannten Verbindungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Emissionsschichten, Schichten mit lochtransportierender Funktion, und Schichten mit elektronentransportierender Funktion. Zur Verwendung in diesen Schichten werden weiterhin neue Materialien, insbesondere Materialien mit lochtransportierenden und elektronentransportierenden Eigenschaften, gesucht.

Im Stand der Technik sind insbesondere Triarylamin-Verbindungen als lochtransportierende Materialien für elektronische Vorrichtungen bekannt. Es sind Triarylamin-Verbindungen mit einer Vielzahl unterschiedlicher Arten von Arylgruppen als Substituenten am Stickstoffatom des Triarylamins bekannt, beispielsweise Phenanthrenylgruppen, Spirobifluorenyl-Gruppen und Fluorenyl-Gruppen. Es besteht jedoch weiterhin Bedarf an alternativen Triarylamin-Verbindungen mit neuartigen Ausführungsformen von Arylgruppen am Stickstoffatom des Triarylamins, die zur Verwendung in elektronischen Vorrichtungen geeignet sind. Es besteht auch Verbesserungsbedarf bezüglich der Leistungsdaten bei Verwendung der genannten Verbindungen in elektronischen Vorrichtungen, insbesondere bezüglich Lebensdauer und Effizienz.

Im Stand der Technik sind als elektronentransportierende Materialien ebenfalls eine Vielzahl von Stoffklassen bekannt, beispielsweise TriazinDerivate, Pyrimidin-Derivate und Chinolin-Derivate. Auch hier besteht Bedarf an alternativen Grundgerüsten und Stoffklassen. Es besteht auch Verbesserungsbedarf bezüglich der Leistungsdaten bei Verwendung der genannten Verbindungen in elektronischen Vorrichtungen, insbesondere bezüglich Lebensdauer und Effizienz.

WO 2015/086108 A1 offenbart Polyzyklische-Verbindungen zur Verwendung in elektronischen Vorrichtungen.

Nun wurde gefunden, dass sich bestimmte Propellan-Verbindungen der unten näher definierten Formeln (I) bis (IV) hervorragend zur Verwendung in elektronischen Vorrichtungen eignen, insbesondere zur Verwendung in OLEDs, nochmals insbesondere darin zur Verwendung als lochtransportierende oder elektronentransportierende Materialien. Bevorzugt bewirken sie bei Verwendung in OLEDs hervorragende Lebensdauer und Effizienz der Vorrichtung.

Gegenstand der Erfindung ist eine Verbindung einer Formel (I), (II), (III) oder (IV), 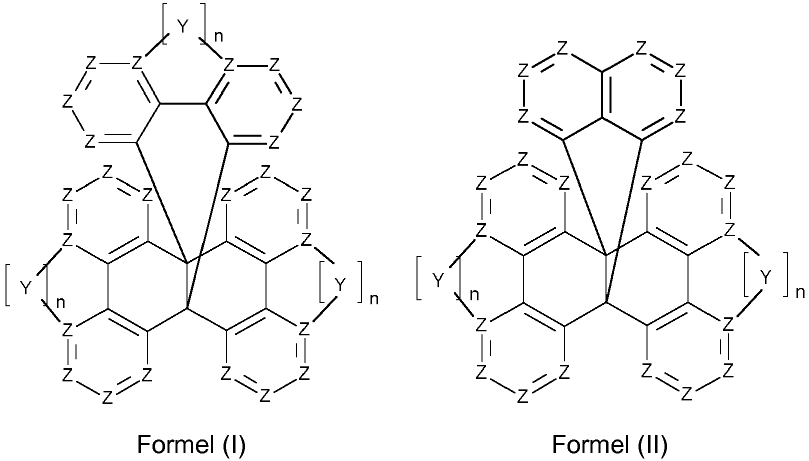 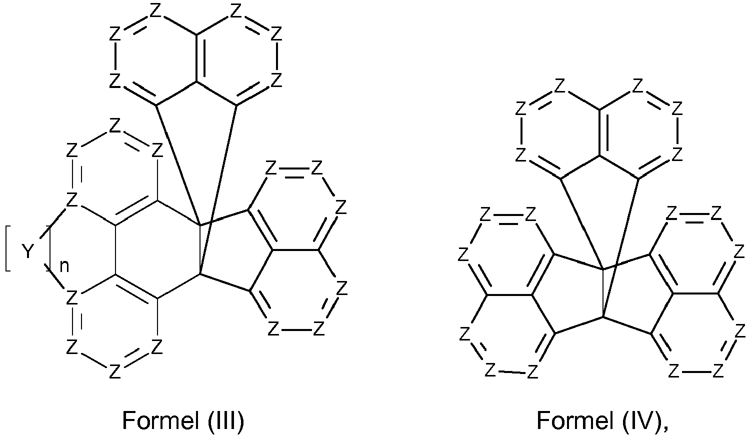 wobei für die auftretenden Variablen gilt:
- Z: ist bei jedem Auftreten gleich oder verschieden gewählt aus N und CR¹ oder C, wobei eine Gruppe Z genau dann gleich C ist, wenn eine Gruppe Y daran gebunden ist;
- Y: ist bei jedem Auftreten gleich oder verschieden gewählt aus BR², C(R²)₂, Si(R²)₂, NR², P(O)R², O, S, SO, SO₂;
- n: ist bei jedem Auftreten gleich oder verschieden 0 oder 1;
- R¹, R²: sind bei jedem Auftreten gleich oder verschieden gewählt aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste R¹ und/oder R² miteinander verknüpft sein können und einen Ring bilden können; wobei die genannten Alkyl-, Alkoxy-, Alkenyl- und Alkinylgruppen und die genannten aromatischen Ringsysteme jeweils mit einem oder mehreren Resten R³ substituiert sein können; wobei an einen oder mehrere der Sechsringe in Formel (I) bis (IV) jeweils ein Indolring ankondensiert sein kann, der wiederum mit Resten R³ substituiert sein kann;
- R³: ist bei jedem Auftreten gleich oder verschieden gewählt aus H, D, F, CN, Si(R⁴)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme jeweils mit einem oder mehreren Resten R⁴ substituiert sein können;
- R⁴: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen und die genannten aromatischen Ringsysteme mit F oder CN substituiert sein können;
dadurch gekennzeichnet, dass mindestens eine Gruppe Z je Formel gewählt aus den Formeln (I), (II), (III) und (IV) gleich CR¹ ist;
und weiterhin dadurch gekennzeichnet, dass genau eine Gruppe R¹ je Formel (I) durch eine Gruppe der Formel (A) oder eine Gruppe der Formel (H) ersetzt ist, 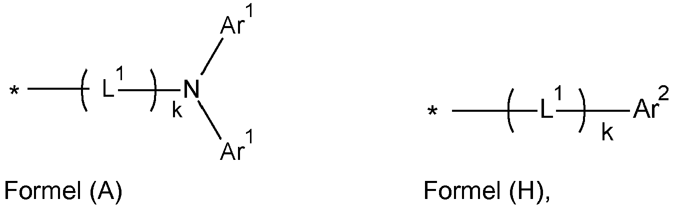 und weiterhin dadurch gekennzeichnet, dass genau eine Gruppe R¹ je Formel gewählt aus den Formeln (II), (III) und (IV) durch eine Gruppe der Formel (A) ersetzt ist,
wobei gilt:
- L¹: ist bei jedem Auftreten gleich oder verschieden ein aromatisches Ringsystem mit 6 bis 24 aromatischen Ringatomen oder ein heteroaromatisches Ringsystem mit 5 bis 24 aromatischen Ringatomen, die jeweils durch einen oder mehrere Reste R⁵ substituiert sein können;
- k: ist gleich 0, 1, 2 oder 3;
- Ar¹: ist bei jedem Auftreten gleich oder verschieden ein aromatisches Ringsystem mit 6 bis 24 aromatischen Ringatomen oder ein heteroaromatisches Ringsystem mit 5 bis 24 aromatischen Ringatomen, das mit einem oder mehreren Resten R⁵ substituiert sein kann;
- Ar²: ist ein heteroaromatisches Ringsystem mit 5 bis 24 aromatischen Ringatomen, das mit einem oder mehreren Resten R⁵ substituiert sein kann;
- R⁵: ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
- R⁶: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
wobei die Gruppe der Formel (A) bzw. die Gruppe der Formel (H) über die mit * markierte Bindung gebunden ist.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Bevorzugt wird unter einem aromatischen Ringsystem eine chemische Gruppe verstanden, in der die darin enthaltenen Arylgruppen miteinander konjugiert sind. Dies bedeutet, dass die enthaltenen Arylgruppen miteinander über Einfachbindungen oder über verbindende Einheiten, die ein freies pi-Elektronenpaar aufweisen, das an der Konjugation teilnehmen kann, verbunden sein müssen. Verbindende Einheiten sind dabei bevorzugt gewählt aus Stickstoffatomen, einzelnen C=C-Einheiten, einzelnen C=C-Einheiten, mehreren miteinander konjugierten C=C-Einheiten oder/oder C=C-Einheiten, -O-, und -S-.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Unter den Formeln (I) bis (IV) ist die Formel (I) bevorzugt.

Bevorzugt sind höchstens drei Gruppen Z pro aromatischem Ring gleich N, und die restlichen Gruppen Z im aromatischen Ring sind gleich CR¹ bzw. C.

Bevorzugt ist Z gleich CR¹ oder C, wobei Z genau dann gleich C ist, wenn eine Gruppe Y daran gebunden ist.

Bevorzugt ist Y bei jedem Auftreten gleich oder verschieden gewählt aus NR², O, S und C(R²)₂. Besonders bevorzugt ist Y bei jedem Auftreten gleich oder verschieden gewählt aus O und NR². Ganz besonders bevorzugt ist Y gleich O.

Bevorzugt ist die Summe der Indices n in einer Verbindung der Formel (I) oder (II) gleich 1 oder 0, besonders bevorzugt gleich 0. Bevorzugt ist die Summe der Indices n in einer Verbindung der Formel (III) gleich 0.

Bevorzugt ist n bei jedem Auftreten gleich 0.

Falls mehrere unterschiedliche Positionen der Gruppe Y möglich sind, so ist die Gruppe Y bevorzugt an denjenigen Sechsring gebunden, der eine Gruppe der Formel (A) oder (H) trägt.

R¹ ist bei jedem Auftreten gleich oder verschieden gewählt aus H, D, F, CN, geradkettigen Alkylgruppen mit 1 bis 12 C-Atomen, verzweigten oder cyclischen Alkylgruppen mit 3 bis 12 C-Atomen, und aromatischen Ringsystemen mit 6 bis 24 aromatischen Ringatomen; wobei zwei oder mehr Reste R¹ miteinander verknüpft sein können und einen Ring bilden können; und wobei die genannten Alkylgruppen und die genannten aromatischen Ringsysteme jeweils mit einem oder mehreren Resten R³ substituiert sein können; wobei an einen der Sechsringe in Formel (I) bis (IV) jeweils ein Indolring ankondensiert sein kann, der wiederum mit Resten R³ substituiert sein kann.

Bevorzugte Beispiele für Verbindungen der Formel (I), die einen an Formel (I) ankondensierten Indolring aufweisen, entsprechen einer der unten gezeigten Formeln (I-In-1) und (I-In-2)

| |
|---|
| 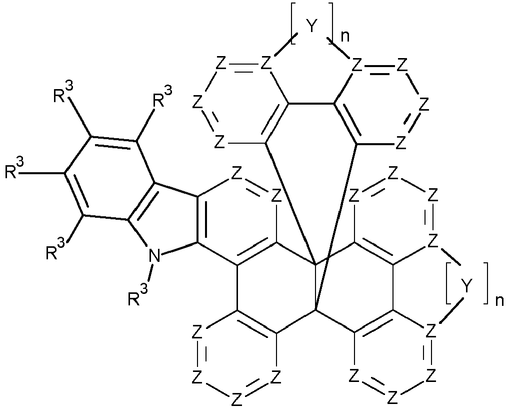 |
| Formel (I-In-1) |
| 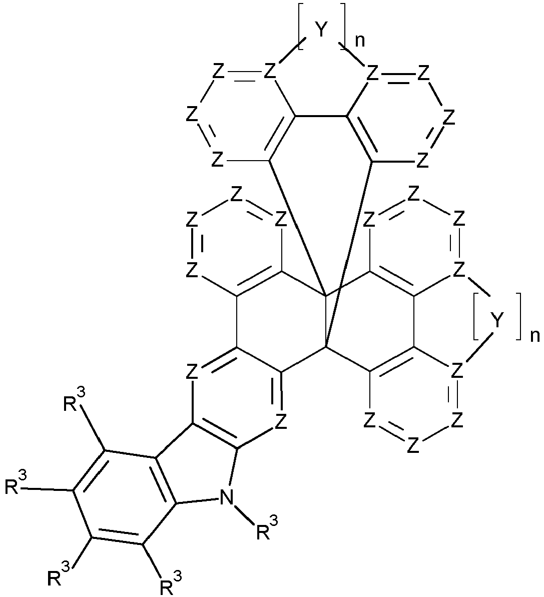 |
| Formel (I-In-2), |

wobei die auftretenden Gruppen und Indices wie für Formel (I) definiert sind, und im Übrigen die in der vorliegenden Anmeldung angegebenen Bevorzugungen gelten.

Besonders bevorzugt ist R¹ bei jedem Auftreten gleich oder verschieden gewählt aus H, D, geradkettigen oder verzweigten Alkylgruppen mit bis zu 12 C-Atomen, und aromatischen Ringsystemen mit 6 bis 24 aromatischen Ringatomen, wobei die genannten Alkylgruppen und die genannten aromatischen Ringsysteme jeweils mit einem oder mehreren Resten R³ substituiert sein können. Ganz besonders bevorzugt ist R¹ gleich H.

R² ist bei jedem Auftreten gleich oder verschieden gewählt aus F, geradkettigen Alkylgruppen mit 1 bis 12 C-Atomen, verzweigten oder cyclischen Alkylgruppen mit 3 bis 12 C-Atomen, und aromatischen Ringsystemen mit 6 bis 24 aromatischen Ringatomen; wobei zwei Reste R² miteinander verknüpft sein können und einen Ring bilden können; und wobei die genannten Alkylgruppen und die genannten aromatischen Ringsysteme jeweils mit einem oder mehreren Resten R³ substituiert sein können. Besonders bevorzugt ist R² gewählt aus geradkettigen oder verzweigten Alkylgruppen mit bis zu 12 C-Atomen, und aromatischen Ringsystemen mit 6 bis 24 aromatischen Ringatomen, wobei zwei Reste R², die an dasselbe Kohlenstoffatom einer Gruppe C(R²)₂ binden, und die Alkylgruppen oder Arylgruppen sind, miteinander zu einer cyclischen Alkylgruppe oder zu einer Fluoren-Gruppe verbunden sein können. Bevorzugt sind dabei cyclische Alkylgruppen mit 5 bis 6 Kohlenstoffatomen.

Bevorzugt ist R³ bei jedem Auftreten gleich oder verschieden gewählt aus H, D, F, CN, geradkettigen Alkylgruppen mit 1 bis 12 C-Atomen, verzweigten oder cyclischen Alkylgruppen mit 3 bis 12 C-Atomen, und aromatischen Ringsystemen mit 6 bis 24 aromatischen Ringatomen; wobei zwei oder mehr Reste R³ miteinander verknüpft sein können und einen Ring bilden können; und wobei die genannten Alkylgruppen und die genannten aromatischen Ringsysteme jeweils mit einem oder mehreren Resten R⁴ substituiert sein können.

Bevorzugt ist R⁴ bei jedem Auftreten gleich oder verschieden gewählt aus H, D, F, CN, geradkettigen Alkylgruppen mit 1 bis 12 C-Atomen, verzweigten oder cyclischen Alkylgruppen mit 3 bis 12 C-Atomen, und aromatischen Ringsystemen mit 6 bis 24 aromatischen Ringatomen; wobei zwei oder mehr Reste R⁴ miteinander verknüpft sein können und einen Ring bilden können; und wobei die genannten Alkylgruppen und die genannten aromatischen Ringsysteme mit F oder CN substituiert sein können.

Bevorzugt ist genau eine Gruppe R¹ je Formel (I) durch eine Gruppe der Formel (A) ersetzt.

In Formel (I) ist die Gruppe der Formel (H) oder (A) bevorzugt in einer der im Folgenden mit 1, 2, und 4 bezeichneten Positionen gebunden, besonders bevorzugt in einer der im Folgenden mit 3 und 4 bezeichneten Positionen, ganz besonders bevorzugt in der im Folgenden mit 4 bezeichneten Position.

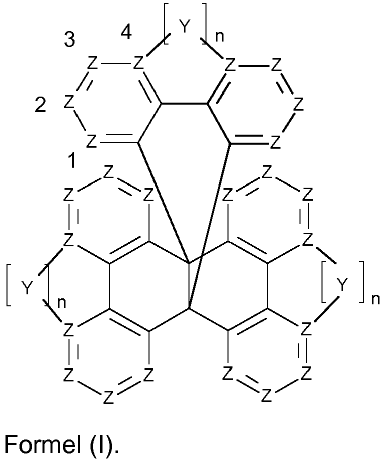

In Formeln (II), (III) und (IV) ist die Gruppe der Formel (A) bevorzugt in der im Folgenden mit "3" bezeichneten Position gebunden.

| | |
|---|---|
| 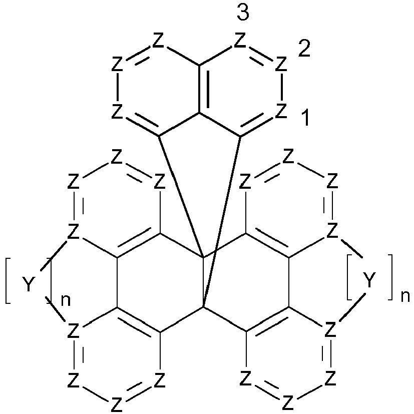 | 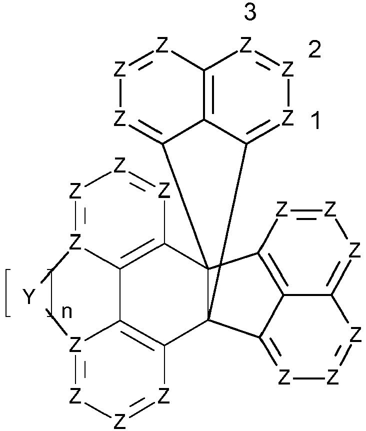 |
| Formel (II) | Formel (III) |
| 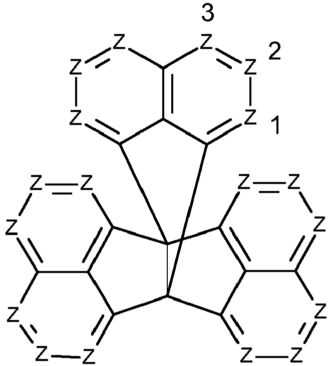 | |
| Formel (IV) | |

In Gruppen der Formel (A) ist k bevorzugt gleich 0 oder 1, besonders bevorzugt gleich 0. L¹ ist bevorzugt ein aromatisches Ringsystem mit 6 bis 24 aromatischen Ringatomen, das mit einem oder mehreren Resten R⁵ substituiert sein kann. Besonders bevorzugt ist L¹ eine divalente Gruppe gewählt aus Phenylen, Biphenylen, Terphenylen, Naphthylen, Dibenzofuran, Dibenzothiophen, Carbazol, und Fluoren, wobei die divalente Gruppe mit einem oder mehreren Resten R⁵ substituiert sein kann. Ar¹ ist bevorzugt bei jedem Auftreten gleich oder verschieden gewählt aus Phenyl, Biphenyl, Terphenyl, Fluorenyl, Naphthyl, Spirobifluorenyl, Pyridyl, Pyrimidyl, Triazinyl, Dibenzofuranyl, benzo-kondensiertem Dibenzofuranyl, Dibenzothiophenyl, benzo-kondensiertem Dibenzothiophenyl, Carbazolyl, und benzo-kondensiertem Carbazolyl, und Kombinationen aus zwei, drei oder vier dieser Gruppen, wobei die genannten Gruppen jeweils mit einem oder mehreren Resten R⁵ substituiert sein können.

In Gruppen der Formel (H) ist k bevorzugt gleich 0 oder 1. L¹ ist bevorzugt ein aromatisches Ringsystem mit 6 bis 24 aromatischen Ringatomen, das mit einem oder mehreren Resten R⁵ substituiert sein kann. Besonders bevorzugt ist L¹ eine divalente Gruppe gewählt aus Phenylen, Biphenylen, Terphenylen, Naphthylen, Dibenzofuran, Dibenzothiophen, Carbazol, und Fluoren, wobei die divalente Gruppe mit einem oder mehreren Resten R⁵ substituiert sein kann. Ar² ist bevorzugt eine Heteroarylgruppe mit 6 bis 24 aromatischen Ringatomen, die mit einem oder mehreren Resten R⁵ substituiert sein kann.

Besonders bevorzugt ist Ar² bei jedem Auftreten gleich oder verschieden gewählt aus Gruppen der folgenden Formeln

| | |
|---|---|
| 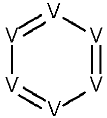 | 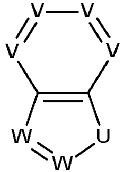 |
| (Ar²-A) | (Ar²-B) |
| 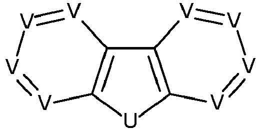 | 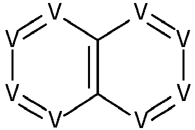 |
| (Ar²-C) | (Ar²-D) |

wobei die auftretenden Variablen wie folgt definiert sind:
- V: ist bei jedem Auftreten gleich oder verschieden N oder CR⁵, wobei in Formel (Ar²-A) und (Ar²-D) mindestens eine Gruppe V je Formel gleich N ist;
- W: ist bei jedem Auftreten gleich oder verschieden N oder CR⁵;
- U: ist gleich NR⁵;
wobei eine Gruppe R⁵ je Formel durch die Bindung an die Gruppe L¹ bzw. die Propellangruppe ersetzt ist.

Ar² ist besonders bevorzugt gewählt aus Pyridin, Pyrimidin, Pyridazin, Pyrazin, Triazin, Carbazol, Imidazol, Pyrazol, Triazol, Benzimidazol, Benzimidazoisochinolin, Imidazophenanthridin, Benzimidazophenanthridin Bathocuproin, Benzimidazobenzimidazol, Chinolin, Chinazolin, Phenanthrolin, Phenanthridin, Diazaphenanthren, und Acridin, die jeweils mit einem oder mehreren Resten R⁵ substituiert sein können.

Gruppen der Formel (A) sind bevorzugt gewählt aus den folgenden Gruppen:

| | |
|---|---|
| 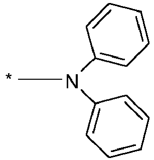 | 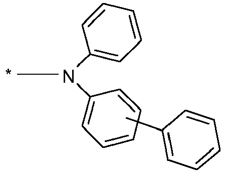 |
| Formel (A-1) | Formel (A-2) |
| 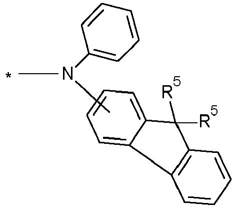 | 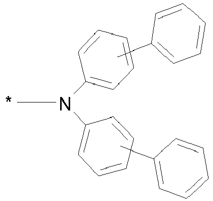 |
| Formel (A-3) | Formel (A-4) |
| 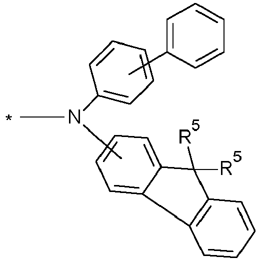 | 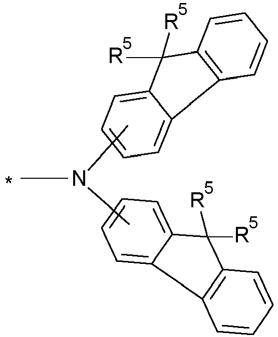 |
| Formel (A-5) | Formel (A-6) |
| 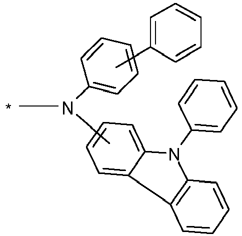 | 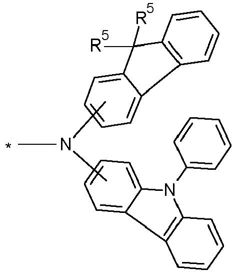 |
| Formel (A-7) | Formel (A-8) |
| 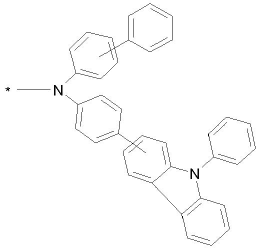 | 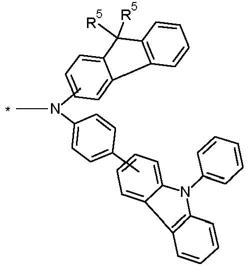 |
| Formel (A-9) | Formel (A-10) |
| 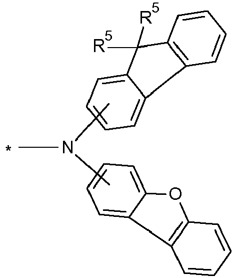 | 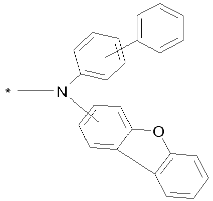 |
| Formel (A-11) | Formel (A-12) |
| 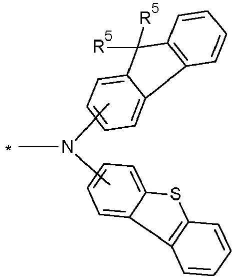 | 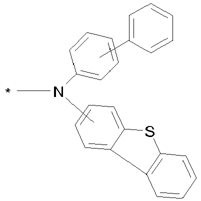 |
| Formel (A-13) | Formel (A-14) |
| 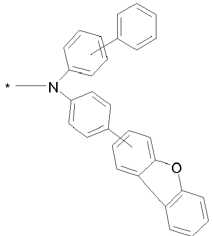 | 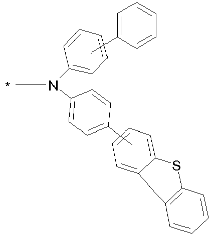 |
| Formel (A-15) | Formel (A-16) |
| 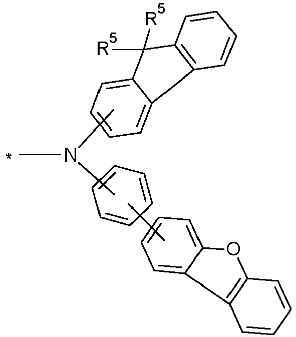 | 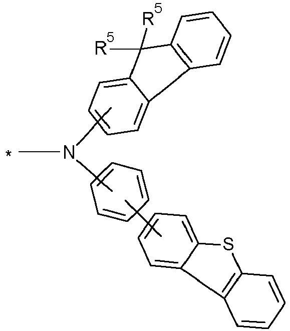 |
| Formel (A-17) | Formel (A-18) |
| 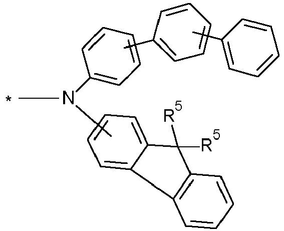 | 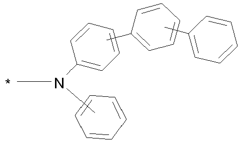 |
| Formel (A-19) | Formel (A-20) |
| 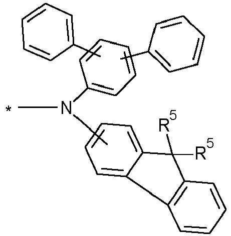 | 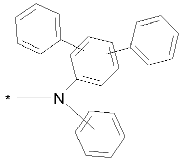 |
| Formel (A-21) | Formel (A-22) |
| 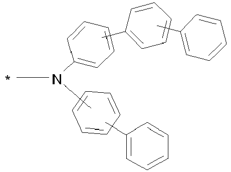 | 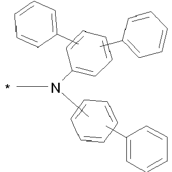 |
| Formel (A-23) | Formel (A-24) |
| 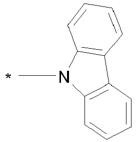 | 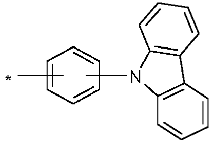 |
| Formel (A-25) | Formel (A-26) |
| 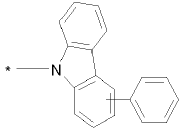 | 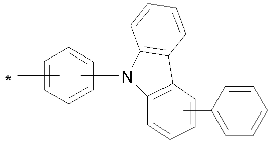 |
| Formel (A-27) | Formel (A-28) |
| 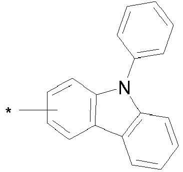 | 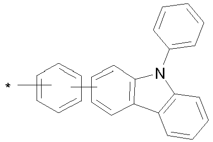 |
| Formel (A-29) | Formel (A-30) |
| 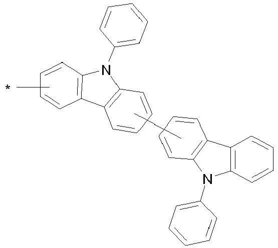 | 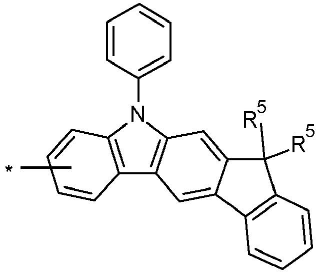 |
| Formel (A-31) | Formel (A-32) |
| 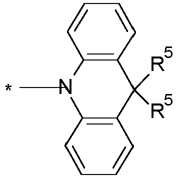 | 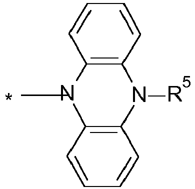 |
| Formel (A-33) | Formel (A-34) |
| 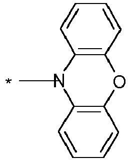 | 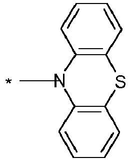 |
| Formel (A-35) | Formel (A-36) |
| 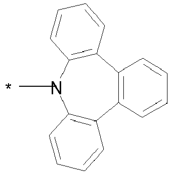 | 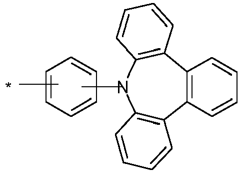 |
| Formel (A-37) | Formel (A-38) |
| 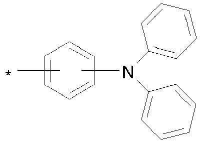 | 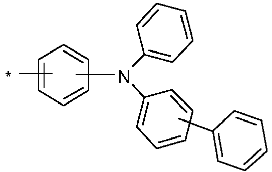 |
| Formel (A-39) | Formel (A-40) |
| 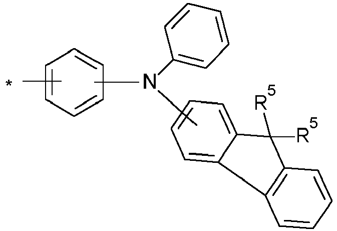 | 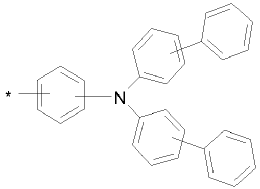 |
| Formel (A-41) | Formel (A-42) |
| 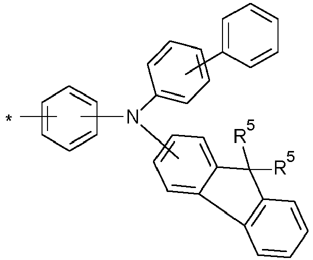 | 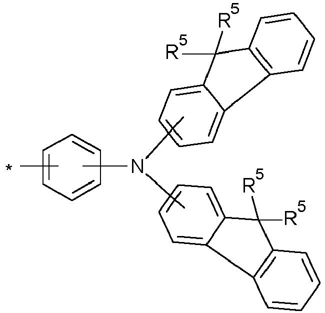 |
| Formel (A-43) | Formel (A-44) |
| 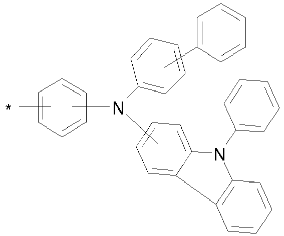 | 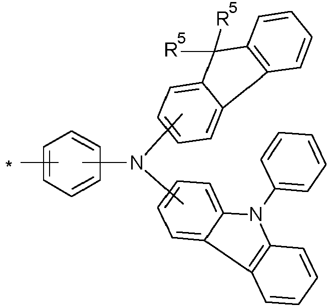 |
| Formel (A-45) | Formel (A-46) |
| 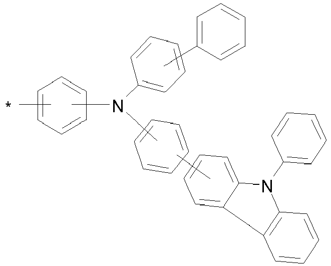 | 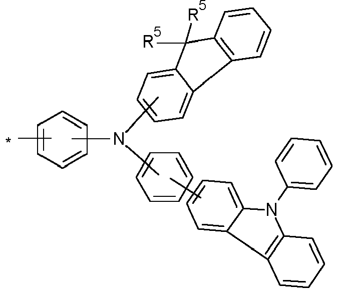 |
| Formel (A-47) | Formel (A-48) |
| 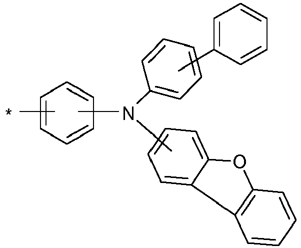 | 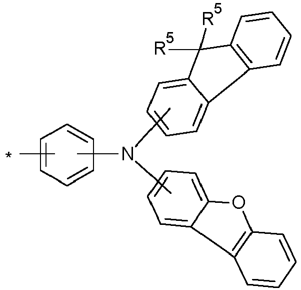 |
| Formel (A-49) | Formel (A-50) |
| 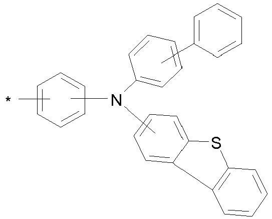 | 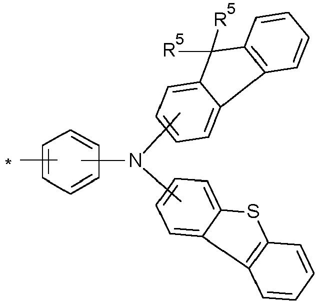 |
| Formel (A-51) | Formel (A-52) |
| 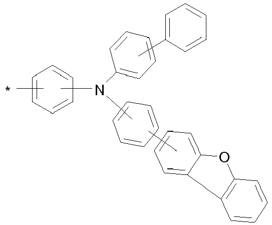 | 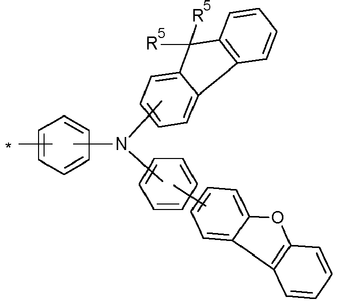 |
| Formel (A-53) | Formel (A-54) |
| 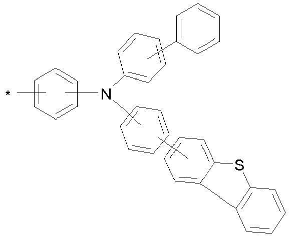 | 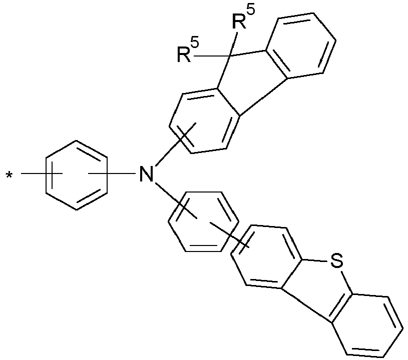 |
| Formel (A-55) | Formel (A-56) |
| 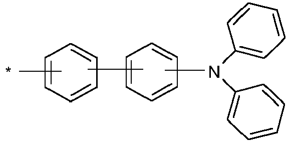 | 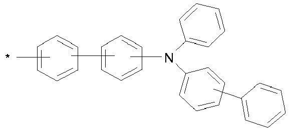 |
| Formel (A-57) | Formel (A-58) |
| 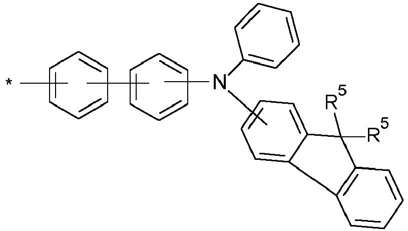 | 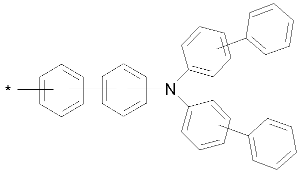 |
| Formel (A-59) | Formel (A-60) |
| 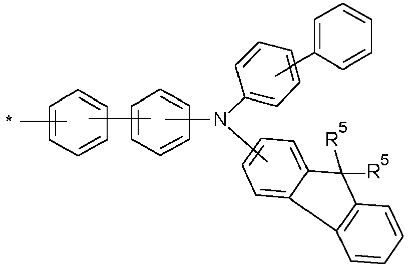 | 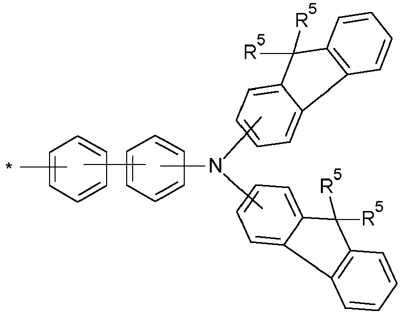 |
| Formel (A-61) | Formel (A-62) |
| 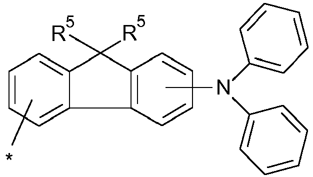 | 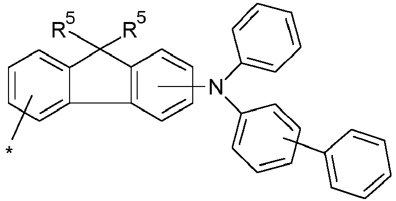 |
| Formel (A-63) | Formel (A-64) |
| 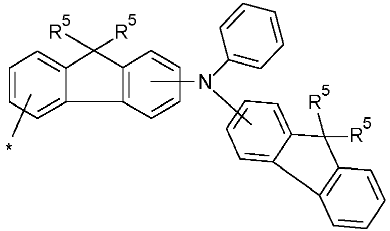 | 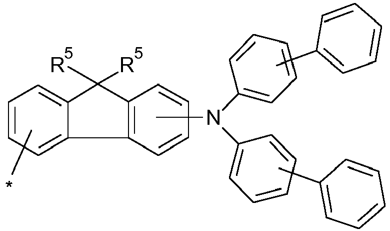 |
| Formel (A-65) | Formel (A-66) |
| 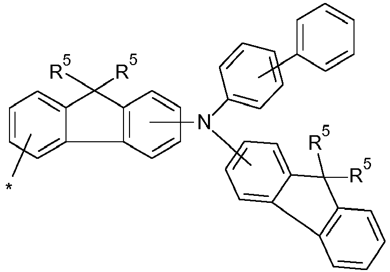 | 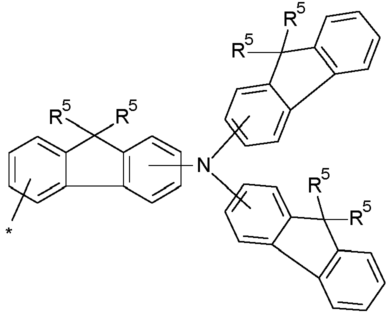 |
| Formel (A-67) | Formel (A-68) |

wobei die Gruppen über die mit * gekennzeichnete Bindung an den Rest der Formel gebunden sind, wobei R⁵ definiert ist wie oben, und bevorzugt den oben genannten bevorzugten Ausführungsformen entspricht, und wobei die Gruppen an den unsubstituiert dargestellten Positionen jeweils mit einem Rest R⁵ substituiert sein können, jedoch bevorzugt in diesen Positionen nicht substituiert sind.

Gruppen der Formel (H) sind bevorzugt gewählt aus den folgenden Gruppen:

| | |
|---|---|
| 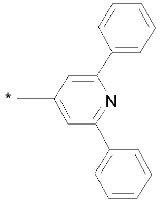 | 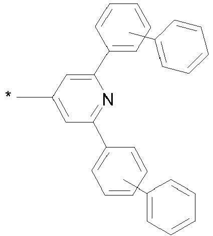 |
| Formel (H-1) | Formel (H-2) |
| 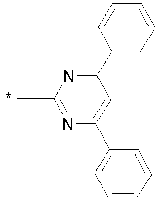 | 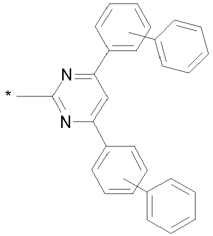 |
| Formel (H-3) | Formel (H-4) |
| 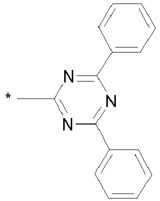 | 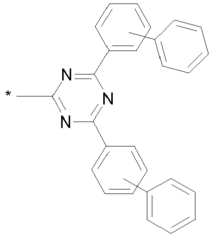 |
| Formel (H-5) | Formel (H-6) |
| 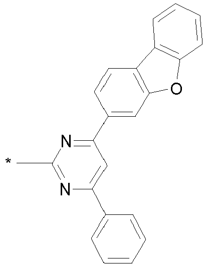 | 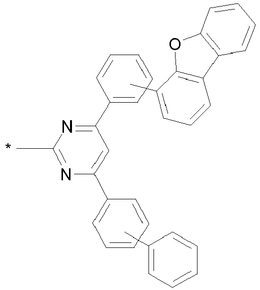 |
| Formel (H-7) | Formel (H-8) |
| 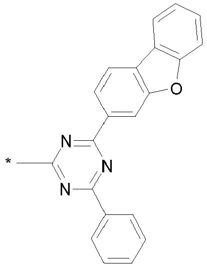 | 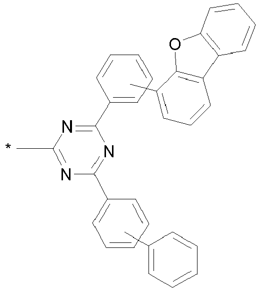 |
| Formel (H-9) | Formel (H-10) |
| 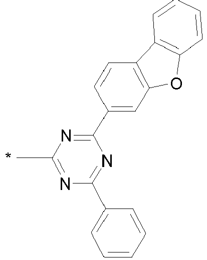 | 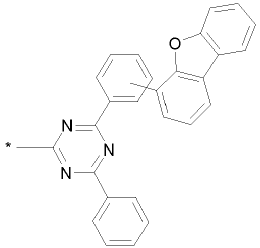 |
| Formel (H-11) | Formel (H-12) |
| 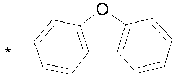 | 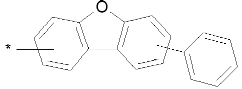 |
| Formel (H-13) | Formel (H-14) |
| 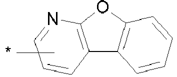 | 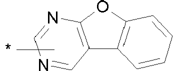 |
| Formel (H-15) | Formel (H-16) |
| 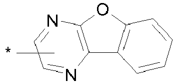 | 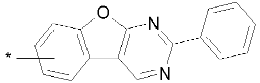 |
| Formel (H-17) | Formel (H-18) |
| 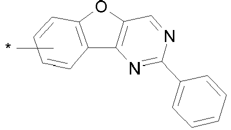 | 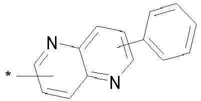 |
| Formel (H-19) | Formel (H-20) |
| 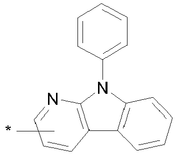 | 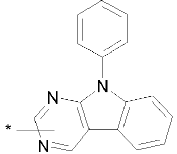 |
| Formel (H-21) | Formel (H-22) |
| 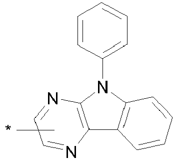 | 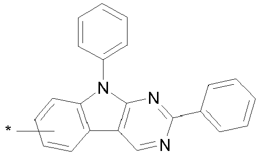 |
| Formel (H-23) | Formel (H-24) |
| 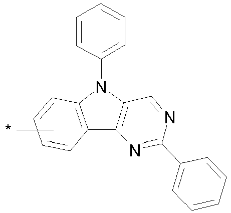 | 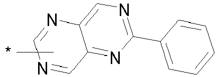 |
| Formel (H-25) | Formel (H-26) |
| 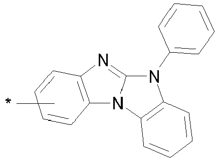 | 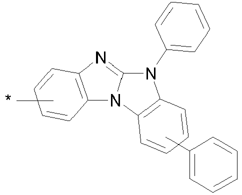 |
| Formel (H-27) | Formel (H-28) |
| 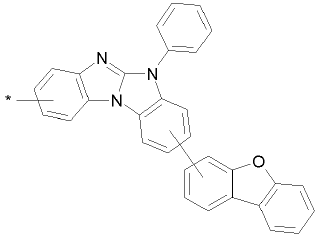 | 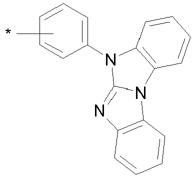 |
| Formel (H-29) | Formel (H-30) |
| 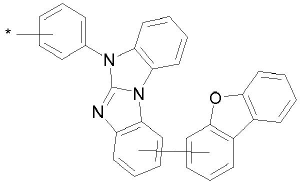 | 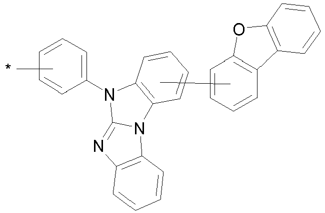 |
| Formel (H-31) | Formel (H-32) |
| 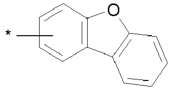 | 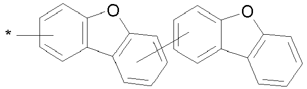 |
| Formel (H-33) | Formel (H-34) |
| 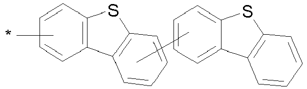 | 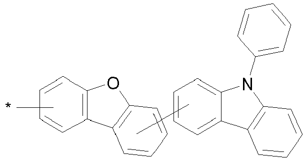 |
| Formel (H-35) | Formel (H-36) |
| 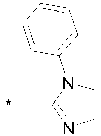 | 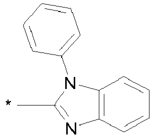 |
| Formel (H-37) | Formel (H-38) |
| 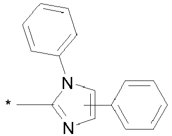 | 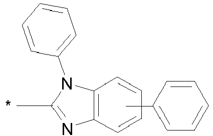 |
| Formel (H-39) | Formel (H-40) |
| 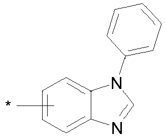 | 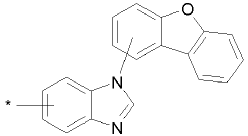 |
| Formel (H-41) | Formel (H-42) |
| 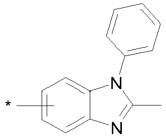 | 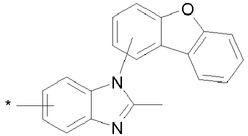 |
| Formel (H-43) | Formel (H-44) |
| 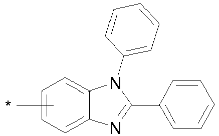 | 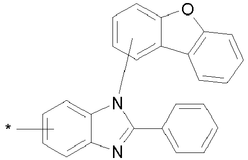 |
| Formel (H-45) | Formel (H-46) |
| 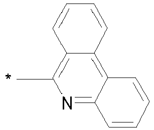 | 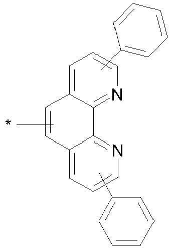 |
| Formel (H-47) | Formel (H-48) |
| 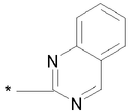 | 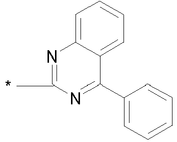 |
| Formel (H-49) | Formel (H-50) |
| 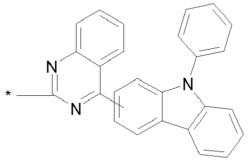 | 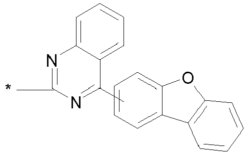 |
| Formel (H-51) | Formel (H-52), |

wobei die Gruppen über die mit * gekennzeichnete Bindung an den Rest der Formel gebunden sind, wobei R⁵ definiert ist wie oben, und bevorzugt den oben genannten bevorzugten Ausführungsformen entspricht, und wobei die Gruppen an den unsubstituiert dargestellten Positionen jeweils mit einem Rest R⁵ substituiert sein können, jedoch bevorzugt in diesen Positionen nicht substituiert sind.

Bevorzugt ist R⁵ bei jedem Auftreten gleich oder verschieden gewählt aus H, D, F, CN, Si(R⁶)₃, N(R⁶)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁶ substituiert sein können; und wobei in den genannten Alkyl- oder Alkoxygruppen eine oder mehrere CH₂-Gruppen durch -C=C-, - R⁶C=CR⁶-, Si(R⁶)₂, C=O, C=NR⁶, -NR⁶-, -O-, -S-, -C(=O)O- oder - C(=O)NR⁶- ersetzt sein können.

Bevorzugte Ausführungsformen der Formel (I) entsprechen den folgenden Formeln (I-H-1) bis (I-H-7) und (I-A-1) bis (I-A-7)

| | |
|---|---|
| 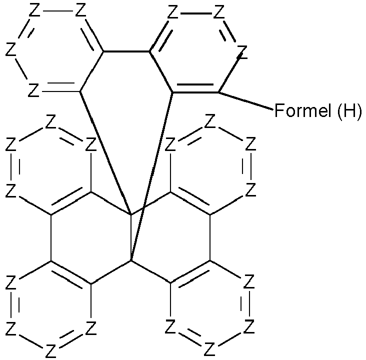 | 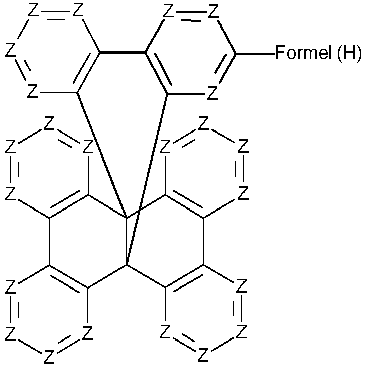 |
| Formel (I-H-1) | Formel (I-H-2) |
| 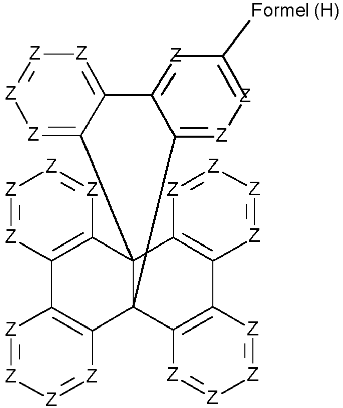 | 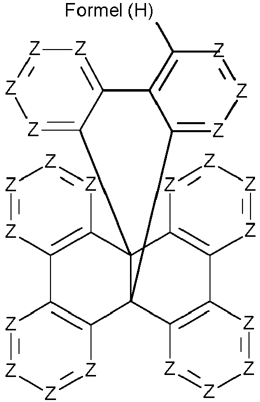 |
| Formel (I-H-3) | Formel (I-H-4) |
| 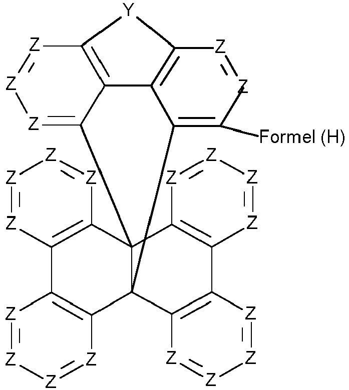 | 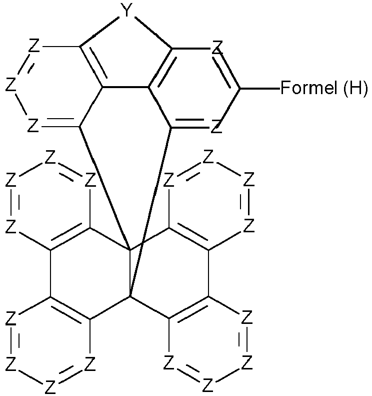 |
| Formel (I-H-5) | Formel (I-H-6) |
| 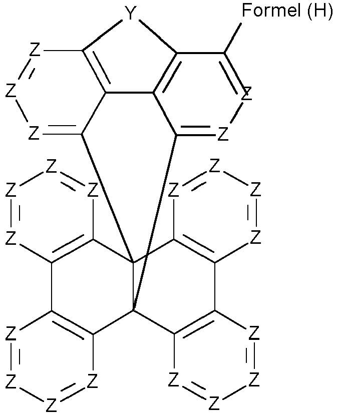 | 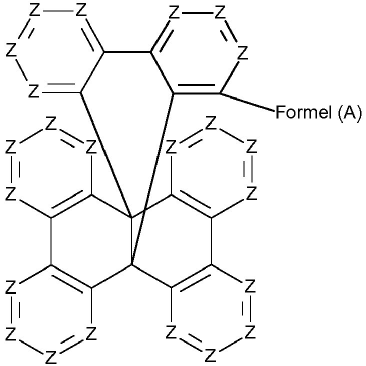 |
| Formel (I-H-7) | Formel (I-A-1) |
| 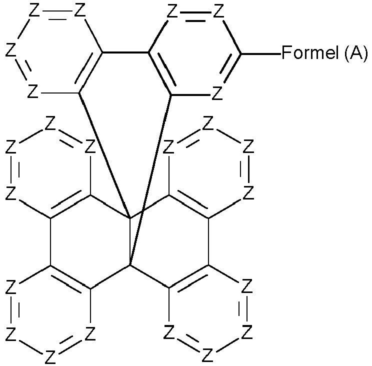 | 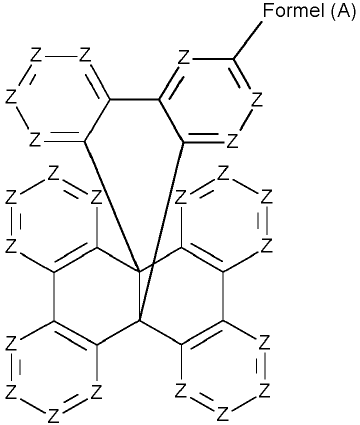 |
| Formel (I-A-2) | Formel (I-A-3) |
| 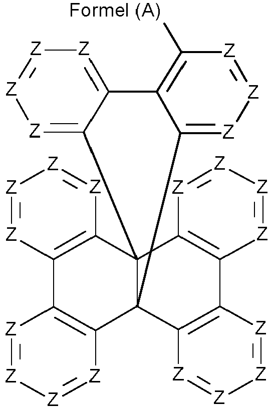 | 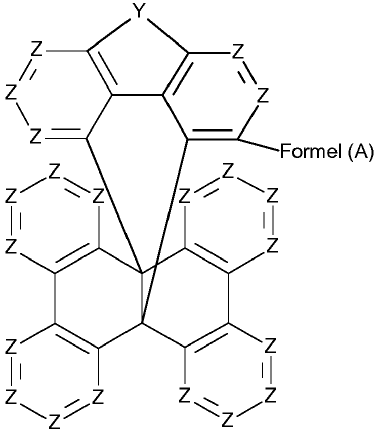 |
| Formel (I-A-4) | Formel (I-A-5) |
| 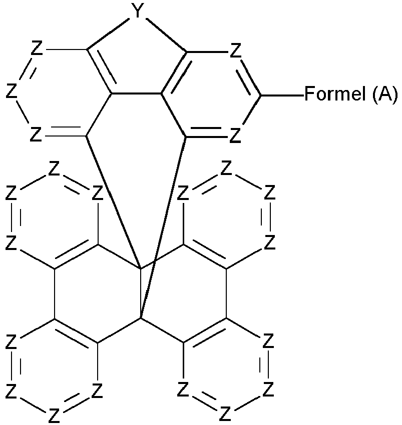 | 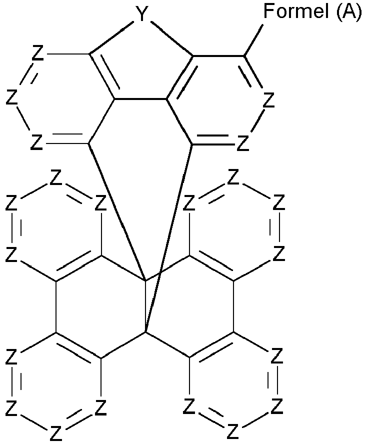 |
| Formel (I-A-6) | Formel (I-A-7), |

wobei die auftretenden Gruppen wie oben definiert sind und bevorzugt ihren bevorzugten Ausführungsformen entsprechen, und wobei "Formel (A)" eine Gruppe der Formel (A), wie oben definiert, ist, und wobei "Formel (H)" eine Gruppe der Formel (H), wie oben definiert, ist. In den oben genannten Formeln ist es insbesondere bevorzugt, dass Z gleich CR¹ ist.

Bevorzugte Ausführungsformen der Formel (II) entsprechen den folgenden Formeln (II-A-1) bis (II-A-3)

| | |
|---|---|
| 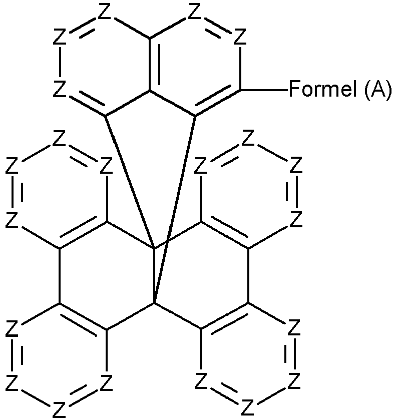 | 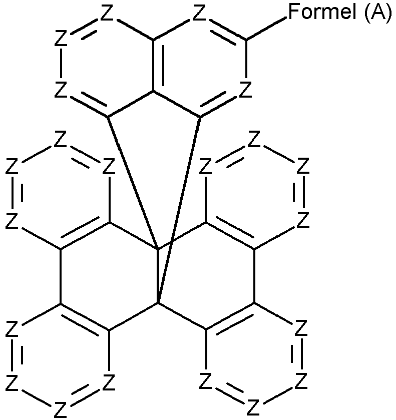 |
| Formel (II-A-1) | Formel (II-A-2) |
| 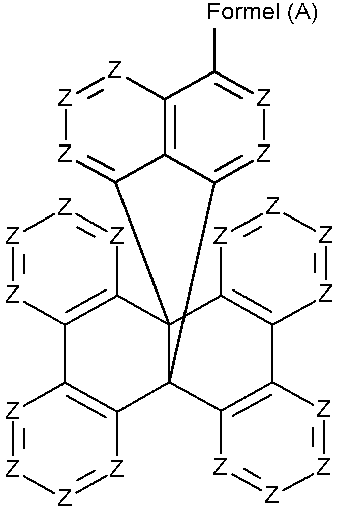 | |
| Formel (II-A-3) | |

wobei die auftretenden Gruppen wie oben definiert sind und bevorzugt ihren bevorzugten Ausführungsformen entsprechen, und wobei "Formel (A)" eine Gruppe der Formel (A), wie oben definiert, ist. In den oben genannten Formeln ist es insbesondere bevorzugt, dass Z gleich CR¹ ist.

Besonders bevorzugt unter den oben genannten Formeln ist die Formel (II-A-3).

Bevorzugte Ausführungsformen der Formel (III) entsprechen den folgenden Formeln (III-A-1) bis (III-A-3)

| | |
|---|---|
| 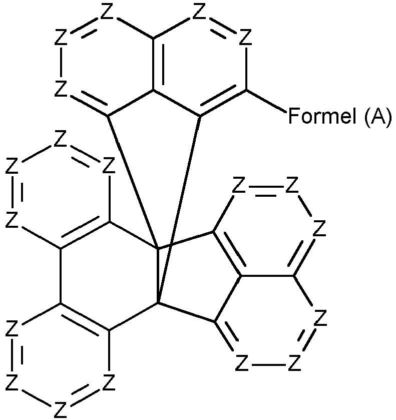 | 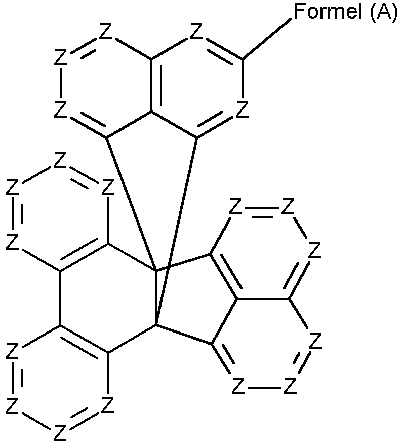 |
| Formel (III-A-1) | Formel (III-A-2) |
| 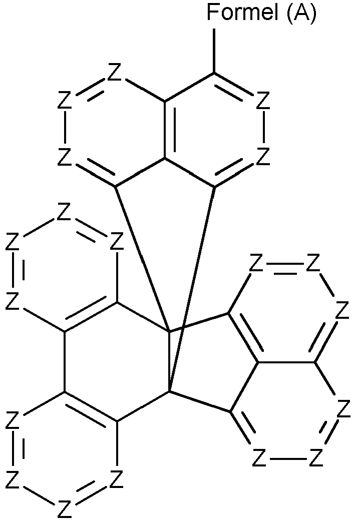 | |
| Formel (III-A-3) | |

wobei die auftretenden Gruppen wie oben definiert sind und bevorzugt ihren bevorzugten Ausführungsformen entsprechen, und wobei "Formel (A)" eine Gruppe der Formel (A), wie oben definiert, ist. In den oben genannten Formeln ist es insbesondere bevorzugt, dass Z gleich CR¹ ist.

Besonders bevorzugt unter den oben genannten Formeln ist die Formel (III-A-3).

Bevorzugte Ausführungsformen der Formel (IV) entsprechen den folgenden Formeln (IV-A-1) bis (IV-A-3)

| | |
|---|---|
| 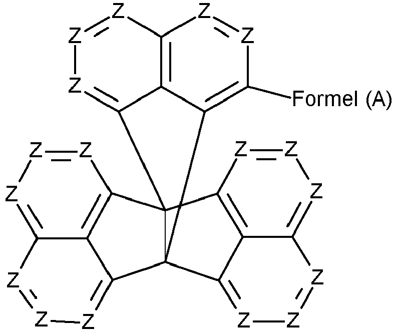 | 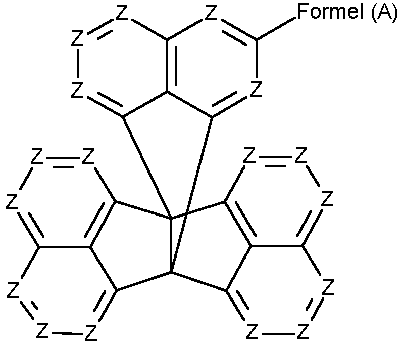 |
| Formel (IV-A-1) | Formel (IV-A-2) |
| 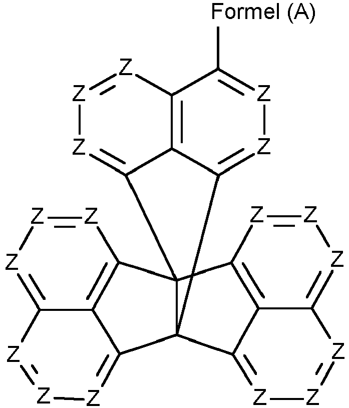 | |
| Formel (IV-A-3) | |

wobei die auftretenden Gruppen wie oben definiert sind und bevorzugt ihren bevorzugten Ausführungsformen entsprechen, und wobei "Formel (A)" eine Gruppe der Formel (A), wie oben definiert, ist. In den oben genannten Formeln ist es insbesondere bevorzugt, dass Z gleich CR¹ ist.

Besonders bevorzugt unter den oben genannten Formeln ist die Formel (IV-A-3).

Bevorzugte Verbindungen der Formeln (I) bis (IV) sind in der folgenden Tabelle gezeigt:

| | |
|---|---|
| 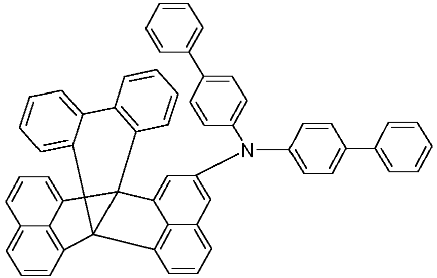 | 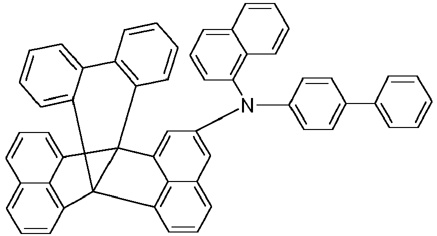 |
| 1 | 2 |
| 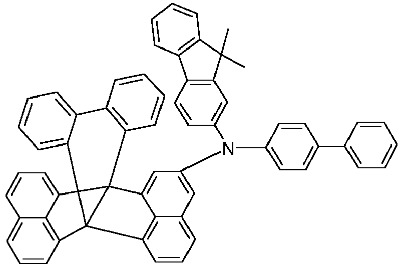 | 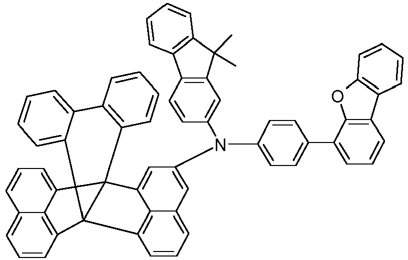 |
| 3 | 4 |
| 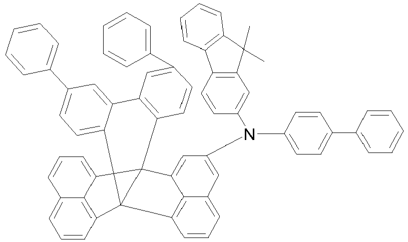 | 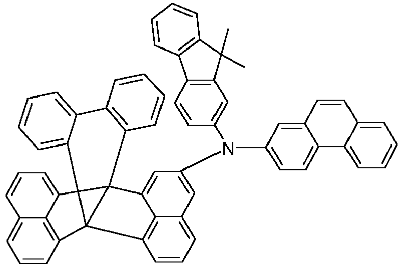 |
| 5 | 6 |
| 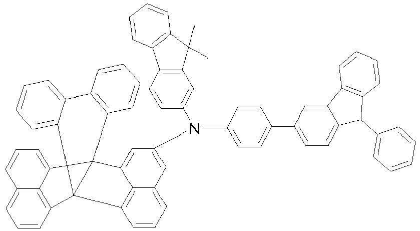 | 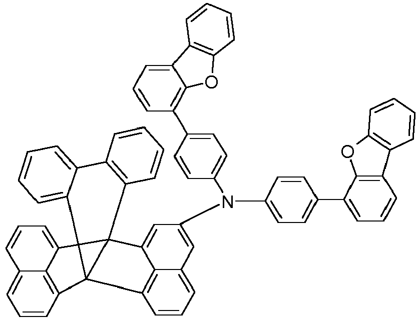 |
| 7 | 8 |
| 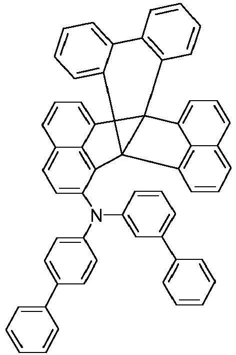 | 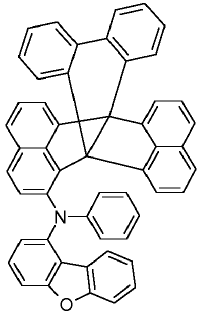 |
| 9 | 10 |
| 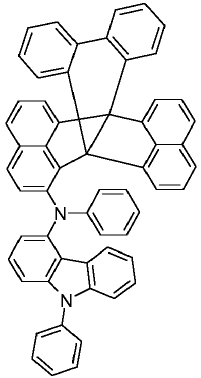 | 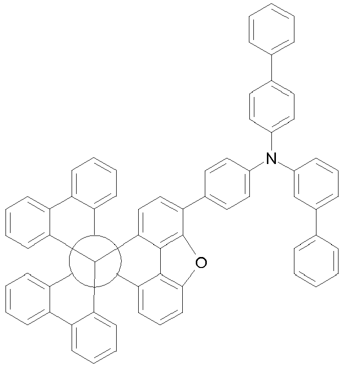 |
| 11 | 12 |
| 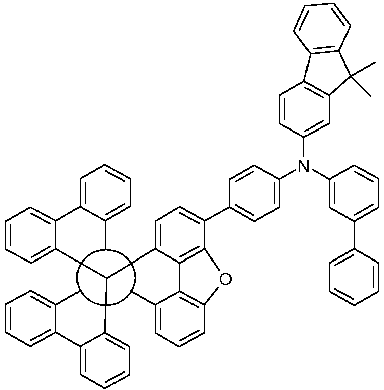 | 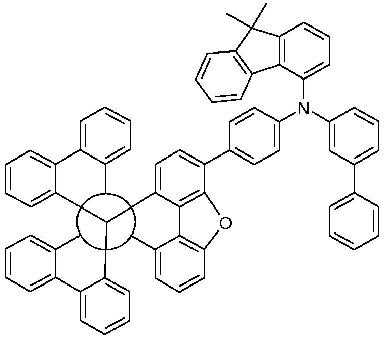 |
| 13 | 14 |
| 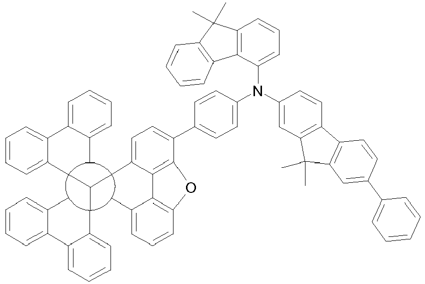 | 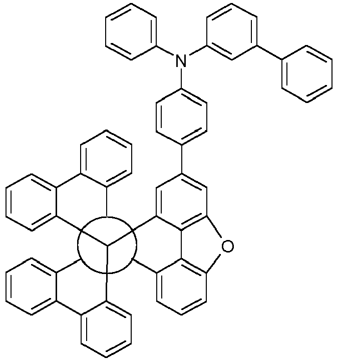 |
| 15 | 16 |
| 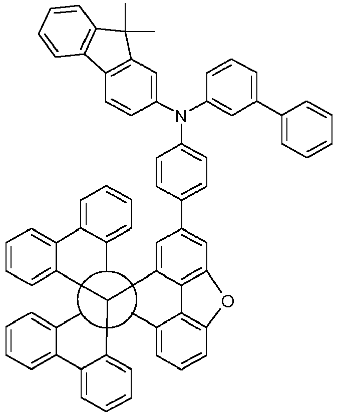 | 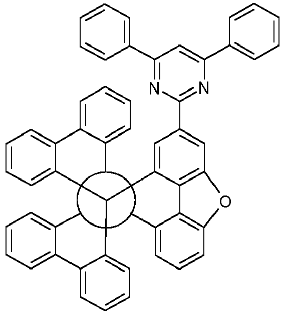 |
| 17 | 18 |
| 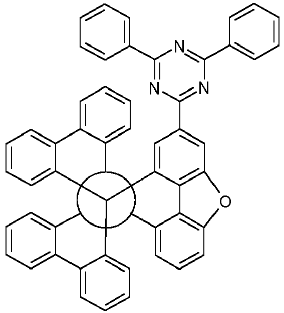 | 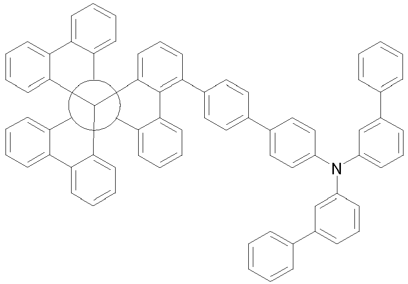 |
| 19 | 20 |
| 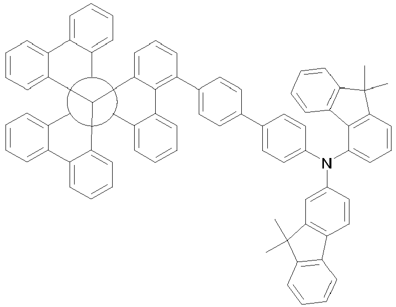 | 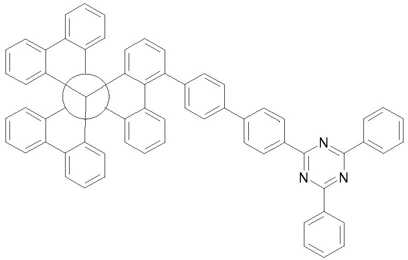 |
| 21 | 22 |

Die erfindungsgemäßen Verbindungen können mittels bekannter Synthesemethoden hergestellt werden.

Die Synthese von Verbindungen der Formel (I) erfolgt ausgehend von Alkoxy-Brom-Biphenyl-Derivaten, die durch Umsetzung mit Lithiumorganylen oder Magnesium in reaktive Nukleophile überführt, und dann an ein gegebenenfalls funktionalisiertes Spiro[9H-fluoren-9,9'(10'H)-phenanthren]-10'-on addiert werden (s. Schema 1). Der so erhaltene tertiäre Alkohol cyclisiert säurekatalysiert unter intramolekularer Umlagerung zum Alkoxy-funktionalisierten [4.4.4]-Propellan (Schritte 1 und 2). Eine Methylether-Spaltung in geschmolzenem Pyridiniumhydrochlorid (Schritt 3) und anschließende Verersterung des gebildeten Phenols mit Trifluormethansulfonsäureanhydrid (Schritt 4) führt zu den reaktiven Triflaten, die dann Palladium- bzw. Kupfer-katalysiert nach literaturbekannten Standardmethoden mit sekundären Aminen in C-N-Kupplungen (z.B. Hartwig-Buchwald-Kupplung) bzw. mit Aryl-/Heteroaryl-Boronsäuren, -Boronsäureestern, Organo-Zinn- oder -Zink-Verbindungen in C-C-Kupplungen (z.B. Suzuki-, Stille-, Negishi-Kupplung, etc.) zu den erfindungsgemäßen Verbindungen umgesetzt werden (Schritt 5).

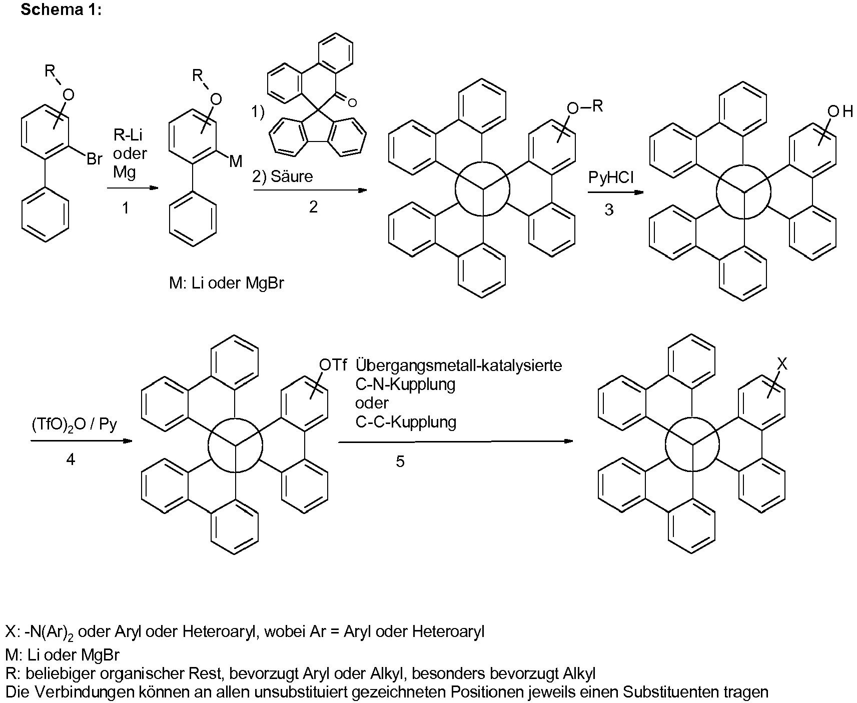

In einer Abwandlung der in Schema 1 gezeigten Synthesesequenz (Schema 1a) kann anstelle des Brom-Alkoxy-Biphenyl-Derivats ein entsprechendes verbrücktes Derivat, beispielsweise ein Brom-Alkoxysubstituiertes Dibenzofuran-Derivat, eingesetzt werden. Dabei werden auf demselben Syntheseweg wie in Schema 1 beschrieben, verbrückte Derivate der Formel (I) erhalten:

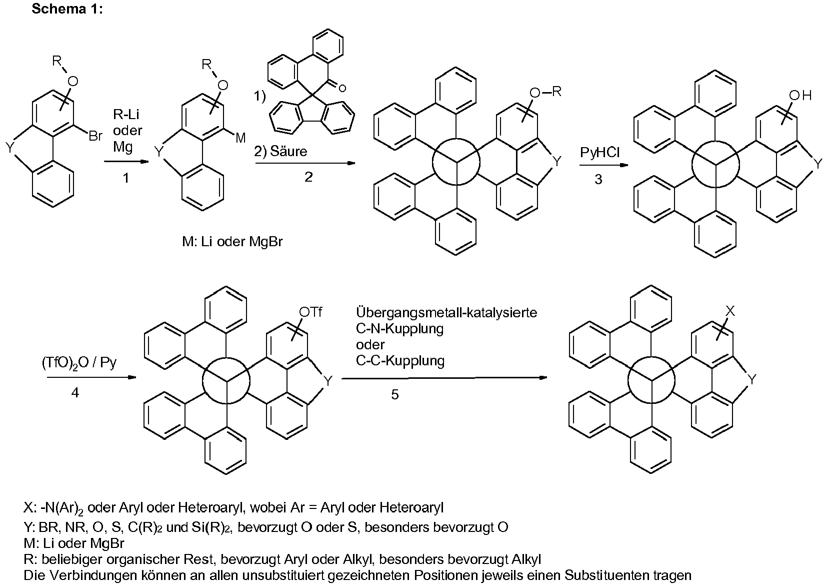

Analog zur in Schema 1 gezeigten Reaktionssequenz können erfindungsgemäße Verbindungen der Formel (II) unter Einsatz von Alkoxy-Brom-Naphthalin-Derivaten anstelle von Alkyoxy-Brom-Biphenyl-Derivaten erhalten werden (Schema 2).

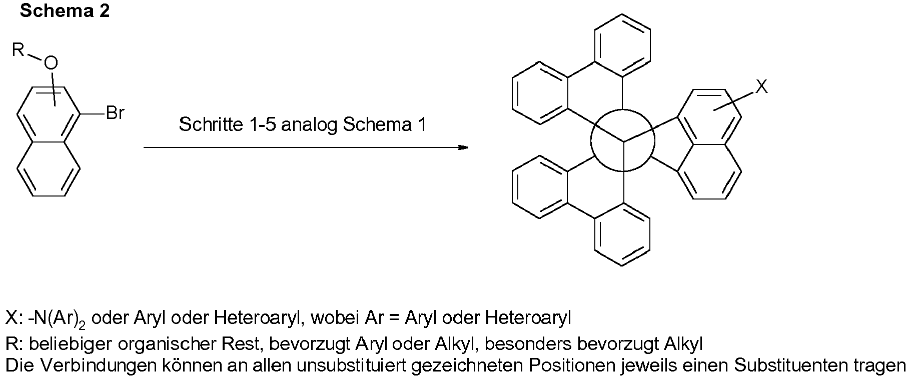

Verbindungen der Formel (III) lassen sich in Analogie zu G. Dyker et al., Angewandte Chemie, 105(12), 1805 aus Methoxy-acenaphth[1,2-a] acenaphthylen-Derivaten und lodbenzol herstellen, wobei sich an den unten gezeigten ersten Schritt in Schema 3 die oben in Schema 1 beschriebene Abfolge der Schritte 3-5 anschließt.

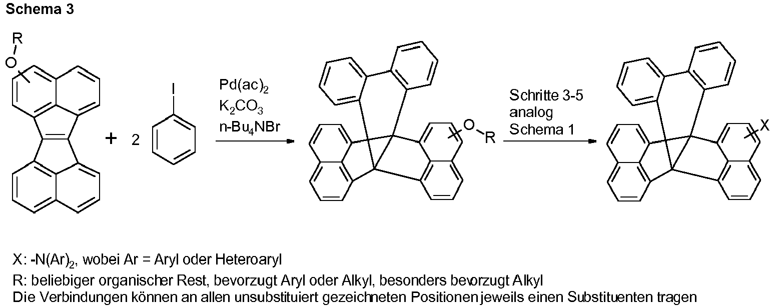

Verbindungen der Formel (IV) lassen sich aus alkoxysubstituierten 1,2-Acenaphthenen und unsubstituierten oder substituierten 1-Naphthylmagnesiumbromiden und säurekatalysierter Umlagerung der intermediären Diole herstellen, wobei sich an den unten gezeigten Schritt 1 in Schema 4 die oben in Schema 1 beschriebene Abfolge der Schritte 3-5 anschließt.

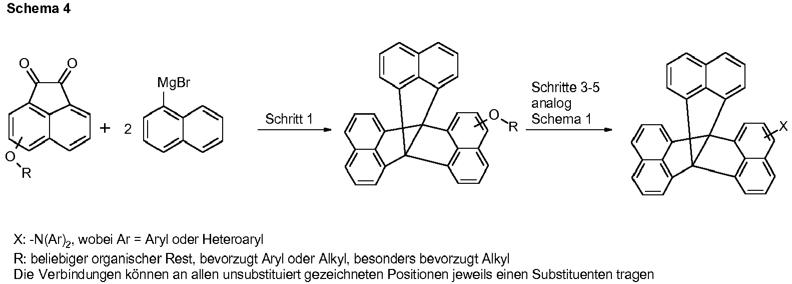

Die oben gezeigten Synthesen stellen Beispiele dar, an die der Fachmann nicht gebunden ist. Er kann im Rahmen seines allgemeinen Fachwissens abgewandelte Synthesen durchführen, um zu den Verbindungen der Formeln (I) bis (IV) zu gelangen.

Gegenstand der vorliegenden Anmeldung ist ein Verfahren zur Herstellung einer erfindungsgemäßen Verbindung, dadurch gekennzeichnet, dass zunächst das Alkoxy-substituierte Grundgerüst hergestellt wird, welches in einem weiteren Schritt in eine reaktive Verbindung überführt wird, bevorzugt in ein Triflat-Derivat, welches in einem weiteren Schritt durch Übergangsmetall-katalysierte Kupplungsreaktion, bevorzugt Hartwig-Buchwald-, Suzuki-, Stille-, oder Negishi-Kupplung, in die erfindungsgemäße Verbindung überführt wird. Bevorzugt sind dabei Hartwig-Buchwald-Kupplung zur Herstellung einer erfindungsgemäßen Verbindung, die eine Aminogruppe direkt gebunden an das Grundgerüst trägt, sowie Suzuki-Kupplung zur Herstellung einer erfindungsgemäßen Verbindung, die eine Aryl- oder Heteroarylgruppe direkt gebunden an das Grundgerüst trägt.

Zur Herstellung einer Verbindung der Formel (I) wird zur Synthese des oben genannten Alkoxy-substituierten Grundgerüsts ein Biphenyl-Derivat eingesetzt, welches sowohl eine Alkoxygruppe als auch ein Halogenatom, bevorzugt Brom, als Substituenten trägt. Diese Biphenyl-Derivat wird zunächst metalliert, bevorzugt durch Lithium-Organyle oder Magnesium, und das metallierte Derivat wird anschließend an ein Spiro[9H-fluoren-9,9'(10'H)-phenanthren]-10'-on-Derivat addiert, woran sich eine Zyklisierung des tertiären Alkohols und Umlagerung anschließt, in der als Endprodukt das oben genannte Alkoxy-substituierte Grundgerüst der Formel (I) entsteht.

Zur Herstellung einer Verbindung der Formel (II) wird zur Synthese des oben genannten Alkoxy-substituierten Grundgerüsts ein Naphthyl-Derivat eingesetzt, welches sowohl eine Alkoxygruppe als auch ein Halogenatom, bevorzugt Brom, als Substituenten trägt. Diese Naphthyl-Derivat wird zunächst metalliert, bevorzugt durch Lithium-Organyle oder Magnesium, und das metallierte Derivat wird anschließend an ein Spiro[9H-fluoren-9,9'(10'H)-phenanthren]-10'-on-Derivat addiert, woran sich eine Zyklisierung des tertiären Alkohols und Umlagerung anschließt, in der als Endprodukt das oben genannte Alkoxy-substituierte Grundgerüst der Formel (II) entsteht.

Zur Herstellung einer Verbindung der Formel (III) wird zur Synthese des oben genannten Alkoxy-substituierten Grundgerüsts ein lodbenzol-Derivat eingesetzt. Dieses lodbenzol-Derivat wird unter Pd-Katalyse mit einem Methoxy-acenaphth[1,2-a]acenaphthylen-Derivat umgesetzt, in einer Sequenz aus Heck-Reaktion und Pd-katalysierter C-C-Kupplung und Pd-katalysierter C-C-Kupplung unter C-H-Aktivierung. Als Endprodukt entsteht das oben genannte Alkoxy-substituierte Grundgerüst der Formel (III).

Zur Herstellung einer Verbindung der Formel (IV) wird zur Synthese des oben genannten alkoxysubstituierten Grundgerüsts ein 1-Naphthylmagnesiumbromid-Derivat mit einem alkoxysubstituierten 1,2-Acenaphthen umgesetzt, wobei das entstehende intermediäre Diol säurekatalysiert umlagert zum alkoxy- oder halogensubstituierten Grundgerüst der Formel (IV).

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), (II), (III) oder (IV), wobei die Bindung(en) zum Polymer, Oligomer oder Dendrimer an beliebigen, in den genannten Formeln mit R¹, R² oder R⁵ substituierten Positionen lokalisiert sein können. Je nach Verknüpfung der Verbindung gemäß Formel (I), (II), (III) oder (IV) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß den oben genannten Formeln direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß den oben genannten Formeln über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I), (II), (III) oder (IV) in Oligomeren, Dendrimeren und Polymeren gelten dieselben Bevorzugungen wie oben für Verbindungen gemäß den oben genannten Formeln beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I), (II), (III) oder (IV) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I), (II), (III) oder (IV) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I), (II), (III) oder (IV) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine erfindungsgemäße Verbindung. Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine erfindungsgemäße Verbindung enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, *Multiphoton Organic EL Device Having Charge Generation Layer*) und/oder organischen oder anorganischen p/n-Übergängen.

Die Abfolge der Schichten der organischen Elektrolumineszenzvorrichtung enthaltend die Verbindung der Formel (I), (II), (III) oder (IV) ist bevorzugt die folgende:
Anode-Lochinjektionsschicht-Lochtransportschicht-wahlweise weitere Lochtransportschicht(en)-wahlweise Elektronenblockierschichtemittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in einer Lochtransportschicht, Lochinjektionsschicht, Elektronenblockierschicht, emittierenden Schicht, lochblockierenden Schicht und/oder elektronentransportierenden Schicht vorhanden, besonders bevorzugt in einer Lochtransportschicht, einer emittierenden Schicht als Matrixmaterial, in einer Lochblockierschicht und/oder in einer Elektronentransportschicht.

Es ist erfindungsgemäß bevorzugt, wenn die erfindungsgemäße Verbindung in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht, einer emittierenden Schicht, einer Lochblockierschicht, und/oder einer Elektronentransportschicht enthalten sein. Besonders bevorzugt ist sie in einer emittierenden Schicht in Kombination mit einer phosphoreszierenden emittierenden Verbindung enthalten.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den erfindungsgemäßen Verbindungen in organischen Elektrolumineszenzvorrichtungen einsetzen. Weitere Beispiele sind in der folgenden Tabelle aufgeführt:

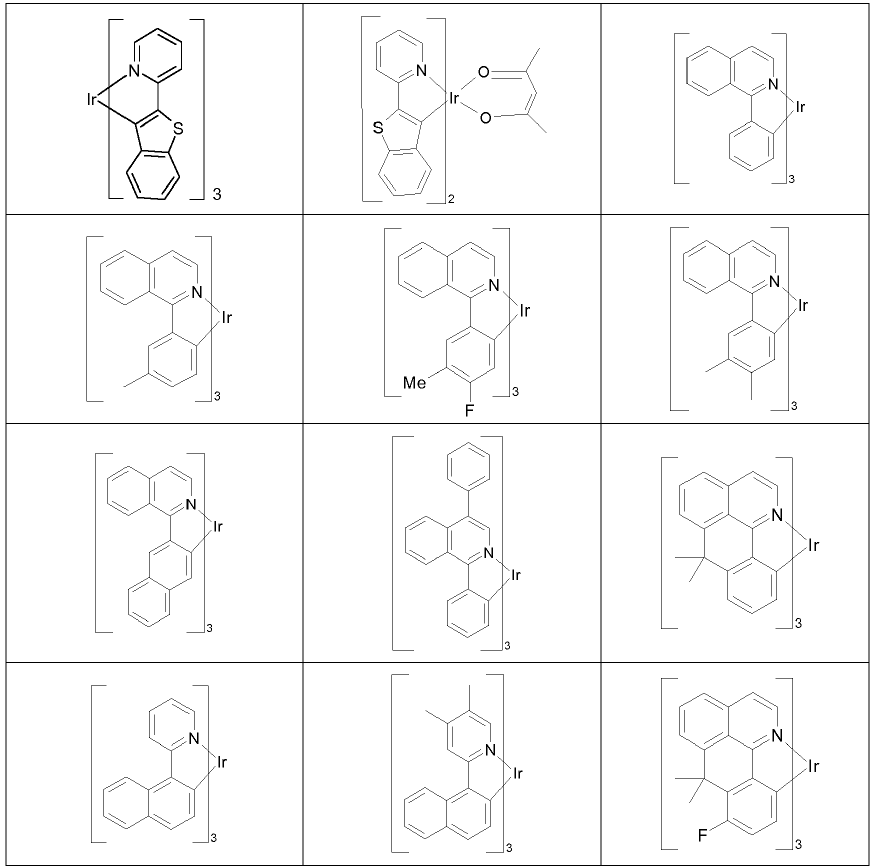

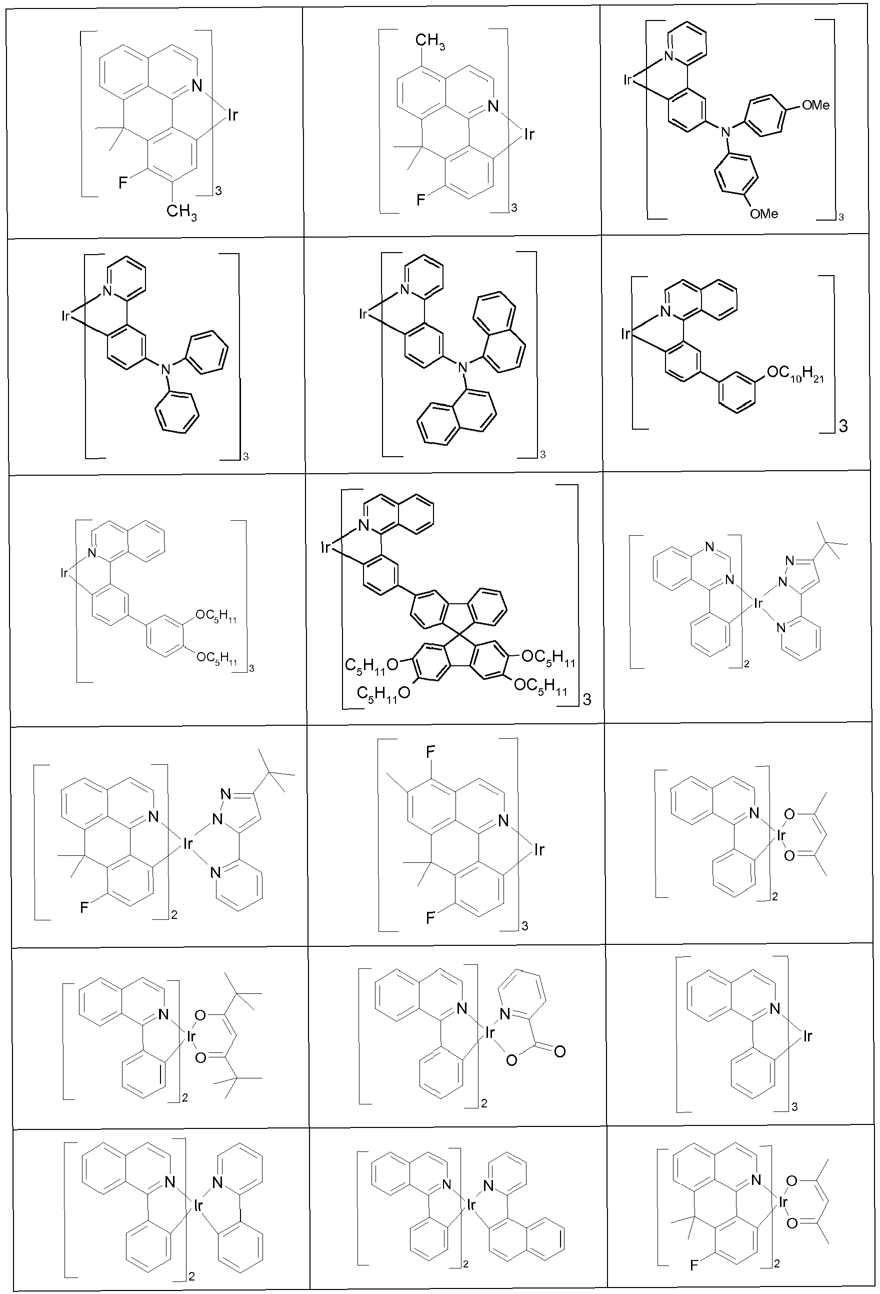

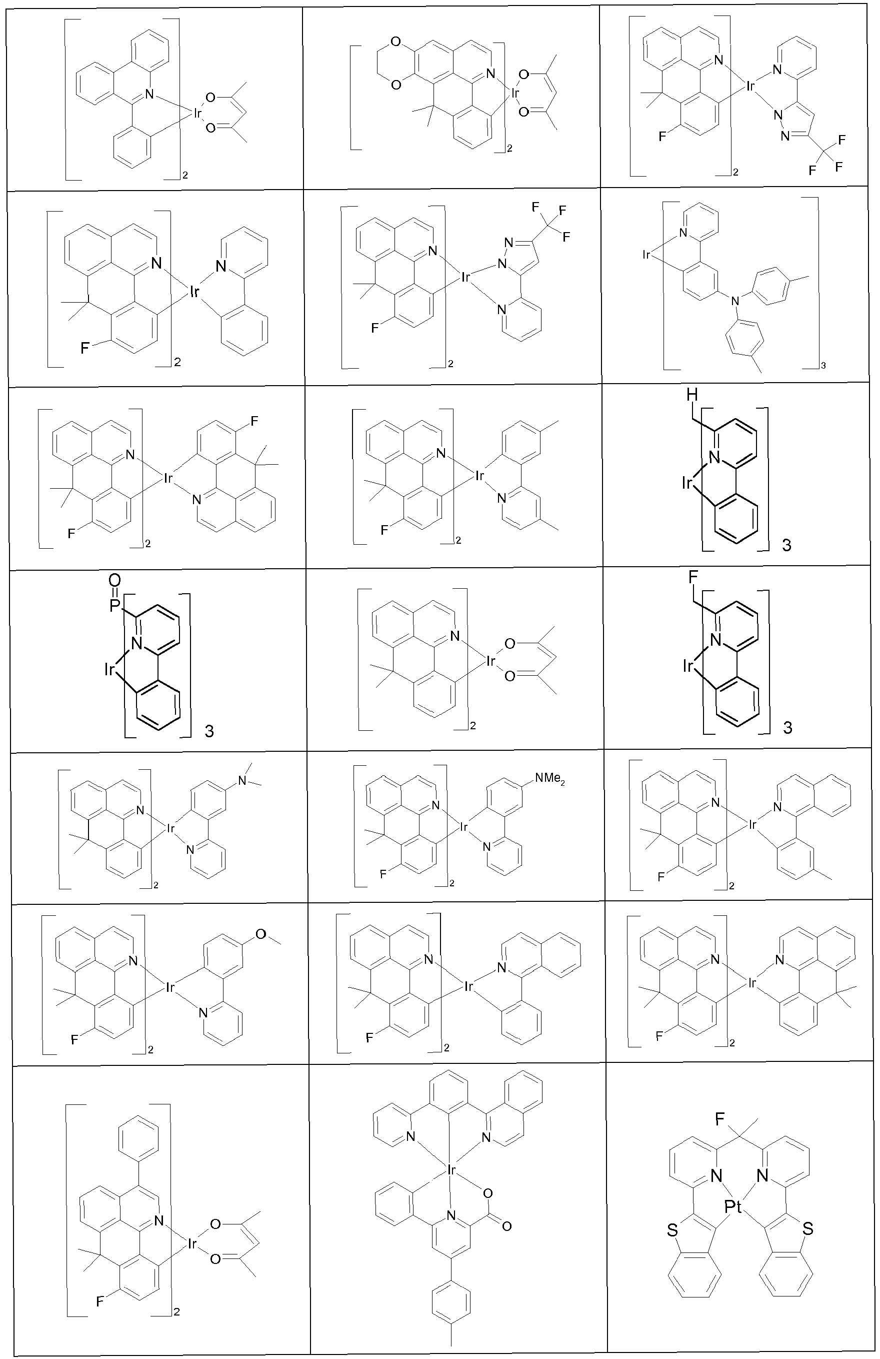

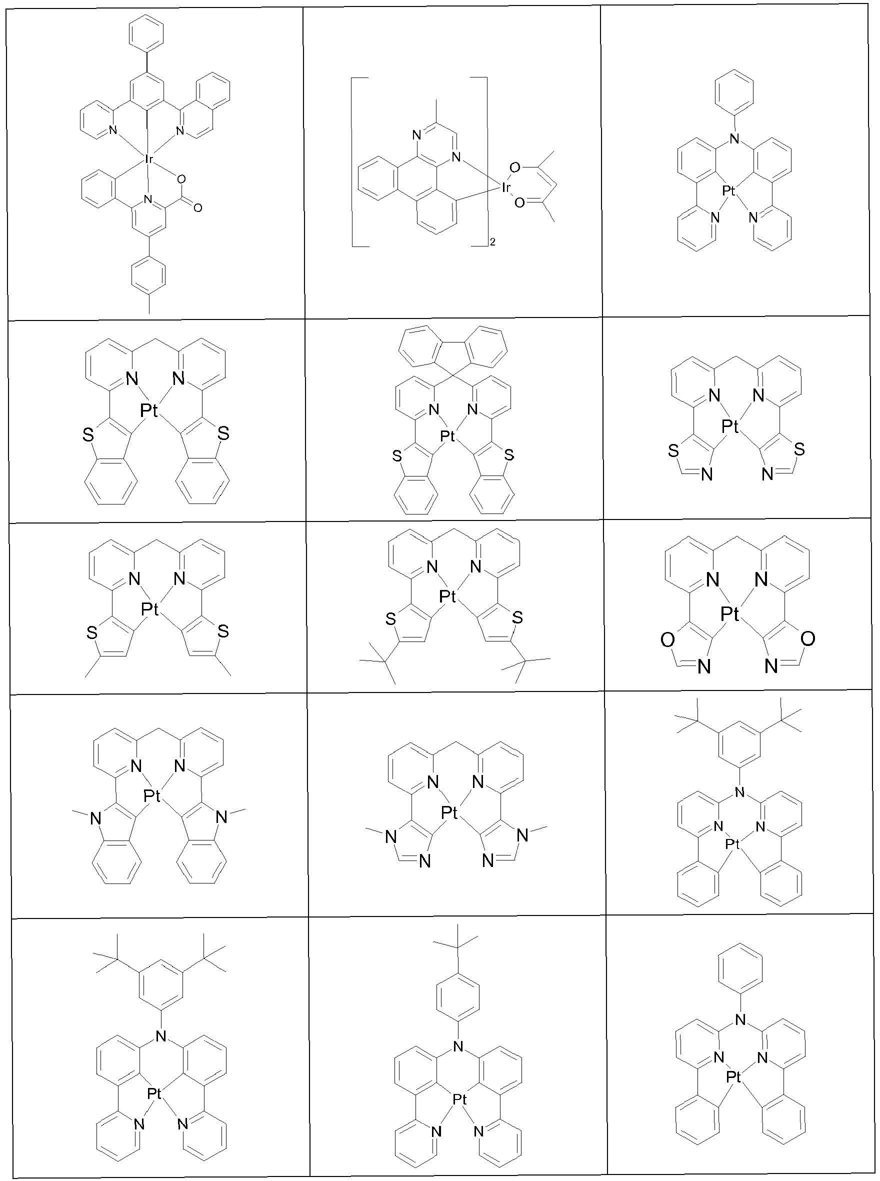

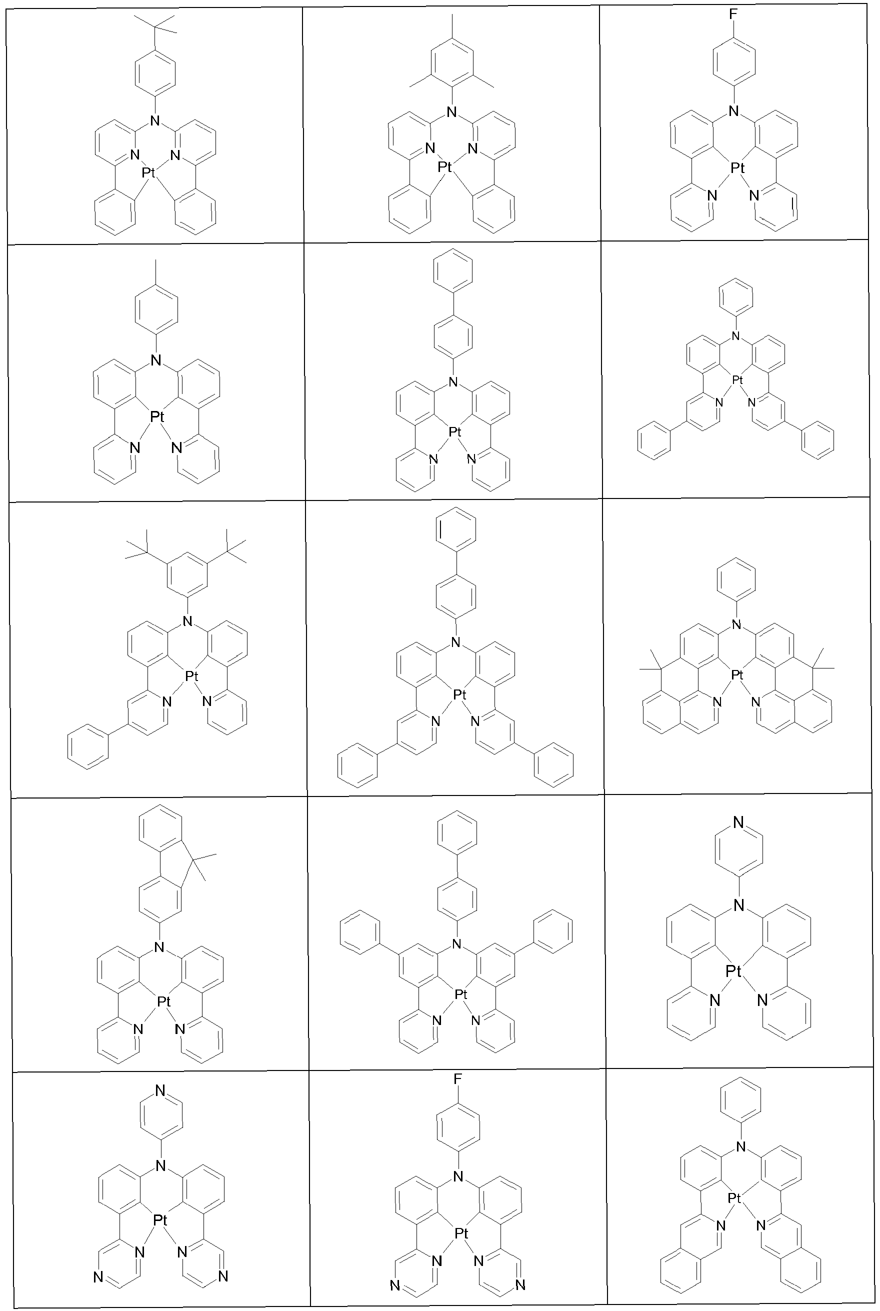

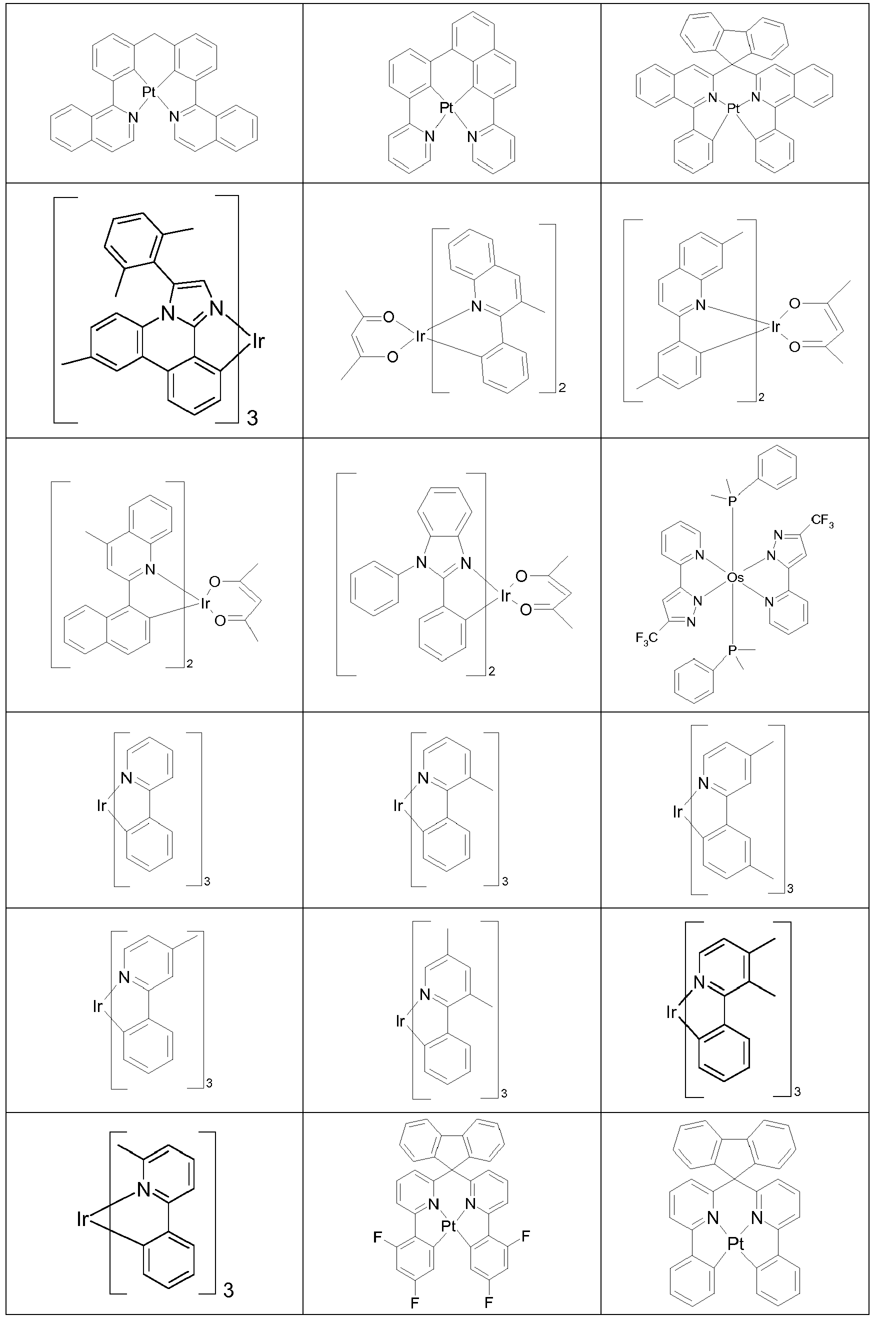

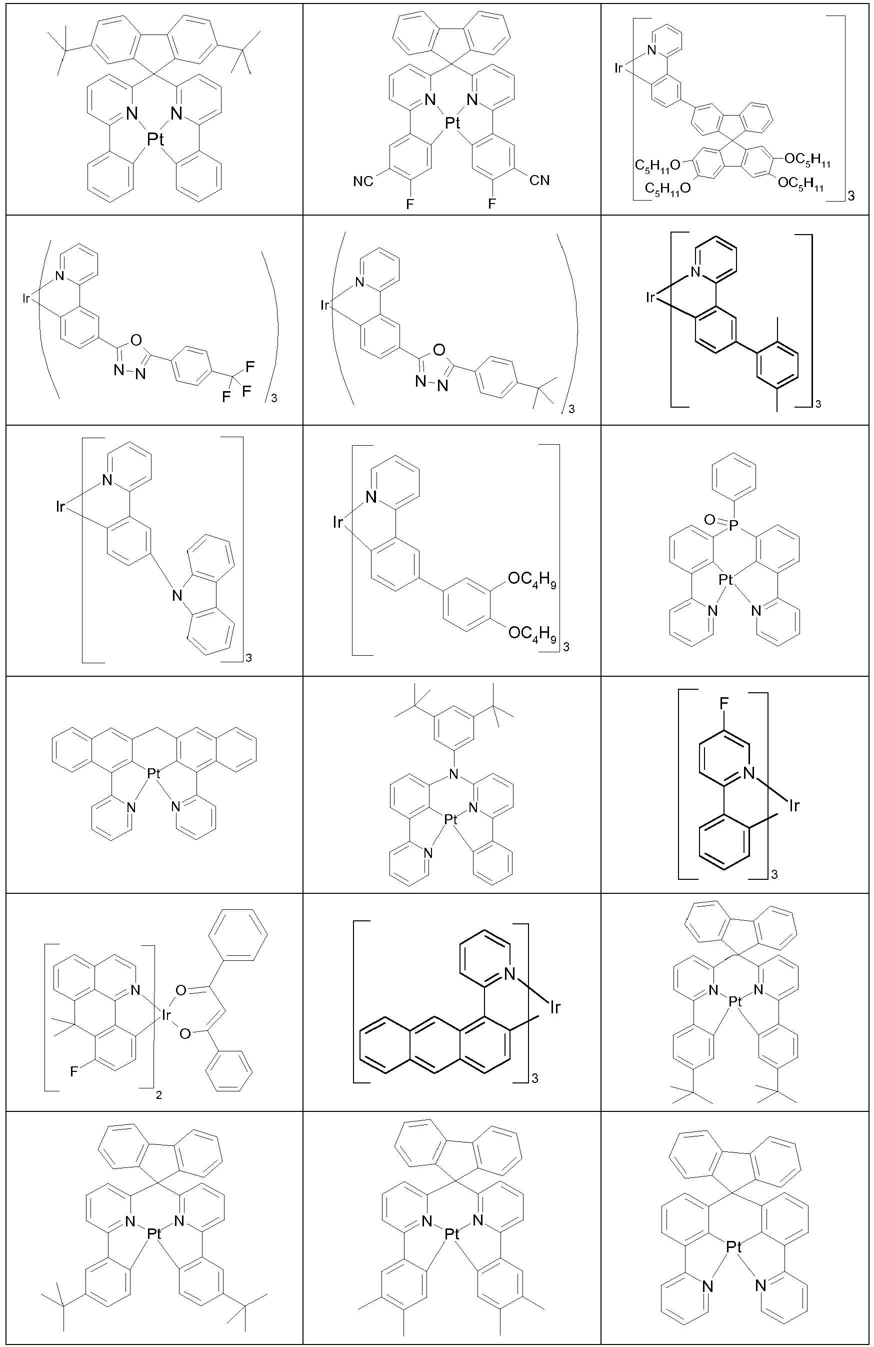

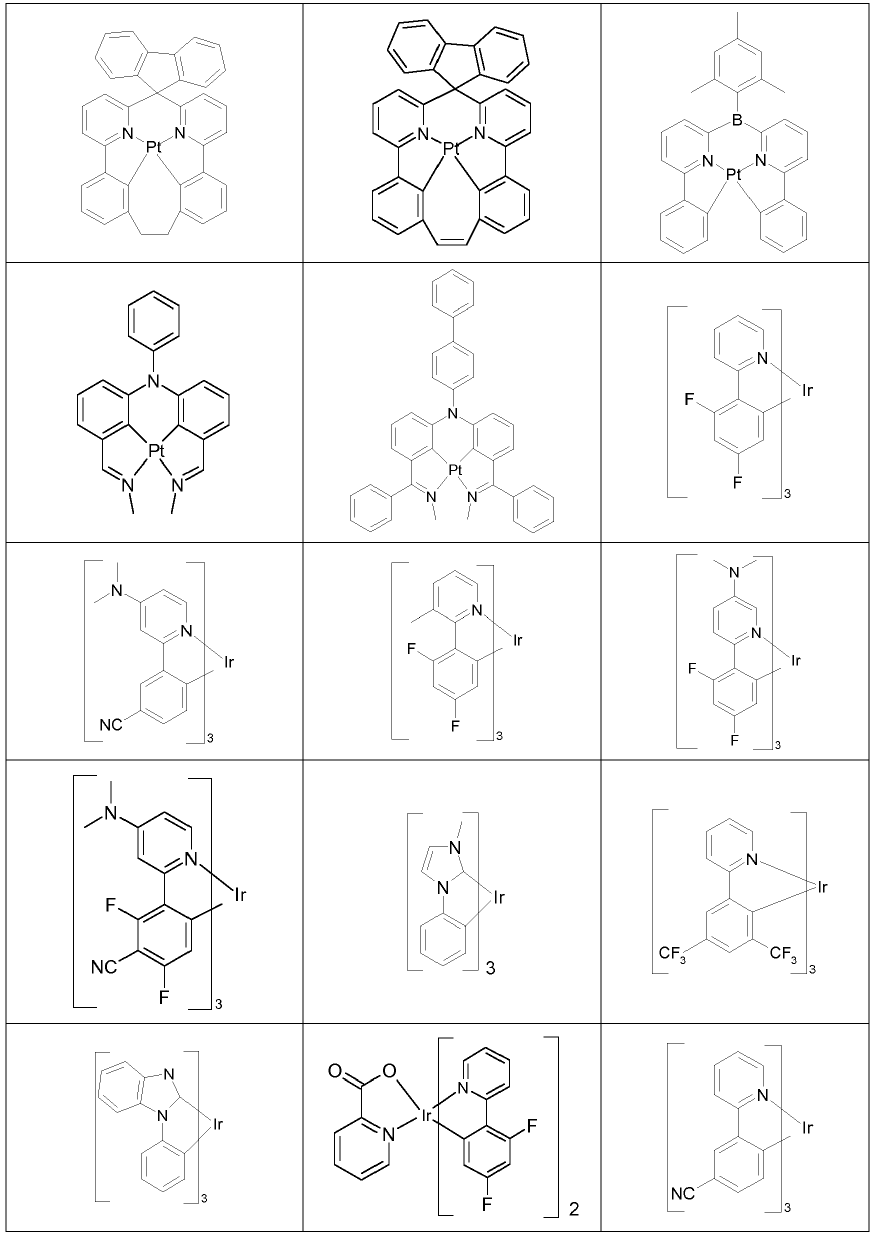

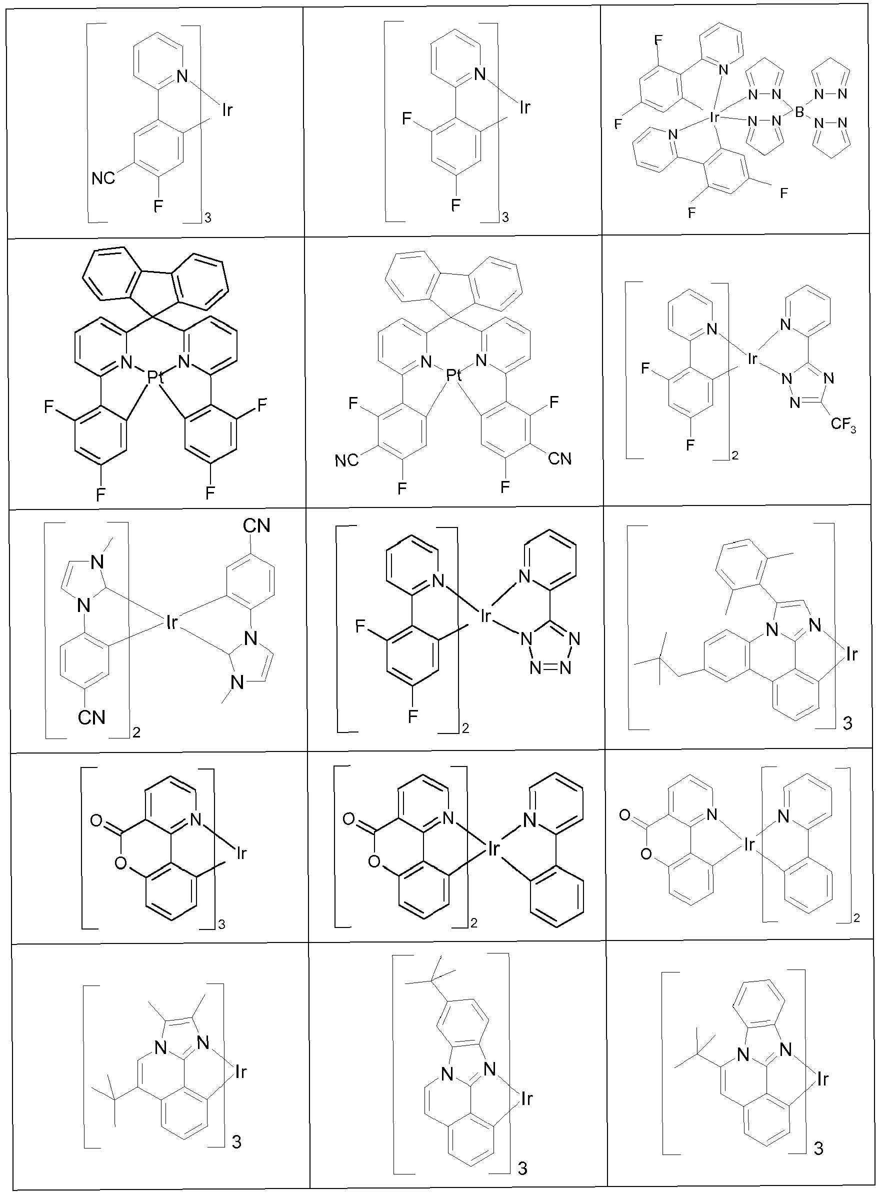

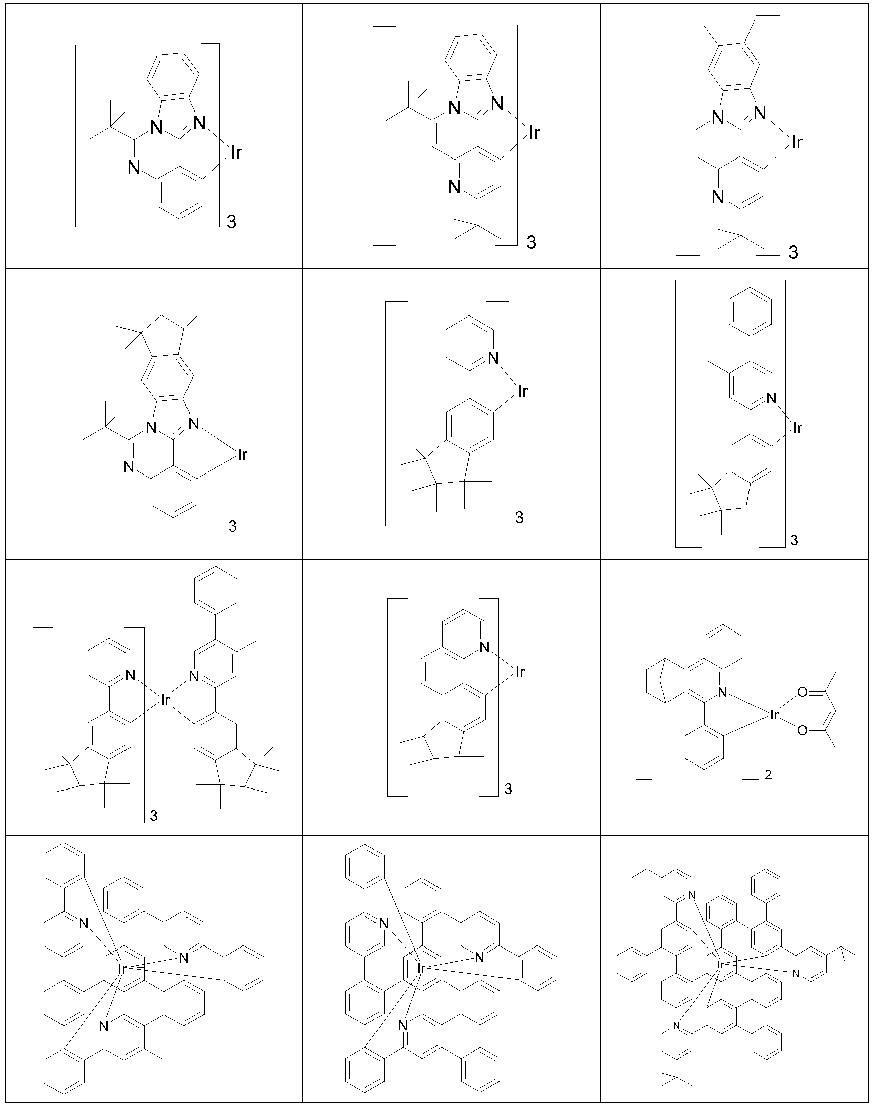

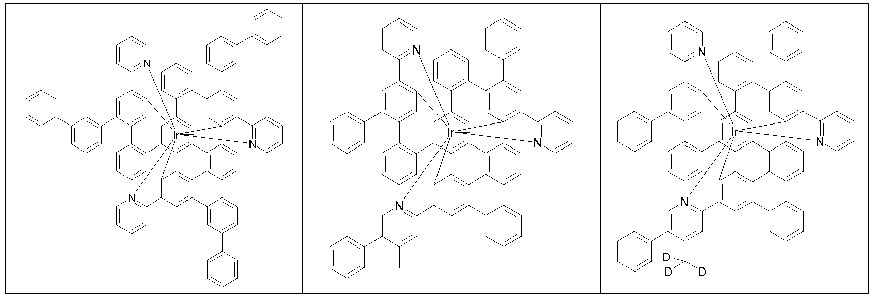

In einer bevorzugten Ausführungsform der Erfindung werden die erfindungsgemäßen Verbindungen als lochtransportierendes Material eingesetzt. Hierzu sind insbesondere Verbindungen der Formeln (I) bis (IV) geeignet, die genau eine Gruppe der Formel (A) enthalten. Die Verbindungen liegen dann bevorzugt in einer Lochtransportschicht, einer Elektronenblockierschicht oder einer Lochinjektionsschicht vor.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I), (II), (III) oder (IV) enthält, besonders bevorzugt genau eine oder zwei eine Verbindung gemäß Formel (I), (II), (III) oder (IV) enthalten.

Wird die erfindungsgemäße Verbindung als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die erfindungsgemäße Verbindung dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 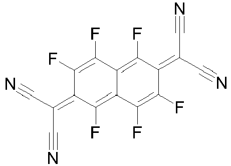 | 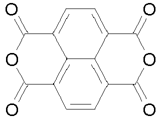 | 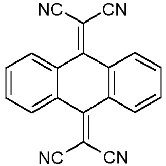 |
| (D-1) | (D-2) | (D-3) |
| 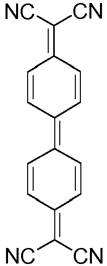 | 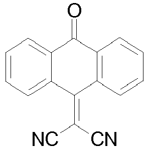 | 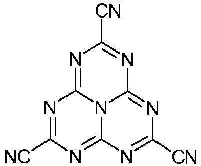 |
| (D-4) | (D-5) | (D-6) |
| 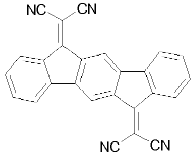 | 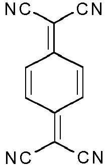 | 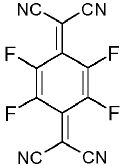 |
| (D-7) | (D-8) | (D-9) |
| 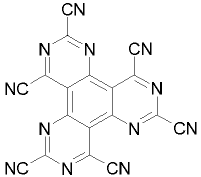 | 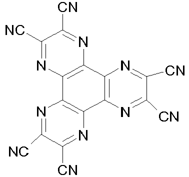 | 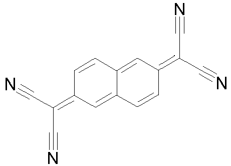 |
| (D-10) | (D-11) | (D-12) |
| 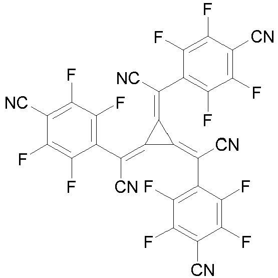 | | |
| (D-13) | | |

In einer weiteren bevorzugten Ausführungsform der Erfindung wird die erfindungsgemäße Verbindung als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt. Diese separate Schicht ist bevorzugt direkt an die Anode angrenzend und zwischen Anode und anodennächster Lochtransportschicht angeordnet.

In einer bevorzugten Ausführungsform der vorliegenden Erfindung wird die erfindungsgemäße Verbindung in einer emittierenden Schicht als Matrixmaterial in Kombination mit einer oder mehreren phosphoreszierenden emittierenden Verbindungen eingesetzt. Hierfür sind insbesondere Verbindungen der Formel (I) - (IV) geeignet, die genau eine Gruppe der Formel (H) aufweisen, insbesondere Verbindungen der oben genannten Formeln, die genau eine Gruppe der Formel (H) aufweisen, die ein oder mehrere Carbazolgruppen enthält. Bevorzugt ist dabei mindestens eine Gruppe L¹ gleich Carbazol, und/oder die Gruppe Ar² ist gleich Carbazol.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die erfindungsgemäßen Verbindungen als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die erfindungsgemäße Verbindung stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Bevorzugte phosphoreszierende emittierende Verbindungen zur Verwendung in Mixed-Matrix-Systemen sind dieselben, wie sie weiter oben als allgemein bevorzugte phosphoreszierende Emittermaterialien aufgeführt wurden.

Gemäß einer weiteren bevorzugten Ausführungsform werden die erfindungsgemäßen Verbindungen in einer oder mehreren Schichten auf der Elektronentransportseite eingesetzt, bevorzugt in einer Loochblockierschicht und/oder einer Elektronentransportschicht. Hierfür sind insbesondere Verbindungen der Formel (I) - (IV) geeignet, die genau eine Gruppe der Formel (H) aufweisen, insbesondere eine Gruppe der Formel (H), in der mindestens eine Gruppe L¹ oder die Gruppe Ar² gewählt ist aus Triazin und Pyrimidin. Bei der Verwendung in einer Elektronentransportschicht ist es bevorzugt, dass sie in Kombination mit einem Metallkomplex, bevorzugt einem Metall-Chinolinat, besonders bevorzugt einem Lithium-Chinolinat, eingesetzt werden.

Im Folgenden werden bevorzugte Ausführungsformen für die verschiedenen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in der noch nicht offengelegten Anmeldung EP 15182993.4 offenbarten erweiterten Benzoindenofluorene, die in den noch nicht offengelegten Anmeldungen EP 15181178.3 und EP 15181177.5 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in der noch nicht offengelegten Anmeldung EP 15180777.3 offenbarten Indeno-Benzofurane, und die in der noch nicht offengelegten Anmeldung EP 15182962.9 offenbarten Phenanthryl-Anthracene.

Bevorzugte Matrixmaterialien für phosphoreszierende emittierende Verbindungen sind neben den erfindungsgemäßen Verbindungen aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugt umfasst die erfindungsgemäße OLED zwei oder mehr unterschiedliche lochtransportierende Schichten. Die erfindungsgemäße Verbindung kann dabei in einer oder in mehreren oder in allen lochtransportierenden Schichten eingesetzt werden. Gemäß einer bevorzugten Ausführungsform wird die erfindungsgemäße Verbindung in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den erfindungsgemäßen Verbindungen bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051 und den noch nicht offen gelegten Anmeldungen PCT/EP2015/002475 und PCT/EP2016/000084, Phenanthren-Diarylamine, z.B. gemäß WO 2015/131976, Spiro-Tribenzotropolone, z.B. gemäß der noch nicht offen gelegten Anmeldung PCT/EP2015/002225, Spirobifluorene mit meta-Phenyldiamingruppen, z.B. gemäß der noch nicht offen gelegten Anmeldung PCT/EP2015/002112, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I), (II), (III) oder (IV) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Beispiele

### A) Synthesebeispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

Nachfolgend werden die [4.4.4]-Propellane der Übersichtlichkeit halber in Form einer Newman-Projektion dargestellt, wie dies auch in der Literatur üblich ist, s. z.B. M. Kimura et al., Bull. Chem. Soc. Jpn., 2006, 79, 11, 1793.

### A: Synthese der Synthone

### Beispiel S1:

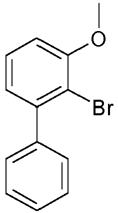

Ein Gemisch aus 31.3 g (100 mmol) 1-Brom-2-iod-6-methoxybenzol [74128-84-0], 12.2 g (100 mmol) Benzolboronsäure [98-80-6], g (300 mmol) Trikaliumphosphat, g (1 mmol) Tetrakis-triphenylphosphinopalladium(0) , 200 ml Toluol, 80 ml Dioxan und 200 ml Wasser wird 16 h unter Rückfluss erhitzt. Nach Erkalten trennt man die wässrige Phase ab, wäscht die organische Phase dreimal mit je 200 ml Wasser, einmal mit 200 ml gesättigter Kochsalzlösung und trocknet dann über Magnesiumsulfat. Man filtriert vom Trockenmittel ab, engt die organische Phase zur Trockene ein und kugelrohr-destilliert das verbliebene Öl im Hochvakuum. Ausbeute: 16.1 g (61 mmol), 61 %; Reinheit: 97 % ig n. ¹H-NMR.

### Beispiel S10:

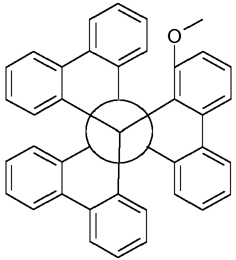

Aus einer auf -78 °C gekühlten Lösung von 26.3 g (100 mmol) S1 in 1000 ml Diethylether und 40 ml (100 mmol) n-Butyllithium, 2.5 M in n-Hexan wird das entsprechende Lithiumreagenz hergestellt. Man fügt unter gutem Rühren 34.4 g (100 mmol) Spiro[9H-fluoren-9,9'(10'H)-phenanthren]-10'-on [1749-36-6] zu, rührt 30 min bei -78 °C nach, lässt dann langsam auf Raumtemperatur erwärmen und rührt 24 h nach. Man stoppt die Reaktion durch Zugabe von 200 ml Wasser und 200 ml gesättigter Ammoniumchlorid-Lösung, trennt die org. Phase ab und trocknet über Magnesiumsulfat. Man filtriert vom Trockenmittel ab, entfernt das Lösungsmittel im Vakuum, nimmt den Rückstand in 500 ml in Eisessig auf und homogenisiert die Mischung unter Erwärmen. Dann gibt man unter gutem Rühren 1 konz. Schwefelsäure und 20 g Phosphorpentoxid zu und erhitzt 2 h unter Rückfluss. Man lässt auf 80 °C erkalten, tropft dann langsam ohne weiteres Heizen 400 ml Wasser zu, saugt vom ausgefallenen Feststoff ab, wäscht dreimal mit je 200 ml Wasser und einmal mit 100 ml Methanol und trocknet im Vakuum. Ausbeute: 38.9 g (76 mmol), 76 %; Reinheit: 97 % ig n. ¹H-NMR.

Analog können folgende Verbindungen dargestellt werden:

| Bsp. | Bromid | Produkt | Ausbeute |
|---|---|---|---|
| S11 | 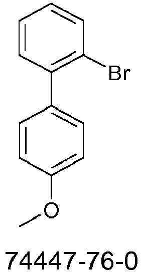 | 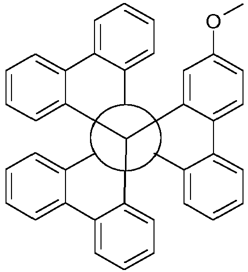 | 84 % |
| S12 | 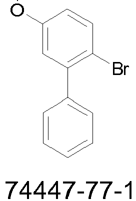 | 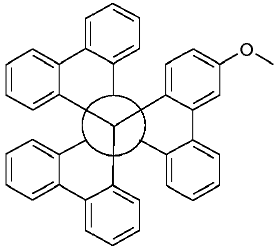 | 80 % |
| S13 | 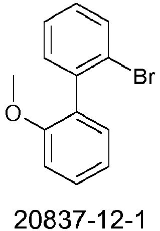 | 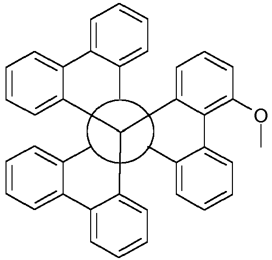 | 73 % |
| S14 | 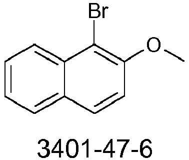 | 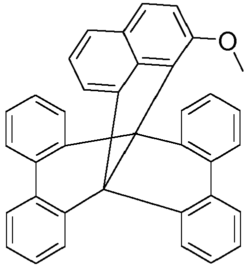 | 38 % |
| S15 | 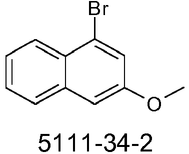 | 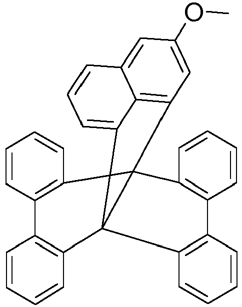 | 43 % |
| S16 | 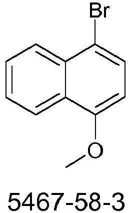 | 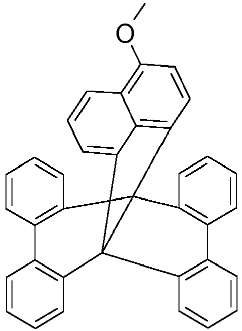 | 45 % |
| S17 | 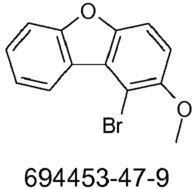 | 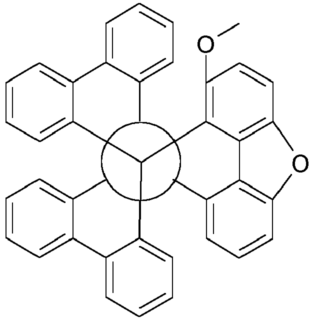 | 48 % |
| S18 | 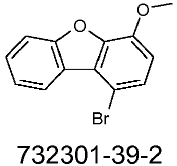 | 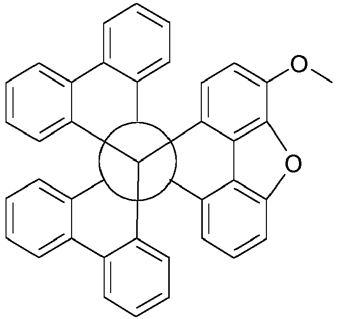 | 43 % |

### Beispiel S30:

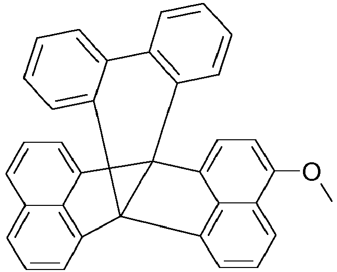

Ein Gemisch aus 3.1 g (10 mmol) 3-Methoxy-acenaphth[1,2-a] acenaphthylen [1088585-98-1], 20.4 g (100 mmol) lodbenzol [591-50-4], 11.1 g (80 mmol) Kaliumcarbonat, 6.5 g (20 mmol) Tetra-n-butylammoniumbromid und 112 mg (0.5 mmol) Palladium(II)acetat in 100 ml DMF wird in einem Rührautoklaven unter Argon 10 Tage bei 120 °C gerührt. Nach Erkalten wird die Reaktionsmischung mit 1000 ml Dichlormethan verdünnt und über ein vorgeschlämmtes Kieselgelbett filtriert. Das Filtrat wird im Vakuum vom Dichlormethan und dann im Hochvakuum vom überschüssigen lodbenzol befreit. Der Rückstand wird flashchromatographisch an einem Säulenautomaten (Torrent der a. A. Semrau) getrennt. Ausbeute: 2.6 g (5.6 mmol), 56 %; Reinheit: 95 % ig n. ¹H-NMR.

### Beispiel S50:

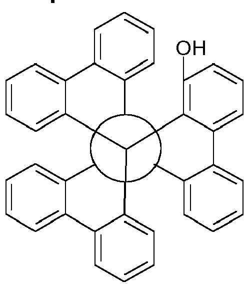

Ein Gemisch aus 51.2 g (100 mmol) S10 und 250 g Pyridiniumhydrochlorid wird 6 h auf 200 °C erhitzt. Man lässt die Reaktionsmischung auf 100 °C erkalten und tropft ohne weiteres Heizen vorsichtig unter gutem Rühren 1000 ml Wasser und dann 50 ml 2 N Salzsäure zu. Nach Erkalten auf ca. 30 °C saugt man vom ausgefallenen Feststoff ab, wäscht diesen dreimal mit je 200 ml Wasser und einmal mit 100 ml Methanol und trocknet im Vakuum. Ausbeute: 38.8 g (73 mmol), 73 %; Reinheit: 97 % ig n. ¹H-NMR.

Analog können folgende Verbindungen dargestellt werden:

| Bsp. | Edukt | Produkt | Ausbeute |
|---|---|---|---|
| S51 | S11 | 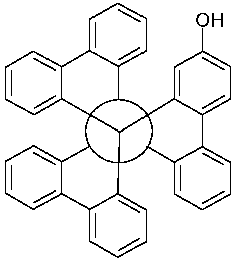 | 87 % |
| S52 | S12 | 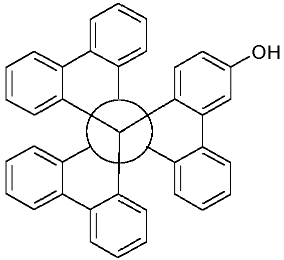 | 85 % |
| S53 | S13 | 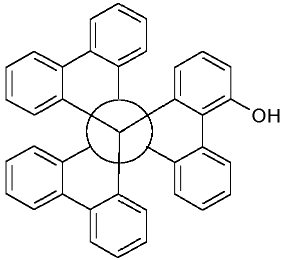 | 79 % |
| S54 | S14 | 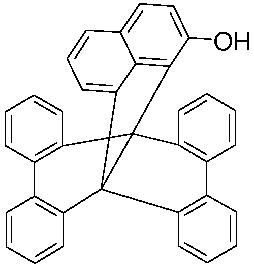 | 80 % |
| S55 | S15 | 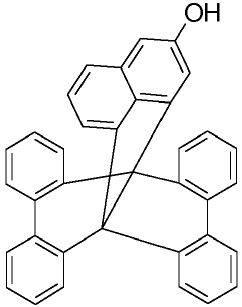 | 87 % |
| S56 | S16 | 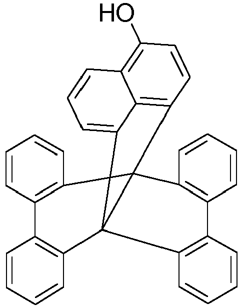 | 85 % |
| S57 | S30 | 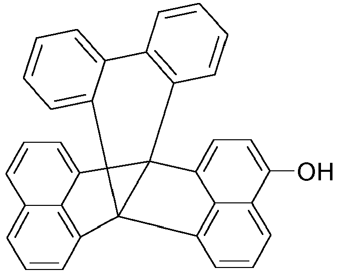 | 90 % |
| S58 | S17 | 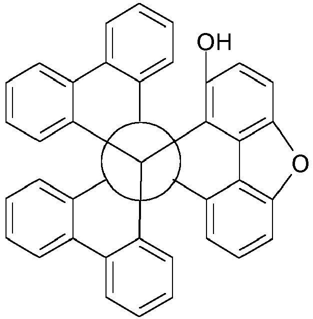 | 88 % |
| S59 | S18 | 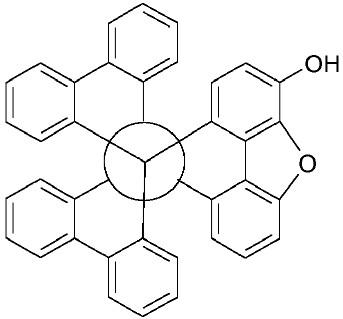 | 94 % |

### Beispiel S100:

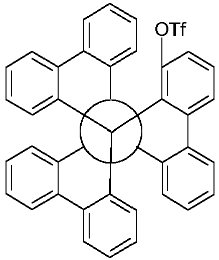

Ein auf 0 °C gekühltes Gemisch aus 49.7 g (100 mmol) S50 und 24.2 ml (300 mmol) Pyridin wird tropfenweise während 1 h mit 25.2 ml (150 mol) Trifluormethan-sulfonsäureanhydrid O(Tf)₂ [358-23-6] versetzt. Man rührt 1 h bei 0 °C nach, lässt dann auf Raumtemperatur erwärmen und rührt 12 h nach. Man gießt das Reaktionsgemisch unter sehr gutem Rührern in 1 l Eiswasser, rührt 10 min. nach und extrahiert dann dreimal mit je 300 ml Dichlormethan. Die vereinigten org. Phasen werden einmal mit 300 ml ges. Kochsalzlösung gewaschen und dann über Magnesiumsulfat getrocknet. Nach Abfiltrieren des Trockenmittels und Entfernen das Lösungsmittels wird der Rückstand aus Acetonitril umkristallisiert. 50.3 g (80 mmol), 80 %; Reinheit: 97 % ig n. ¹H-NMR.

Analog können folgende Verbindungen dargestellt werden:

| Bsp. | Bromid | Produkt | Ausbeute |
|---|---|---|---|
| S101 | S51 | 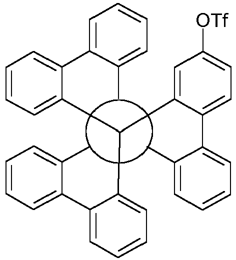 | 93 % |
| S102 | S52 | 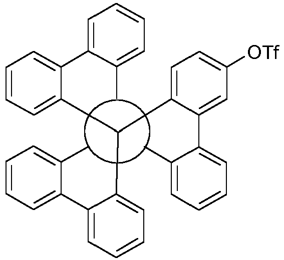 | 90 % |
| S103 | S53 | 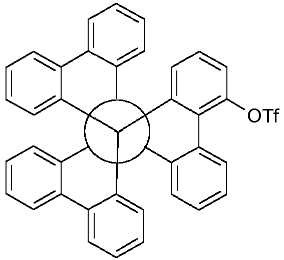 | 85 % |
| S104 | S54 | 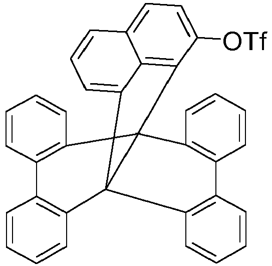 | 70 % |
| S105 | S55 | 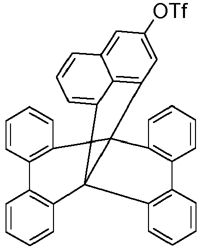 | 87 % |
| S106 | S56 | 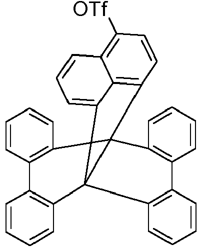 | 85 % |
| S107 | S57 | 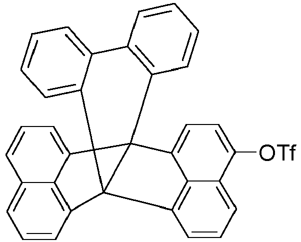 | 92 % |
| S108 | S58 | 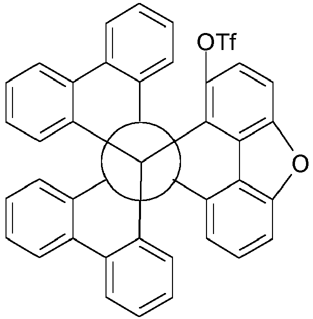 | 77 % |
| S109 | S59 | 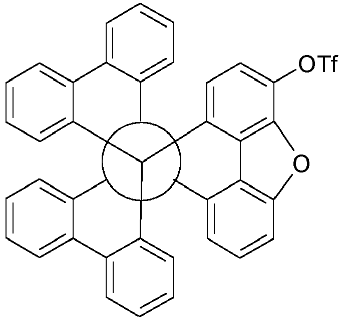 | 87 % |

### Beispiel S150:

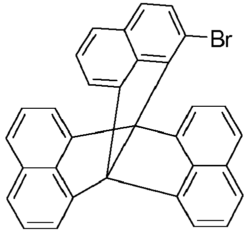

Aus 23.8 g (100 mmol) 1-Bromnaphthalin [90-11-9] und 2.4 g (100 mmol) Magnesiumspänen in 800 ml THF wird das entsprechende Grignardreagenz hergestellt. Man fügt unter gutem Rühren 13.1 g (50 mmol) 3-Brom-1,2-acenaphthylendion [21170-55-8] zu und rührt 4 h bei 50 °C. Nach Erkalten auf Raumtemperatur quenscht man durch vorsichtige Zugabe von 20 ml Wasser. Man entfernt das THF weitgehend im Vakuum, nimmt den Rückstand in 1500 ml Toluol auf, wäscht zweimal mit Wasser, einmal mit gesättigter Kochsalzlösung. Zur Trocknung entfernt man am Wasserabscheider 500 ml Toluol, gibt dann tropfenweise unter gutem Rühren 10 ml Trifluormethan-sulfonsäure zu und erhitzt die Reaktionsmischung weitere 16 h am Wasserabscheider. Dann destilliert man weitere 800 ml Toluol ab,lässt auf ca. 50 °C erkalten, tropft 500 ml Metnanol zu, rührt 30 min. nach, saugt vom Feststoff ab, wäscht diesen dreimal mit je 100 ml Methanol und trocknet im Vakuum. Ausbeute: 8.2 g (17 mmol), 34 %; Reinheit: 97 % ig n. ¹H-NMR.

Analog können folgende Verbindungen dargestellt werden:

| Bsp. | Edukt | Produkt | Ausbeute |
|---|---|---|---|
| S151 | 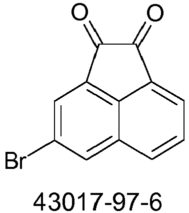 | 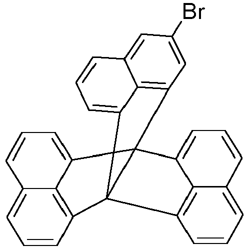 | 44 % |
| S152 | 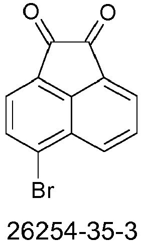 | 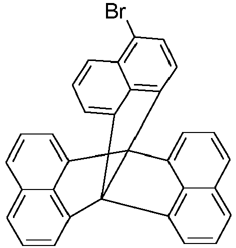 | 46 % |
| S153 | 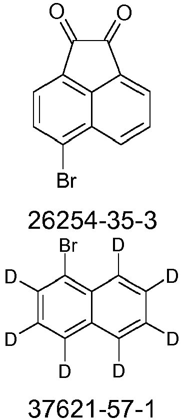 | 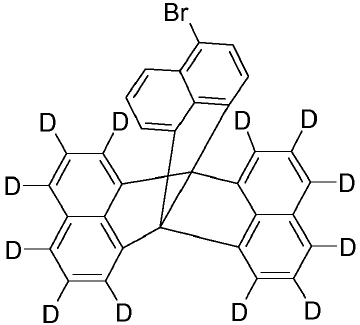 | 44 % |

### B: Erfindungsgemäße Verbindungen

### Beispiel H1

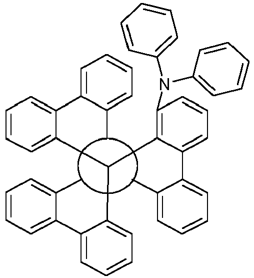

Ein Gemisch aus 62.8 g (100 mmol) S100, 18.6 g (110 mmol) Diphenylamin, 13.5 g (120 mmol) Natrium-tert-butylat, 449 mg (2 mmol) Palladiumacetat, 1230 mg (3 mmol) S-Phos und 800 ml Toluol wird 16 h unter Rückfluss erhitzt. Man setzt 1 ml Hydratzinhydrat zu und erhitzt 1 h unter Rüchfluss. Die Reaktionsmischung wird bei 50 °C unter Rühren mit 400 ml Wasser versetzt, dann trennt man die org. Phase ab und filtriert noch warm über ein mit Toluol vorgeschlämmtes Celit-Bett. Das Filtrat wird im Vakuum auf ca. 100 ml eingeengt und dann in der Wärme unter gutem Rühren mit 200 ml Methanol versetzt. Man lässt unter Rühren erkalten, saugt vom auskristallisierten Produkt ab und wäscht dieses dreimal mit je 100 ml Methanol. Die weitere Reinigung erfolgt durch fünfmalige Heißextraktion mit Toluol (Vorlagemenge 250 ml) und anschließende zweimalige fraktionierte Sublimation im Vakuum (T ca. 300 °C, p ca. 10⁻⁵ mbar). Ausbeute: 45.0 g (68 mmol), 68 %; Reinheit: ca. 99.9 % ig n. HPLC.

Analog können folgende Verbindungen dargestellt werden:

| Bsp. | Ed u kte | Produkt | Ausbeute |
|---|---|---|---|
| H2 | 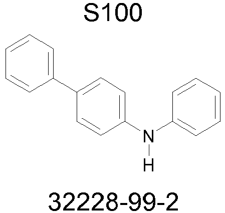 | 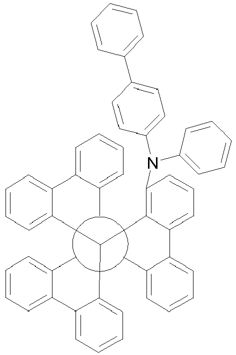 | 55 % |
| H3 | 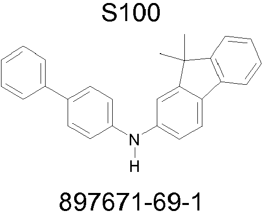 | 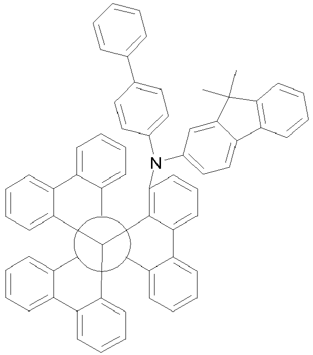 | 46 % |
| H4 | 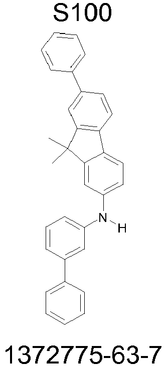 | 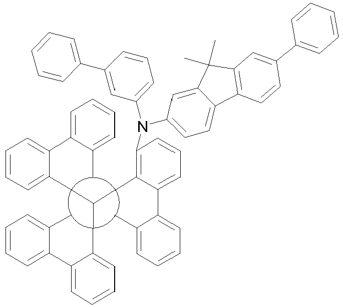 | 43 % |
| H5 | 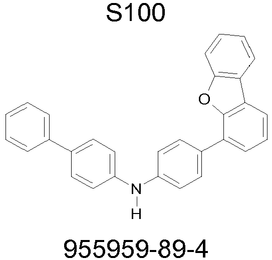 | 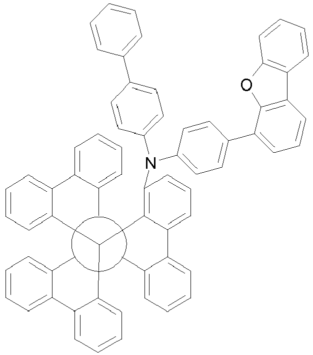 | 48 % |
| H6 | 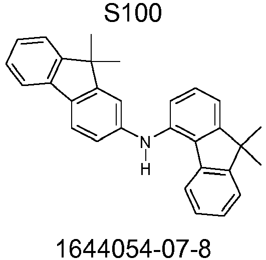 | 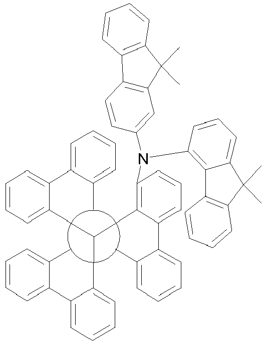 | 40 % |
| H7 | 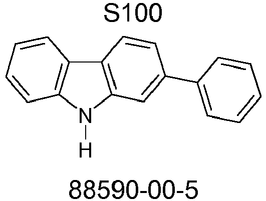 | 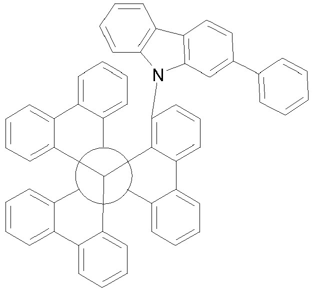 | 52 % |
| H8 | 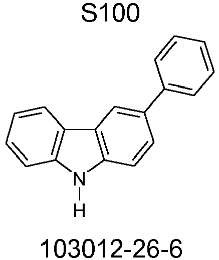 | 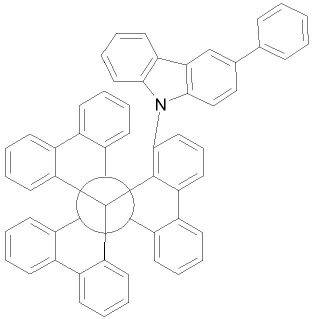 | 49 % |
| H9 | 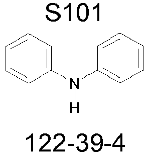 | 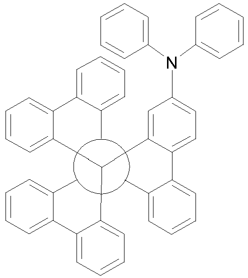 | 75 % |
| H10 | 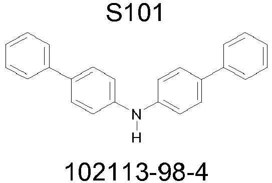 | 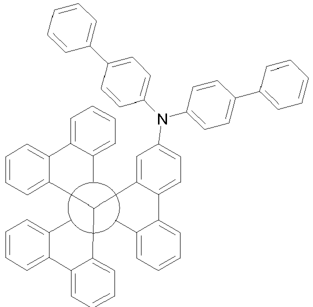 | 73 % |
| H11 | 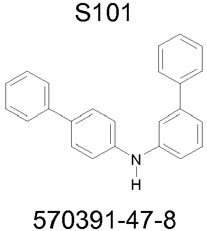 | 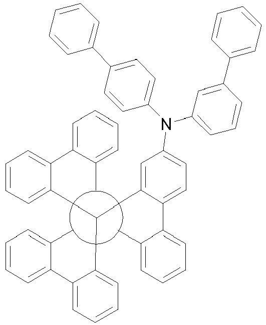 | 75 % |
| H12 | 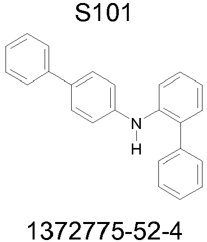 | 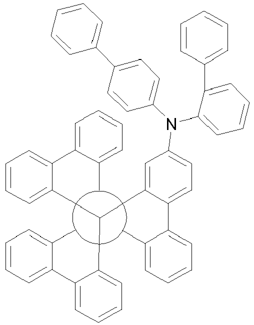 | 70 % |
| H13 | 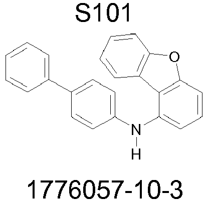 | 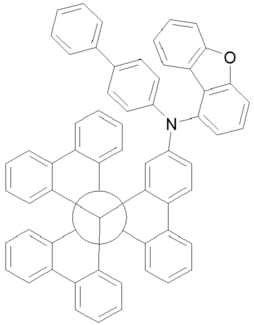 | 68 % |
| H14 | 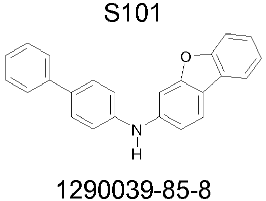 | 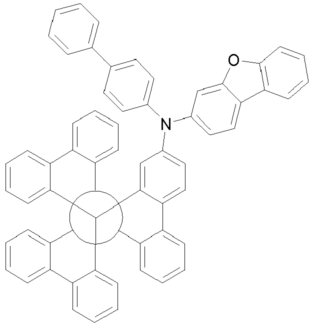 | 74 % |
| H15 | 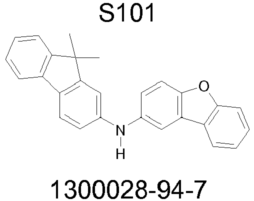 | 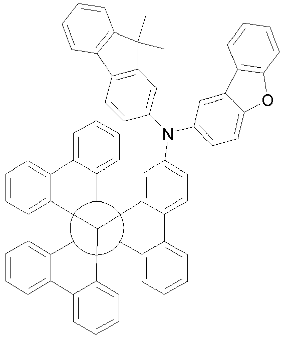 | 70 % |
| H16 | 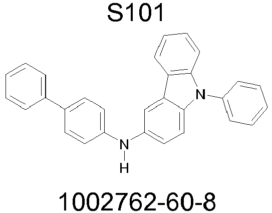 | 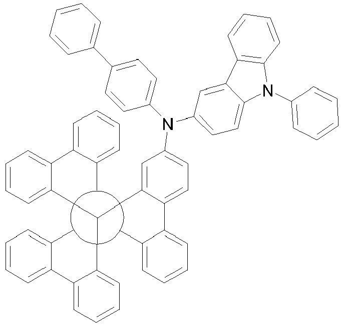 | 71 % |
| H17 | 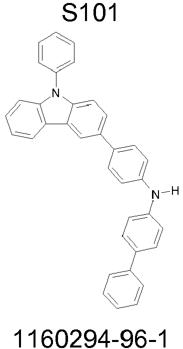 | 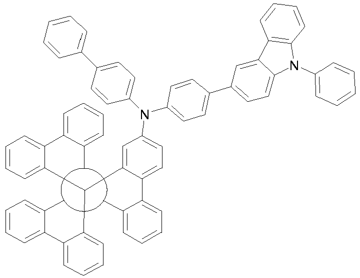 | 69 % |
| H18 | 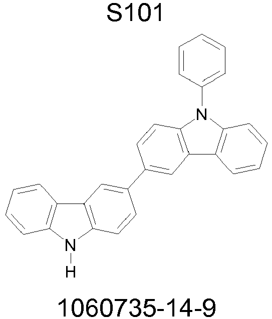 | 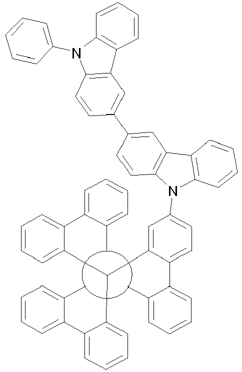 | 76 % |
| H19 | 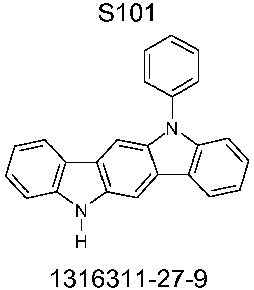 | 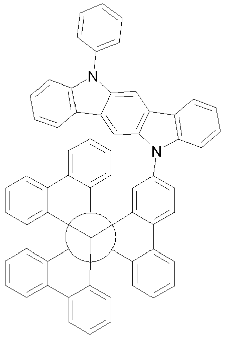 | 70 % |
| H20 | 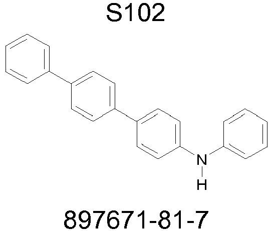 | 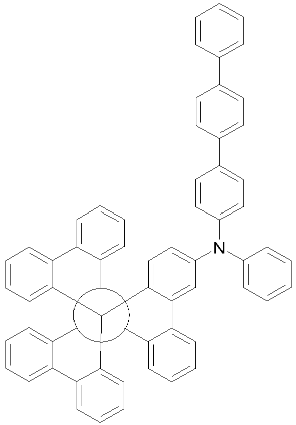 | 78 % |
| H21 | 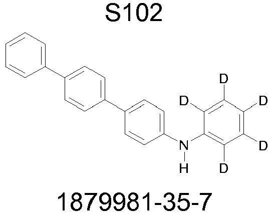 | 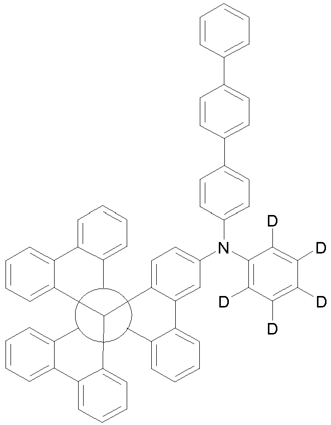 | 75 % |
| H22 | 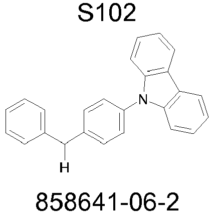 | 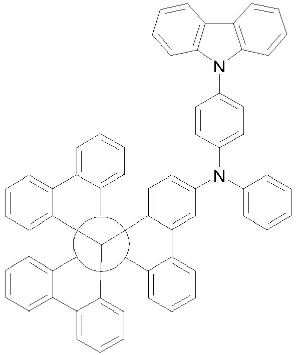 | 73 % |
| H23 | 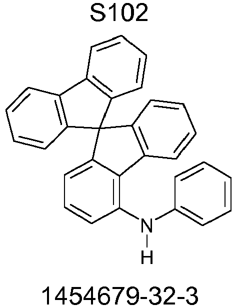 | 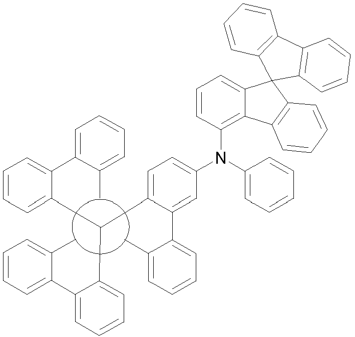 | 70 % |
| H24 | 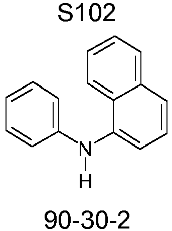 | 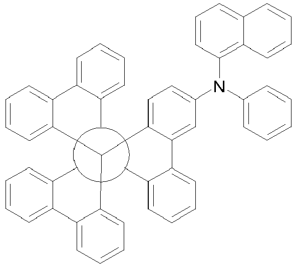 | 74 % |
| H25 | 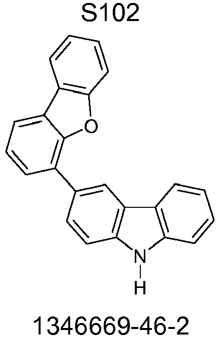 | 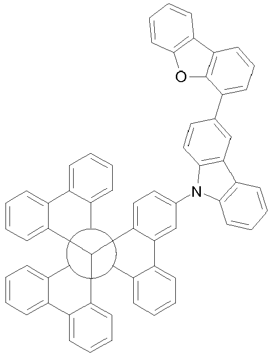 | 71 % |
| H26 | 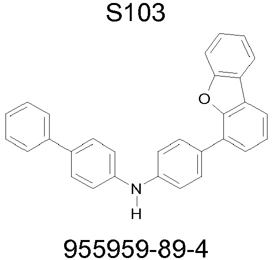 | 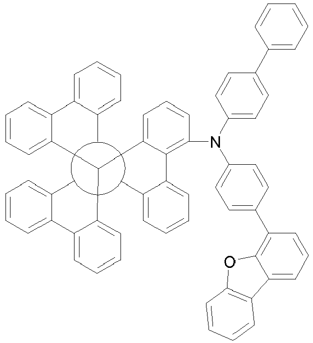 | 68 % |
| H27 | 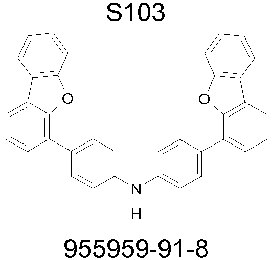 | 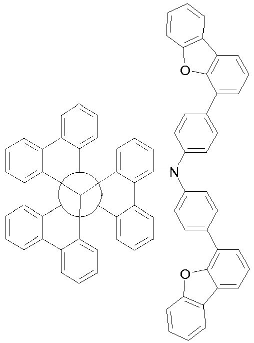 | 65 % |
| H28 | 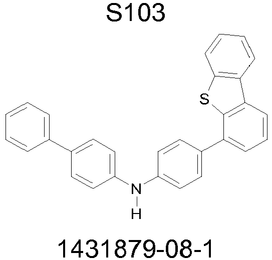 | 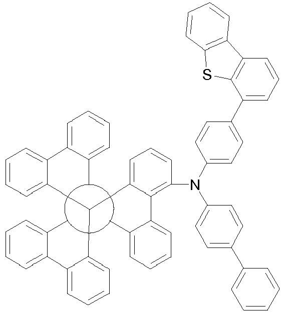 | 67 % |
| H29 | 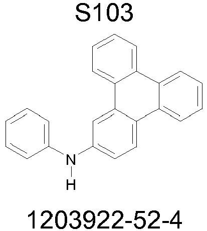 | 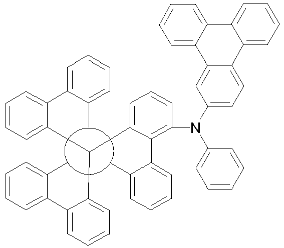 | 63 % |
| H30 | 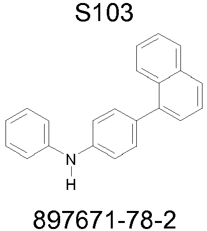 | 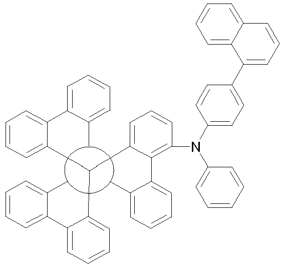 | 65 % |
| H31 | 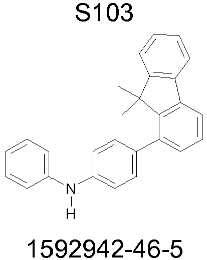 | 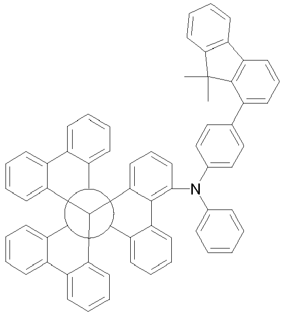 | 66 % |
| H32 | 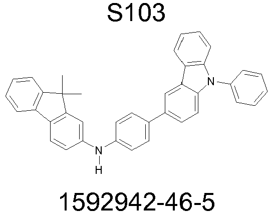 | 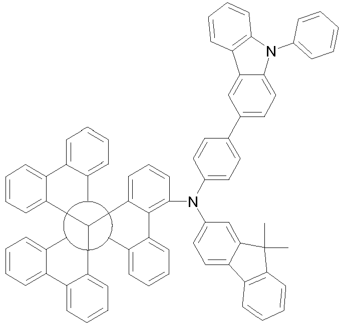 | 65 % |
| H33 | 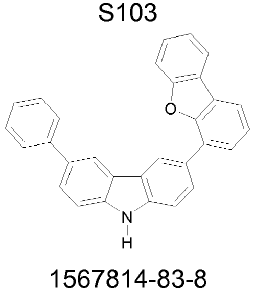 | 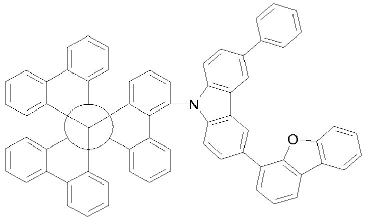 | 67 % |
| H34 | 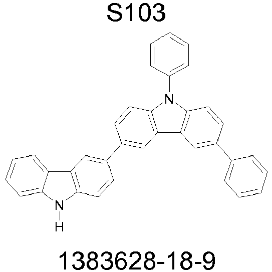 | 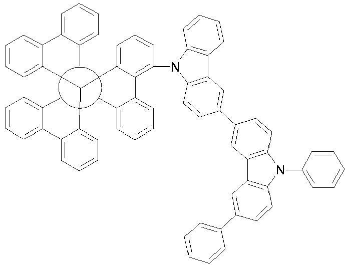 | 62 % |
| H35 | 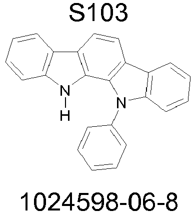 | 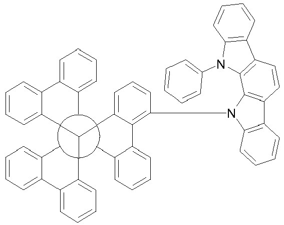 | 46 % |
| H36 | 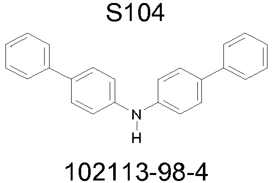 | 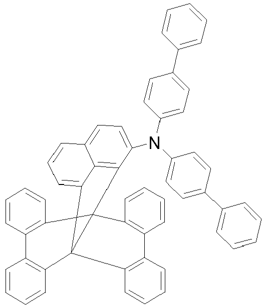 | 39 % |
| H37 | 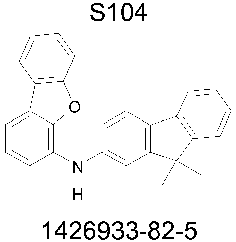 | 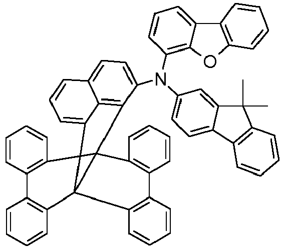 | 41 % |
| H38 | 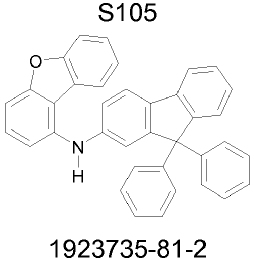 | 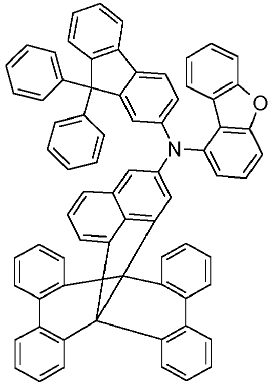 | 65 % |
| H39 | 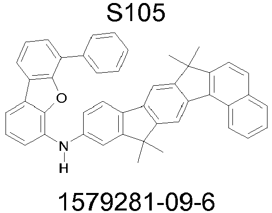 | 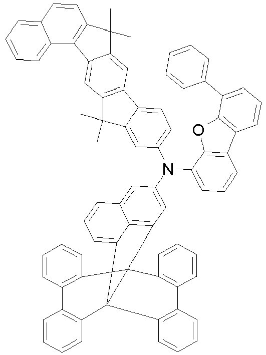 | 69 % |
| H40 | 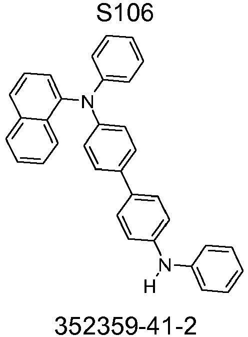 | 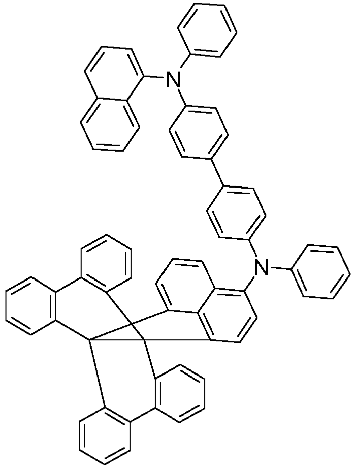 | 65 % |
| H41 | 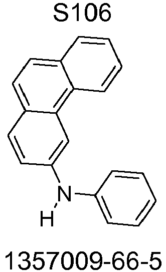 | 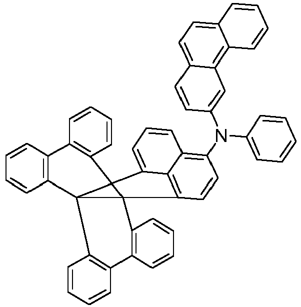 | 62 % |
| H42 | 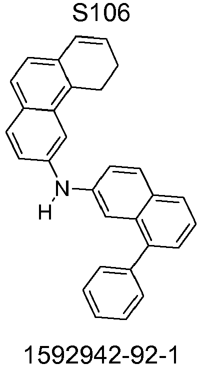 | 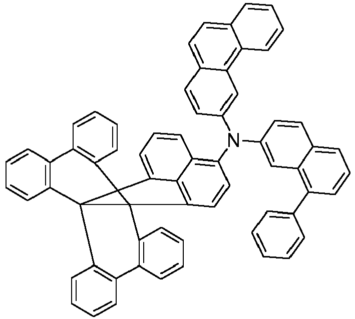 | 68 % |
| H43 | 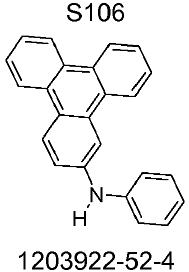 | 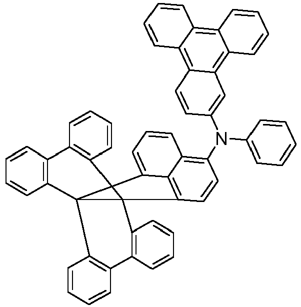 | 67 % |
| H44 | 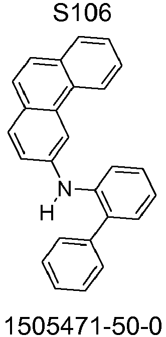 | 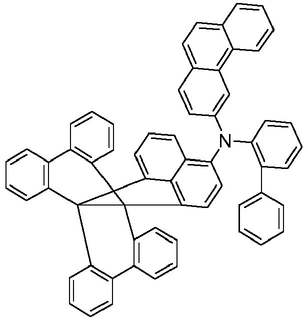 | 69 % |
| H45 | 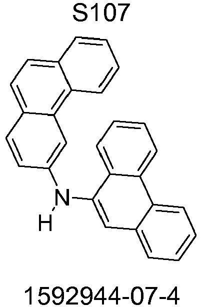 | 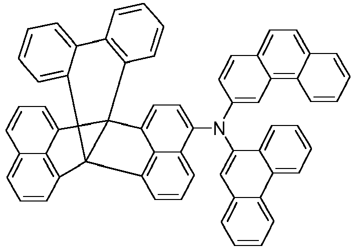 | 66 % |
| H46 | 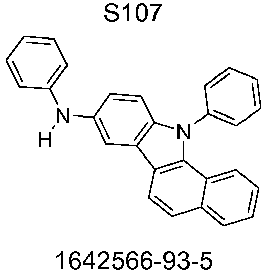 | 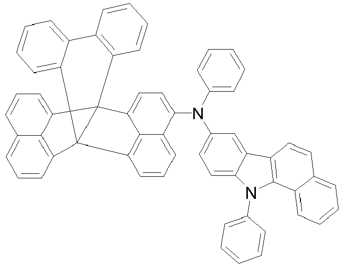 | 67 % |
| H47 | 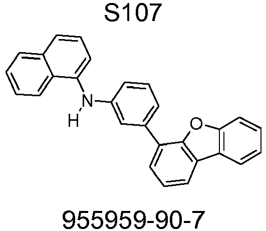 | 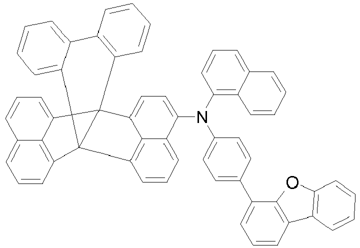 | 65 % |
| H48 | 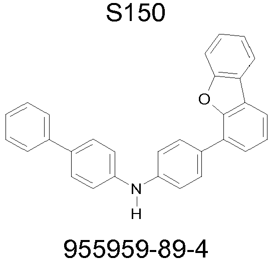 | 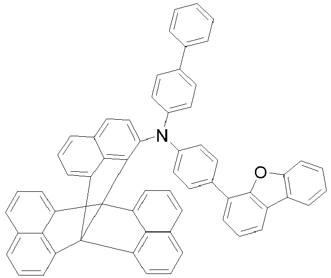 | 40 % |
| H49 | 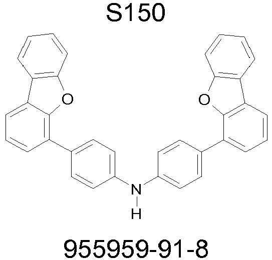 | 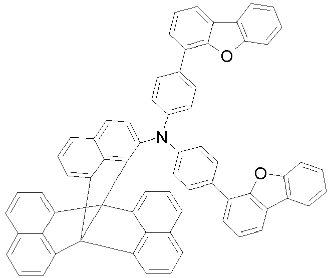 | 39 % |
| H50 | 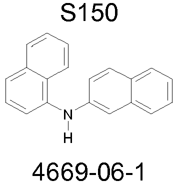 | 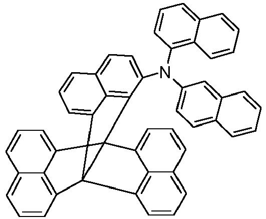 | 42 % |
| H51 | 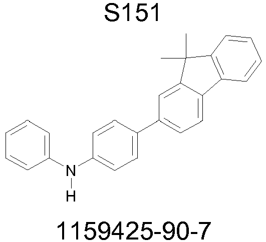 | 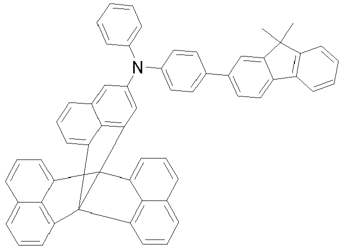 | 68 % |
| H52 | 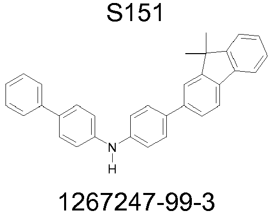 | 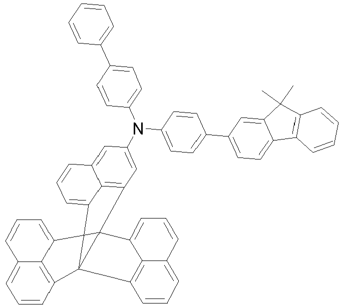 | 72 % |
| H53 | 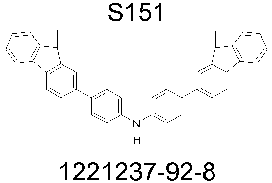 | 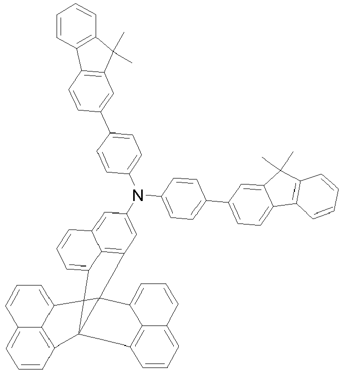 | 70 % |
| H54 | 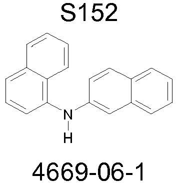 | 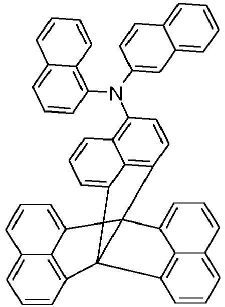 | 67 % |
| H55 | 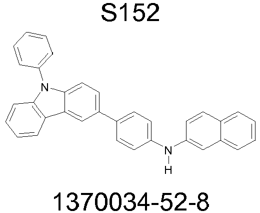 | 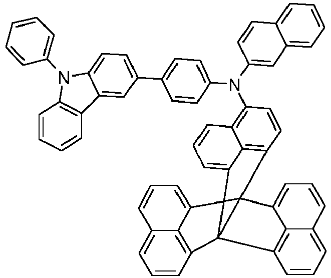 | 65 % |
| H56 | 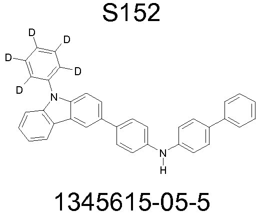 | 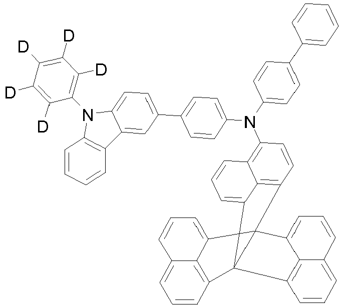 | 65 % |
| H57 | 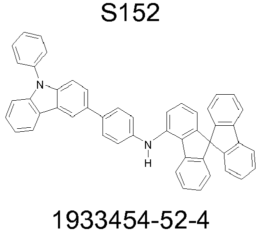 | 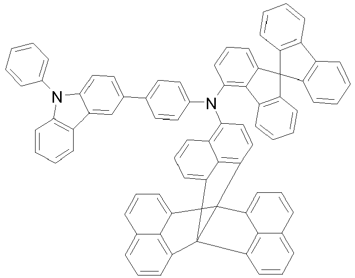 | 53 % |
| H58 | 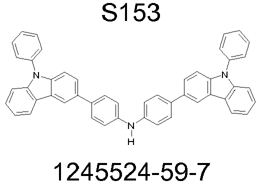 | 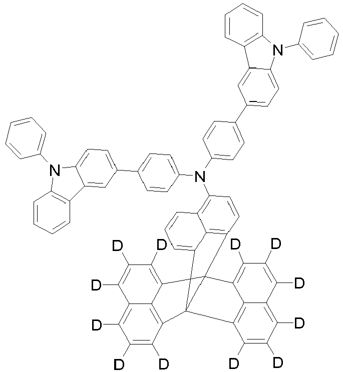 | 68 % |
| H59 | 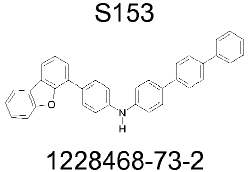 | 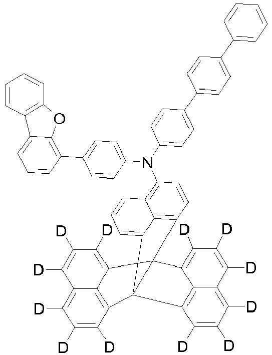 | 67 % |
| H60 | 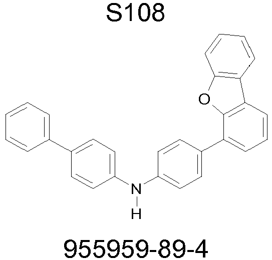 | 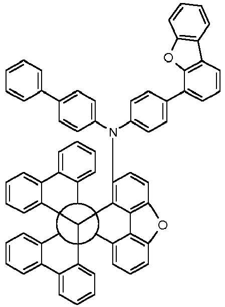 | 37 % |
| H61 | 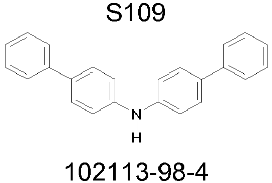 | 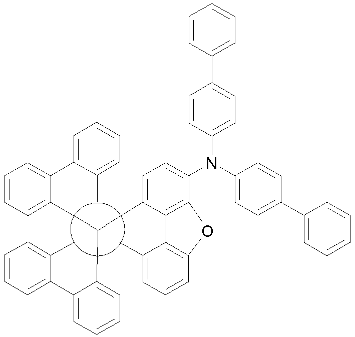 | 87 % |

### Beispiel H100

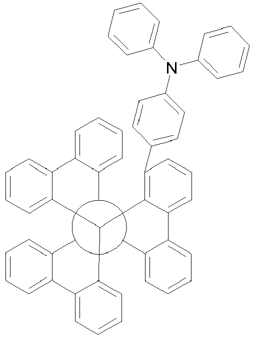

Ein Gemisch aus 62.8 g (100 mmol) S100, 37.1 g (110 mmol) *N,N-*Diphenylamino-4-(4,4,5,5-tetramethyl-1,3,2-dioxaborolan-2-yl)-benzol [267221-88-5], 63.7 g (300 mmol) Trikaliumphosphat, 820 mg (2 mmol) S-Phos, 225 mg (1 mmol) Palladiumacetat, 400 ml Toluol, 200 ml Dioxan und 400 ml Wasser wird 20 h unter Rückfluss erhitzt. Man setzt 1 ml Hydrazinhydrat zu und erhitzt 1 h unter Rüchfluss. Man lässt auf 60 °C erkalten, trennt die wässrige Phase ab, wäscht die org. Phase zweimal mit je 300 ml Wasser, einmal mit 300 ml ges. Kochsalzlösung und filtriert noch warm über ein mit Toluol vorgeschlämmtes Celit-Bett. Das Filtrat wird im Vakuum auf ca. 100 ml eingeengt und dann in der Wärme unter gutem Rühren mit 200 ml Methanol versetzt. Man lässt unter Rühren erkalten, saugt vom auskristallisierten Produkt ab und wäscht dieses dreimal mit je 100 ml Methanol. Die weitere Reinigung erfolgt durch fünfmalige Heißextraktion mit Butylacetat (Vorlagemenge 250 ml) und anschließende zweimalige fraktionierte Sublimation im Vakuum (T ca. 310 °C, p ca. 10⁻⁵ mbar). Ausbeute: 31.9 g (44 mmol), 44 %; Reinheit: ca. 99.9 % ig n. HPLC.

Analog können folgende Verbindungen dargestellt werden:

| Bsp. | Edukte | Produkt | Ausbeute |
|---|---|---|---|
| H101 | 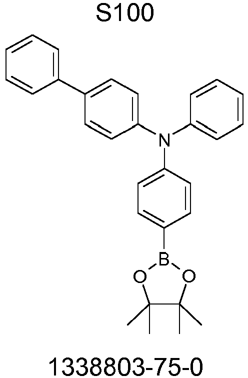 | 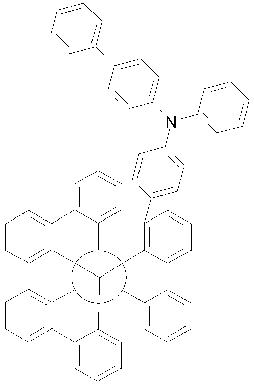 | 40 % |
| H102 | 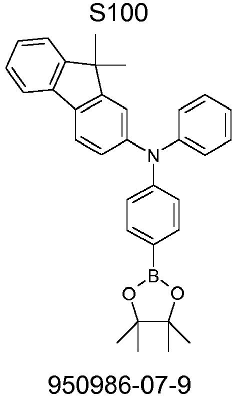 | 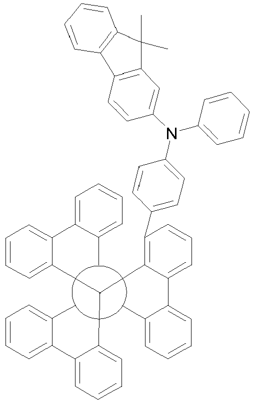 | 45 % |
| H103 | 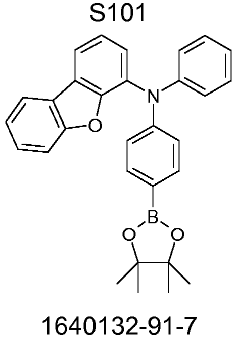 | 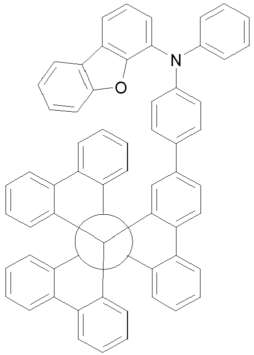 | 73 % |
| H104 | 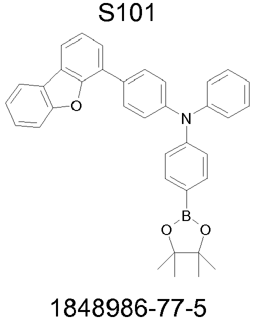 | 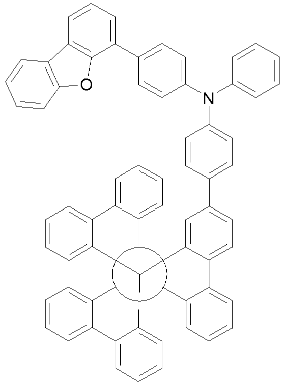 | 70 % |
| H105 | 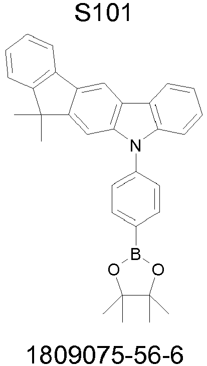 | 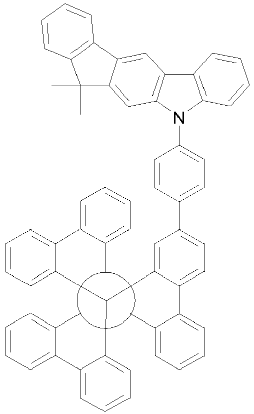 | 70 % |
| H106 | 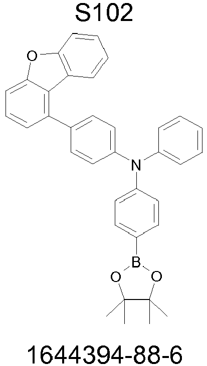 | 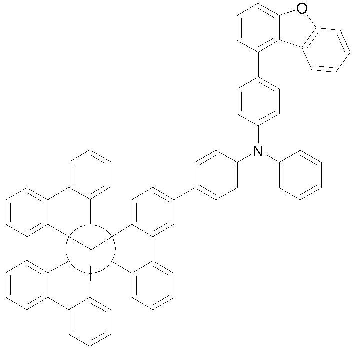 | 74 % |
| H107 | 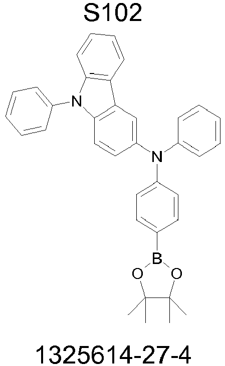 | 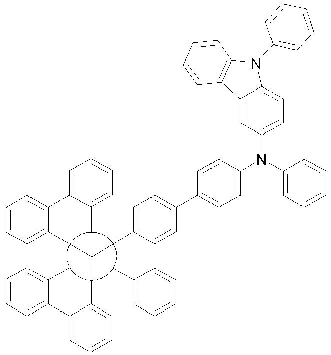 | 72 % |
| H108 | 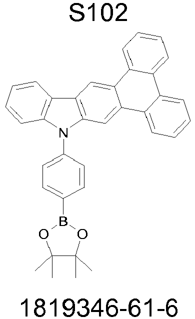 | 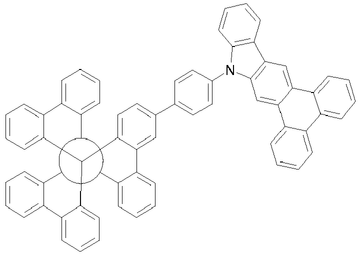 | 70 % |
| H109 | 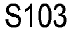 | 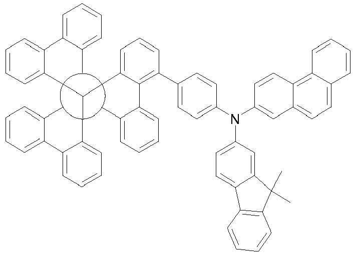 | 58 % |
| | 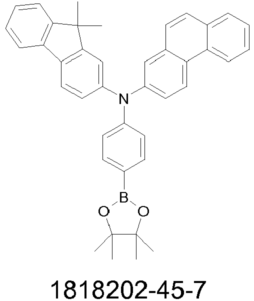 | | |
| H110 | 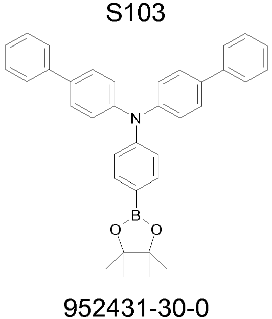 | 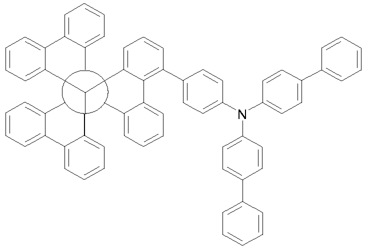 | 63 % |
| H111 | 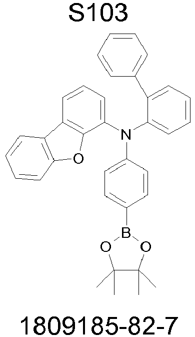 | 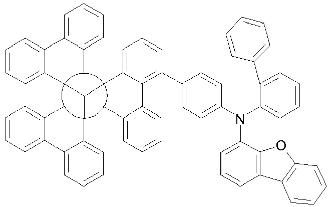 | 60 % |
| H112 | 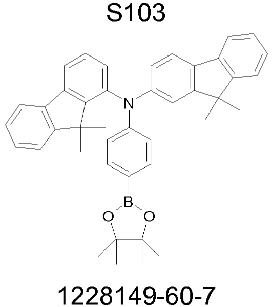 | 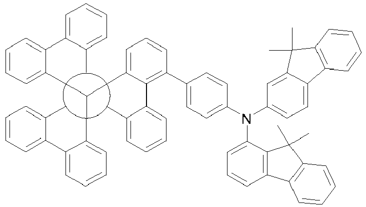 | 61 % |
| H113 | 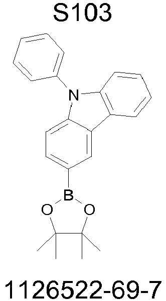 | 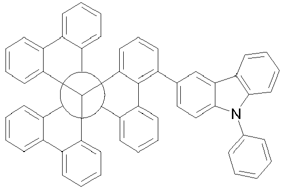 | 66 % |
| H114 | 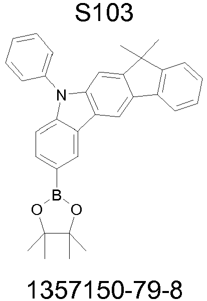 | 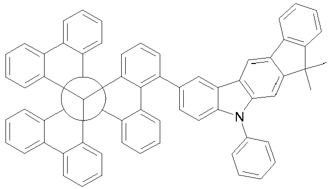 | 63 % |
| H115 | 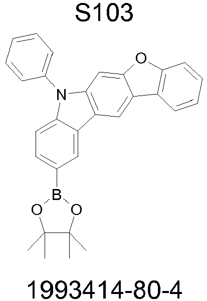 | 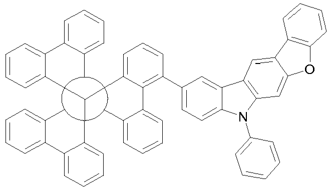 | 58 % |
| H116 | 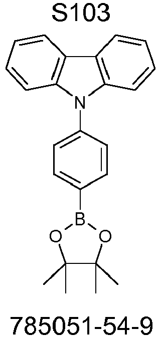 | 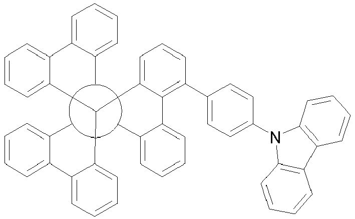 | 68 % |
| H117 | 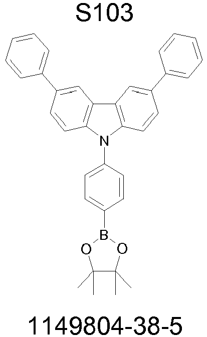 | 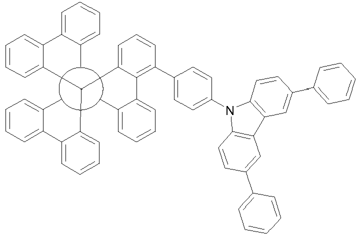 | 65 % |
| H118 | 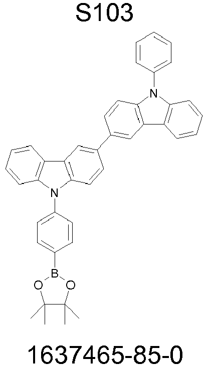 | 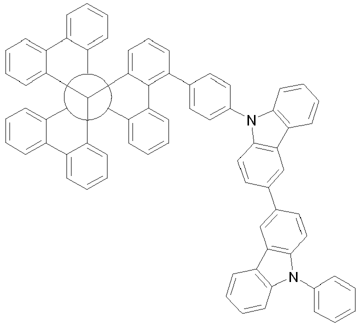 | 66 % |
| H119 | 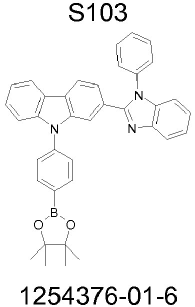 | 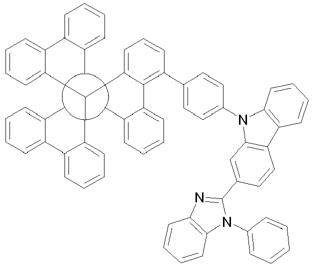 | 64 % |
| H120 | 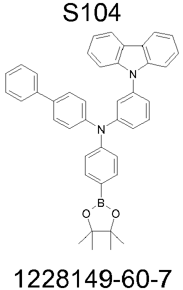 | 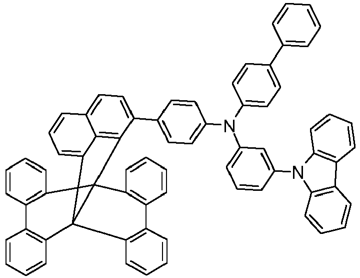 | 38 % |
| H121 | 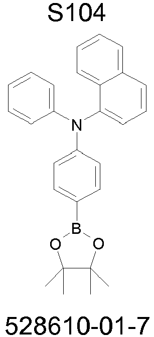 | 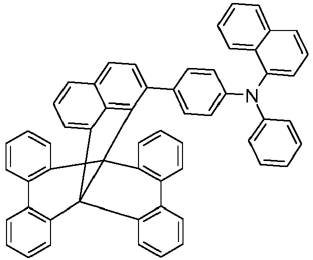 | 41 % |
| H122 | 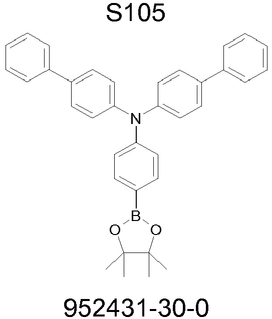 | 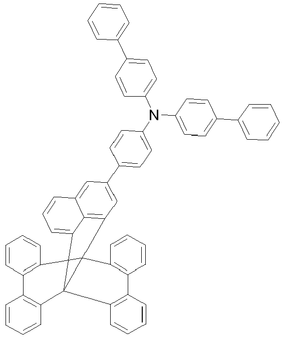 | 70 % |
| H123 | 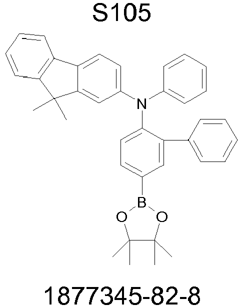 | 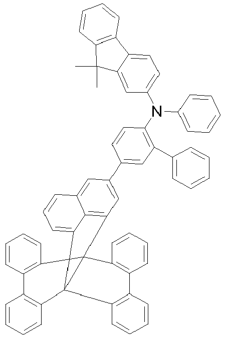 | 68 % |
| H124 | 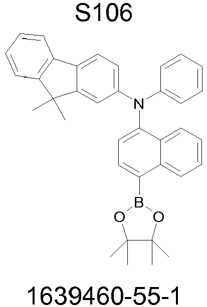 | 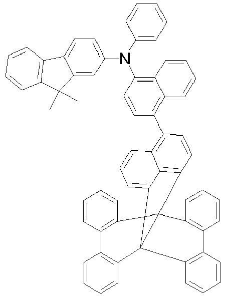 | 73 % |
| H125 | 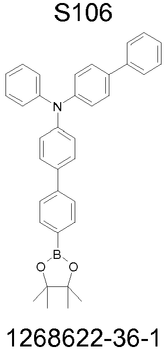 | 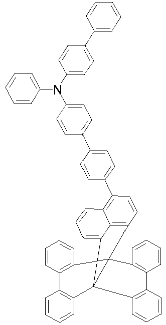 | 70 % |
| H126 | 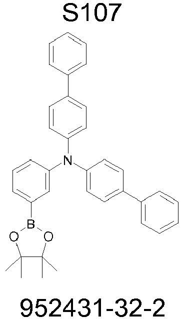 | 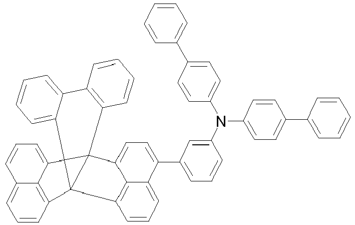 | 67 % |
| H127 | 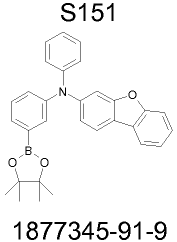 | 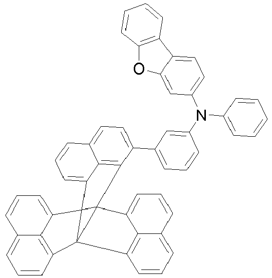 | 65 % |
| H128 | 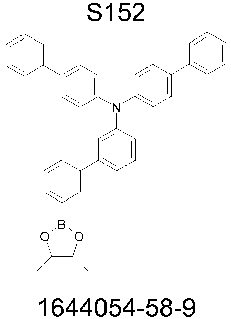 | 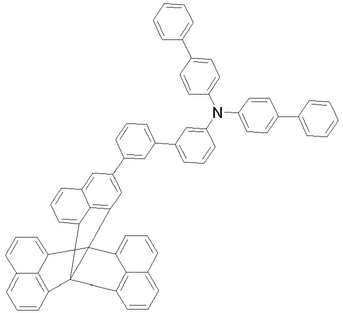 | 69 % |
| H129 | 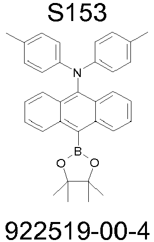 | 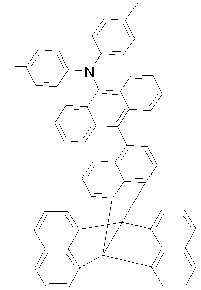 | 49 % |
| H130 | 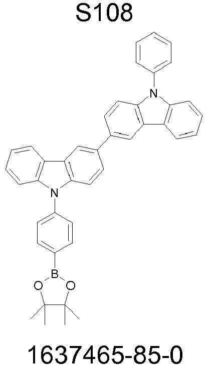 | 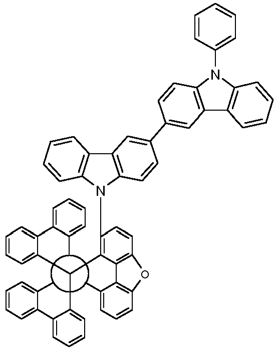 | 33 % |
| H131 | 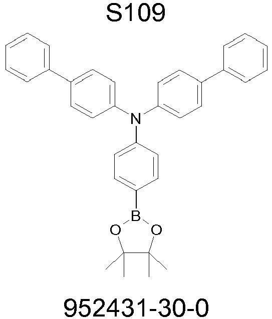 | 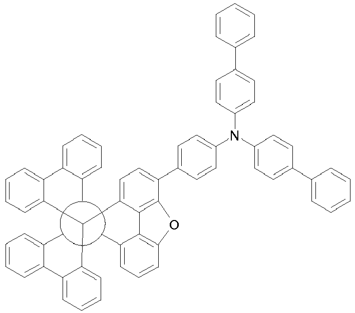 | 71 % |

### Beispiel E1:

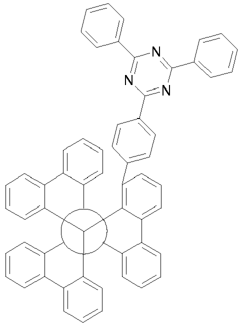

Ein Gemisch aus 62.8 g (100 mmol) S100, 47.9 g (110 mmol) 2,4-Diphenyl-6-[4-(4,4,5,5-tetramethyl-1,3,2-dioxaborolan-2-yl)phenyl]-1,3,5-triazin [1219956-23-6], 63.7 g (300 mmol) Trikaliumphosphat, 820 mg (2 mmol) S-Phos, 225 mg (1 mmol) Palladiumacetat, 400 ml Toluol, 200 ml Dioxan und 400 ml Wasser wird 20 h unter Rückfluss erhitzt. Man lässt auf 60 °C erkalten, trennt die wässrige Phase ab, wäscht die org. Phase zweimal mit je 300 ml Wasser, einmal mit 300 ml ges. Kochsalzlösung und filtriert noch warm über ein mit Toluol vorgeschlämmtes Celit-Bett. Das Filtrat wird im Vakuum auf ca. 100 ml eingeengt und dann in der Wärme unter gutem Rühren mit 200 ml Methanol versetzt. Man lässt unter Rühren erkalten, saugt vom auskristallisierten Produkt ab und wäscht dieses dreimal mit je 100 ml Methanol. Die weitere Reinigung erfolgt durch fünfmalige Heißextraktion mit Toluol (Vorlagemenge 250 ml) und anschließende zweimalige fraktionierte Sublimation im Vakuum (T ca. 320 °C, p ca. 10⁻⁵ mbar). Ausbeute: 38.6 g (49 mmol), 49 %; Reinheit: ca. 99.9 % ig n. HPLC.

Analog können folgende Verbindungen dargestellt werden:

| | | | |
|---|---|---|---|
| Bsp. | Ed u kte | Produkt | Ausbeute |
| E2 | 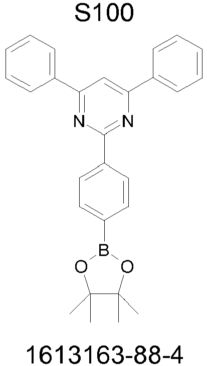 | 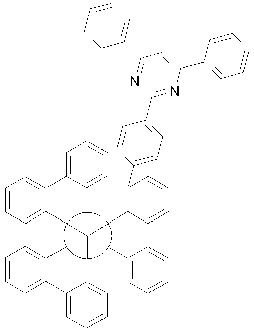 | 46 % |
| E3 | 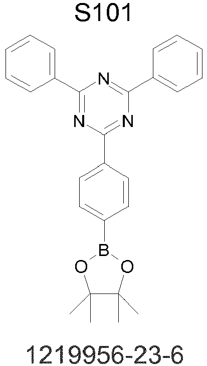 | 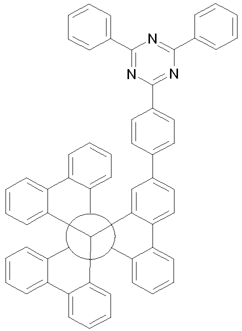 | 72 % |
| E4 | 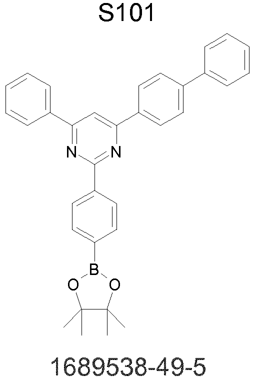 | 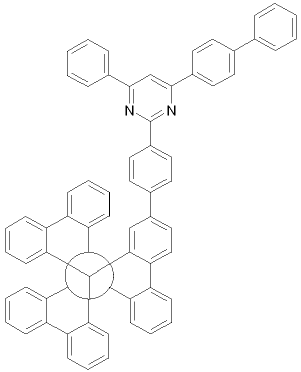 | 70 % |
| E5 | 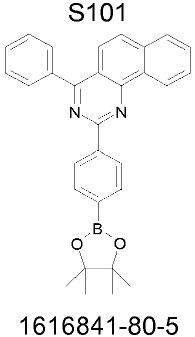 | 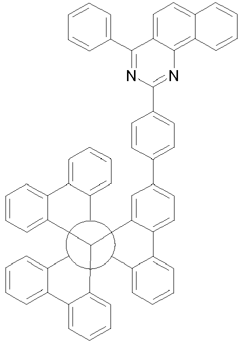 | 68 % |
| E6 | 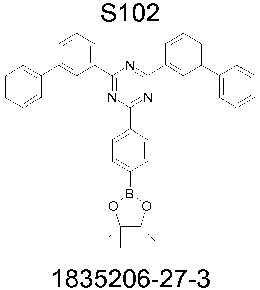 | 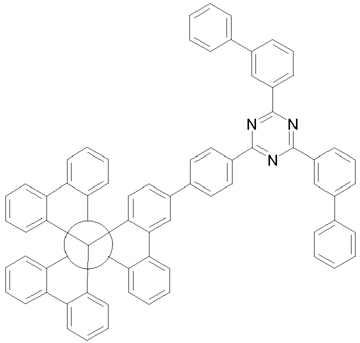 | 75 % |
| E7 | 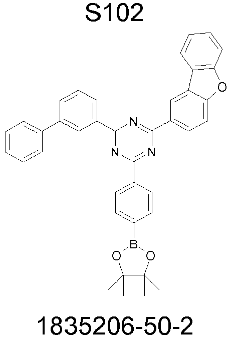 | 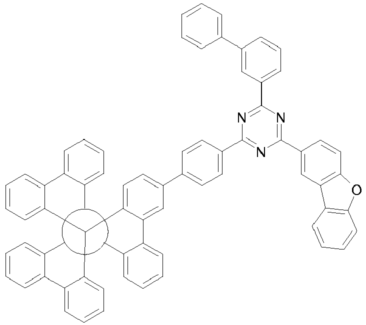 | 77 & |
| E8 | 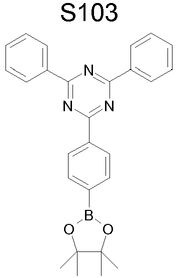 | 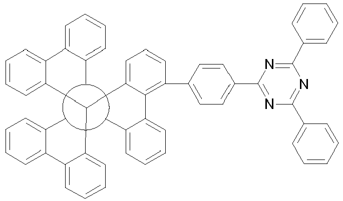 | 64 % |
| | 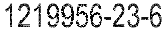 | | |
| E9 | 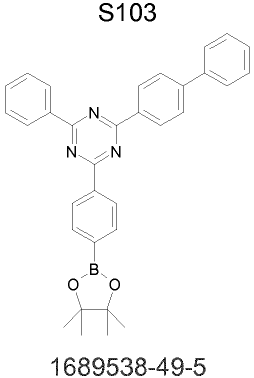 | 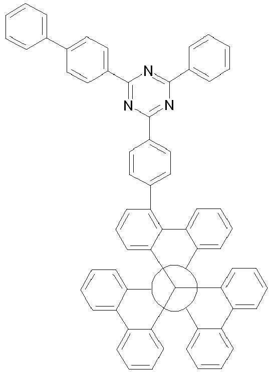 | 67 % |
| E10 | 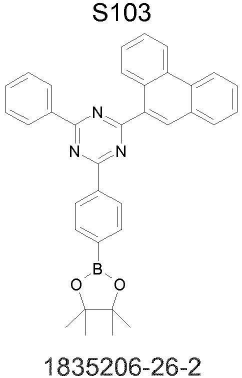 | 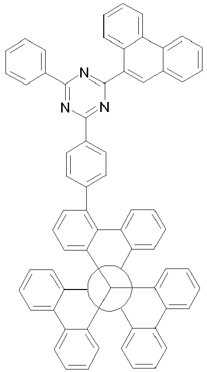 | 63 % |
| E11 | 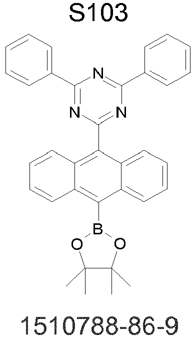 | 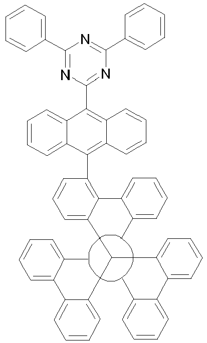 | 60 % |
| E12 | 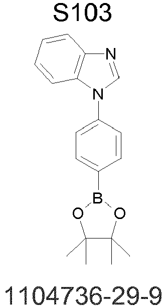 | 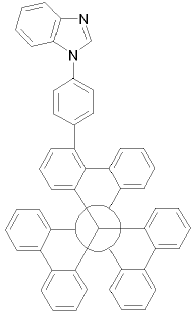 | 63 % |
| E13 | 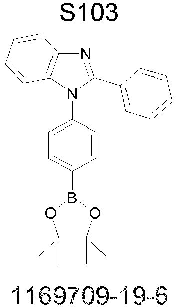 | 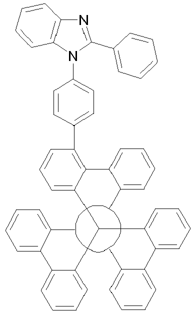 | 61 % |
| E14 | 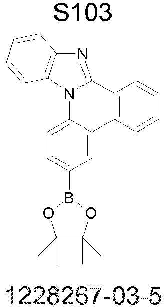 | 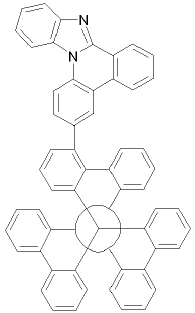 | 67 % |
| E15 | 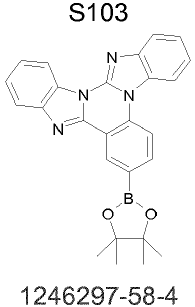 | 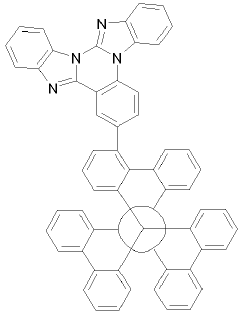 | 65 % |
| E16 | 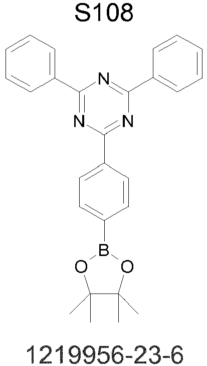 | 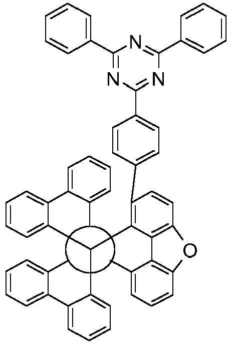 | 38 % |
| E17 | 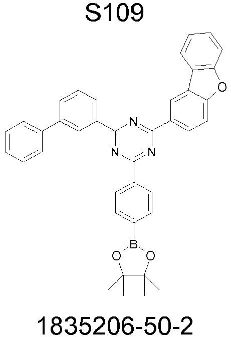 | 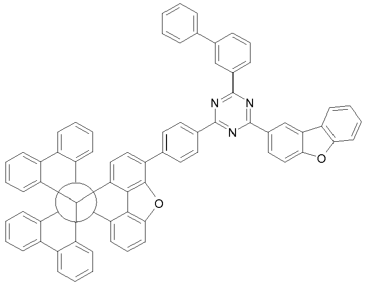 | 69 % |

### B) Devicebeispiele

### 1) Allgemeine Beschreibung der Herstellung und Vermessung der OLEDs

In den folgenden Beispielen werden die Herstellung und die Device-Daten verschiedener OLEDs vorgestellt.

Die OLEDs können wie folgt hergestellt werden: Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 25 Minuten mit UV-Ozon vorbehandelt (UV-Ozon Generator PR-100, Firma UVP), innerhalb 30 min zur verbesserten Prozessierung mit 25 nm PEDOT:PSS beschichtet (bezogen als CLEVIOS™ P VP AI 4083 von Heraeus Precious Metals GmbH Deutschland, aus wässriger Lösung aufgeschleudert), und anschließend bei 180°C 10 min lang ausgeheizt.

Diese beschichteten Glasplättchen bilden die Substrate, auf welche die OLEDs aufgebracht werden.

Die OLEDs haben folgenden Schichtaufbau: Substrat / Lochtransportschicht 1 (HTL1) bestehend aus der Verbindung HTM (s. Tabelle 3) dotiert mit 5 % NDP-9 (kommerziell erhältlich von der Fa. Novaled), 20 nm / Lochtransportschicht 2 (HTL2) / optionale Elektronenblockierschicht (EBL) / Emissionsschicht (EML) / Lochblockierschicht (HBL) / Elektronentransportschicht (ETL) und abschließend eine Kathode. Die Kathode wird durch eine 100 nm dicke Aluminiumschicht gebildet. Die genaue Zusammensetzung der Schichten HTL1, EBL (falls vorhanden), EML, HBL und ETL ist in den folgenden Tabellen gezeigt.

Zur Herstellung der OLEDs werden die entsprechend in einer Schicht verwendeten Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Co-Verdampfung in einem bestimmten Volumenanteil beigemischt wird. Eine Angabe wie M1:M2:Ir1 (55%:35%:10%) bedeutet hierbei, dass das Material M1 in einem Volumenanteil von 55%, M2 in einem Anteil von 35% und Ir1 in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 3 gezeigt.

Die OLEDs werden standardmäßig charakterisiert. Hierfür werden die Elektrolumineszenzspektren, die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. EQE1000 bezeichnet die externe Quanteneffizienz bei einer Betriebsleuchtdichte von 1000 cd/m².

Als Lebensdauer LD80 wird die Zeit definiert, nach der die Leuchtdichte bei Betrieb mit einem konstantem Strom von 40 mA/cm² auf 80% der Startleuchtdichte absinkt.

### 2) Verwendung der erfindungsgemäßen Verbindungen in einer Lochtransportschicht

Die folgenden Beispiele zeigen, dass die erfindungsgemäßen Verbindungen als Lochtransportmaterialien in einer Lochtransportschicht verwendet werden können.

In den Beispielen D1 bis D11 und D14 werden die erfindungsgemäßen Verbindungen H3, H5, H6, H9, H10, H13, H26 und H104 als Lochtransportmaterialien in grün emittierenden Triplett-OLEDs (Emitter jeweils Ir1 oder Ir2) verwendet.

In den Beispielen D18 bis D24 werden die erfindungsgemäßen Verbindungen H9, H37, H47, H49 und H131 als Lochtransportmaterialien in gelb emittierenden Triplett-OLEDs (Emitter jeweils Ir3 oder Ir4) verwendet.

| **Bsp.** | **HTL2 Dicke** | **EML Dicke** | **HBL Dicke** | **ETL Dicke** |
|---|---|---|---|---|
| D1 | H9 | M1:Ir1 | ETM1 | ETM1:ETM2 |
| | 40nm | (85%:15%) | 10nm | (50%:50%) |
| | | 30nm | | 30nm |
| D2 | s.o. | M1:Ir2 | s.o. | s.o. |
| | | (85%:15%) | | |
| | | 30nm | | |
| D3 | s.o. | M1:M2:Ir1 | s.o. | s.o. |
| | | (45%:45%:10%) | | |
| | | 30nm | | |
| D4 | s.o. | M1:M2:Ir2 | s.o. | s.o. |
| | | (45%:45%:10%) | | |
| | | 30nm | | |
| D5 | H3 | M1:M2:Ir1 | s.o. | s.o. |
| | 40nm | (45%:45%:10%) | | |
| | | 30nm | | |
| D6 | H5 | s.o. | s.o. | s.o. |
| | 40nm | | | |
| D7 | H6 | s.o. | s.o. | s.o. |
| | 40nm | | | |
| D8 | | M1:M2:Ir1 | s.o. | s.o. |
| | H10 40nm | (35%:55%:10%) | | |
| | | 30nm | | |
| D9 | H13 | M1:M2:Ir1 | s.o. | s.o. |
| | 40nm | (45%:40%:15%) | | |
| | | 30nm | | |
| D10 | H20 | M1:M2:Ir2 | s.o. | s.o. |
| | 40nm | (40%:45%:15%) | | |
| | | 30nm | | |
| D11 | H26 | s.o. | s.o. | s.o. |
| | 40nm | | | |
| D14 | H104 | s.o. | s.o. | s.o. |
| | 40nm | | | |
| D18 | H9 | M1:Ir3 | s.o. | s.o. |
| | 40nm | (80%:20%) | | |
| | | 30nm | | |
| D19 | s.o. | M1:Ir4 | s.o. | s.o. |
| | | (85%:15%) | | |
| | | 30nm | | |
| D20 | H131 | M1:Ir3 | s.o. | s.o. |
| | 40nm | (80%:20%) | | |
| | | 30nm | | |
| D22 | H37 | M1:Ir4 | s.o. | s.o. |
| | 40 | (85%:15%) | | |
| | nm | 30nm | | |
| D23 | H47 | M1:Ir4 | s.o. | s.o. |
| | 40 | (85%:15%) | | |
| | nm | 30nm | | |
| D24 | H49 | M1:Ir4 | s.o. | s.o. |
| | 40 | (85%:15%) | | |
| | nm | 30nm | | |

Bei den betreffenden OLEDs werden hervorragende Leistungsdaten gemessen, die im Folgenden gezeigt sind:

| **Bsp.** | **EQE1000 (%)** | **U1000 (V)** | **CIE x/y** | **LD80 (h)** |
|---|---|---|---|---|
| D1 | 20.3 | 3.1 | 0.33/0.64 | 120 |
| D2 | 19.2 | 3.1 | 0.36/0.62 | 150 |
| D3 | 20.5 | 3.0 | 0.33/0.64 | 140 |
| D4 | 19.5 | 3.0 | 0.36/0.62 | 180 |
| D5 | 20.7 | 3.0 | 0.33/0.64 | 240 |
| D6 | 20.4 | 2.9 | 0.33/0.64 | 230 |
| D7 | 20.9 | 3.0 | 0.33/0.64 | 210 |
| D8 | 20.5 | 3.0 | 0.33/0.64 | 240 |
| D9 | 20.1 | 3.1 | 0.33/0.63 | 260 |
| D10 | 19.3 | 3.0 | 0.36/0.62 | 430 |
| D11 | 19.7 | 3.0 | 0.36/0.62 | 470 |
| D14 | 19.7 | 3.1 | 0.37/0.62 | 460 |
| D18 | 17.0 | 2.9 | 0.39/0.59 | 300 |
| D19 | 19.1 | 3.0 | 0.46/0.53 | 360 |
| D20 | 17.6 | 3.0 | 0.40/0.58 | 520 |
| D22 | 19.0 | 2.8 | 0.46/0.52 | 380 |
| D23 | 19.5 | 2.9 | 0.46/0.53 | 370 |
| D24 | 19.7 | 2.9 | 0.46/0.53 | 410 |

### 3) Verwendung der erfindungsgemäßen Verbindungen in einer Lochtransportschicht und als Matrixmaterial in einer emittierenden Schicht

Die folgenden Beispiele zeigen, dass die erfindungsgemäßen Verbindungen als Lochtransportmaterialien in einer Lochtransportschicht und zusätzlich in derselben OLED auch als Matrixmaterialien in einer emittierenden Schicht verwendet werden können.

In den Beispielen D12 und D13 wird jeweils die erfindungsgemäße Verbindung H26 als Lochtransportmaterial eingesetzt, Zusätzlich ist in der emittierenden Schicht die erfindungsgemäße Verbindung H32 bzw. H34 als Co-Host (h-type TMM) zusammen mit dem weiteren Matrixmaterial M1 vorhanden. Als Triplett-Emitter wird jeweils der grüne Emitter Ir-2 eingesetzt.

| **Bsp.** | **HTL2 Dicke** | **EML Dicke** | **HBL Dicke** | **ETL Dicke** |
|---|---|---|---|---|
| D12 | H26 | M1:H32:Ir2 | ETM1 | ETM1:ETM2 |
| | 40nm | (40%:45%:15%) | 10nm | (50%:50%) |
| | | 30nm | | 30nm |
| D13 | s.o. | M1:H34:Ir2 | s.o. | s.o. |
| | | (40%:45%:15%) | | |
| | | 30nm | | |

Bei den betreffenden OLEDs werden hervorragende Leistungsdaten gemessen, die im Folgenden gezeigt sind:

| **Bsp.** | **EQE1000 (%)** | **U1000 (V)** | **CIE x/y** | **LD80 (h)** |
|---|---|---|---|---|
| D12 | 19.8 | 2.9 | 0.37/0.62 | 450 |
| D13 | 19.4 | 3.1 | 0.36/0.62 | 480 |

### 4) Verwendung der erfindungsgemäßen Verbindungen in der Lochblockierschicht und der Elektronentransportschicht, in einigen Fällen zusätzlich auch in der emittierenden Schicht, der Elektronenblockierschicht und der Lochtransportschicht

Das Beispiel D15 zeigt, dass die erfindungsgemäßen Verbindungen als Materialien der Lochblockierschicht und zusätzlich in derselben OLED auch als Materialien der Elektronentransportschicht verwendet werden können.

In Beispiel D15 wird jeweils die erfindungsgemäße Verbindung E3 als Lochblockiermaterial eingesetzt, Zusätzlich ist in der Elektronentransportschicht dieselbe Verbindung E3 in Mischung mit dem weiteren Elektronentransportmaterial ETM2 vorhanden. Als Triplett-Emitter wird der grüne Emitter Ir-2 eingesetzt.

| **Bsp.** | **HTL2 Dicke** | **EBL Dicke** | **EML Dicke** | **HBL Dicke** | **ETL Dicke** |
|---|---|---|---|---|---|
| D15 | HTM | --- | M1:M2:Ir2 | E3 | E3:ETM2 |
| | 40nm | | (45%:45%:10%) | 10nm | (50%:50%) |
| | | | 30nm | | 30nm |

Bei der betreffenden OLED werden hervorragende Leistungsdaten gemessen, die im Folgenden gezeigt sind:

| **Bsp.** | **EQE1000 (%)** | **U1000 (V)** | **CIE x/y** | **LD80 (h)** |
|---|---|---|---|---|
| D15 | 19.9 | 2.9 | 0.37/0.62 | 390 |

In den Beispielen D16, D17 und D21 werden erfindungsgemäße Verbindungen zusätzlich in der Lochtransportschicht (D16, D17) eingesetzt. In Beispiel D21 sind erfindungsgemäße Verbindungen sogar in allen Schichten der OLED von HTL bis ETL vorhanden:

| **Bsp.** | **HTL2 Dicke** | **EBL Dicke** | **EML Dicke** | **HBL Dicke** | **ETL Dicke** |
|---|---|---|---|---|---|
| D16 | H26 | --- | M1:M2:Ir2 | E3 | E3:ETM2 |
| | 40nm | | (45%:45%:10%) | 10nm | (50%:50%) |
| | | | 30nm | | 30nm |
| D17 | H26 | --- | M1:M2:Ir2 | E8 | E8:ETM2 |
| | 40nm | | (45%:45%:10%) | 10nm | (50%:50%) |
| | | | 30nm | | 30nm |
| D21 | H26 | H131 | M1:H34:Ir3 | E8 | E8:ETM2 |
| | 30nm | 10nm | (40%:50%:10%) | 10nm | (50%:50%) |
| | | | 30nm | | 30nm |

Bei der betreffenden OLED werden hervorragende Leistungsdaten gemessen, die im Folgenden gezeigt sind:

| **Bsp.** | **EQE1000 (%)** | **U1000 (V)** | **CIE x/y** | **LD80 (h)** |
|---|---|---|---|---|
| D16 | 19.5 | 3.0 | 0.37/0.62 | 430 |
| D17 | 19.6 | 3.1 | 0.36/0.62 | 450 |
| D21 | 18.2 | 3.0 | 0.40/0.58 | 550 |

### 6) Vergleich der erfindungsgemäßen Verbindung H9 mit der Verbindung HTM-Ref

Die Performance von OLEDs enthaltend das Material H9 wird mit Referenz-OLEDs enthaltend das Material HTM-Ref in verschiedenen Device-Aufbauten verglichen.

Es werden sechs verschiedene Device-Aufbauten a) bis f) herangezogen:
a) D-Ref1 vs. D1
b) D-Ref2 vs. D2
c) D-Ref3 vs. D3
d) D-Ref4 vs. D4
e) D-Ref5 vs. D18
f) D-Ref6 vs. D19

Die exakten Device-Aufbauten, die Aufbauten mit unterschiedlichen Triplett-Emittern und unterschiedlichen Matrixmaterialien umfassen, sind die folgenden:

| **Bsp.** | **HTL2 Dicke** | **EML Dicke** | **HBL Dicke** | **ETL Dicke** |
|---|---|---|---|---|
| D-Ref1 | HTM-Ref | M1:Ir1 | ETM1 | ETM1:ETM2 |
| | 40nm | (85%:15%) | 10nm | (50%:50%) |
| | | 30nm | | 30nm |
| D1 | H9 | s.o. | s.o. | s.o. |
| | 40nm | | | |
| D-Ref2 | | M1:Ir2 | s.o. | s.o. |
| | HTM-Ref 40nm | (85%:15%) | | |
| | | 30nm | | |
| D2 | H9 | s.o. | s.o. | s.o. |
| | 40nm | | | |
| D-Ref3 | HTM-Ref | M1:M2:Ir1 | s.o. | s.o. |
| | 40nm | (45%:45%:10%) | | |
| | | 30nm | | |
| D3 | H9 | s.o. | s.o. | s.o. |
| | 40nm | | | |
| D-Ref4 | HTM-Ref | M1:M2:Ir2 | s.o. | s.o. |
| | 40nm | (45%:45%:10%) | | |
| | | 30nm | | |
| D4 | H9 | s.o. | s.o. | s.o. |
| | 40nm | | | |
| D-Ref5 | | M1:Ir3 | s.o. | s.o. |
| | HTM-Ref 40nm | (80%:20%) | | |
| | | 30nm | | |
| D18 | H9 | s.o. | s.o. | s.o. |
| | 40nm | | | |
| D-Ref6 | HTM-Ref | M1:Ir4 | s.o. | s.o. |
| | 40nm | (85%:15%) | | |
| | | 30nm | | |
| D19 | H9 | s.o. | s.o. | s.o. |
| | 40nm | | | |

Die erhaltenen Messdaten unter anderem für Effizienz und Lebensdauer sind in der folgenden Tabelle gezeigt:

| **Bsp.** | **EQE1000 (%)** | **U1000 (V)** | **CIE x/y** | **LD80 (h)** |
|---|---|---|---|---|
| D-Ref1 | 19.2 | 3.0 | 0.33/0.63 | 70 |
| D1 | 20.3 | 3.1 | 0.33/0.64 | 120 |
| D-Ref2 | 18.7 | 3.1 | 0.36/0.62 | 95 |
| D2 | 19.2 | 3.1 | 0.36/0.62 | 150 |
| D-Ref3 | 19.4 | 3.0 | 0.33/0.63 | 90 |
| D3 | 20.5 | 3.0 | 0.33/0.64 | 140 |
| D-Ref4 | 19.0 | 2.9 | 0.36/0.62 | 130 |
| D4 | 19.5 | 3.0 | 0.36/0.62 | 180 |
| D-Ref5 | 16.7 | 2.9 | 0.40/0.58 | 130 |
| D18 | 17.0 | 2.9 | 0.39/0.59 | 300 |
| D-Ref6 | 18.8 | 3.0 | 0.46/0.53 | 160 |
| D19 | 19.1 | 3.0 | 0.46/0.53 | 360 |

Wie aus den Messdaten ersichtlich wird, ist in allen direkten Vergleichen zwischen H9 und HTM-Ref die Lebensdauer und die Effizienz stark verbessert, bei vergleichbaren CIE-Koordinaten und Spannung.

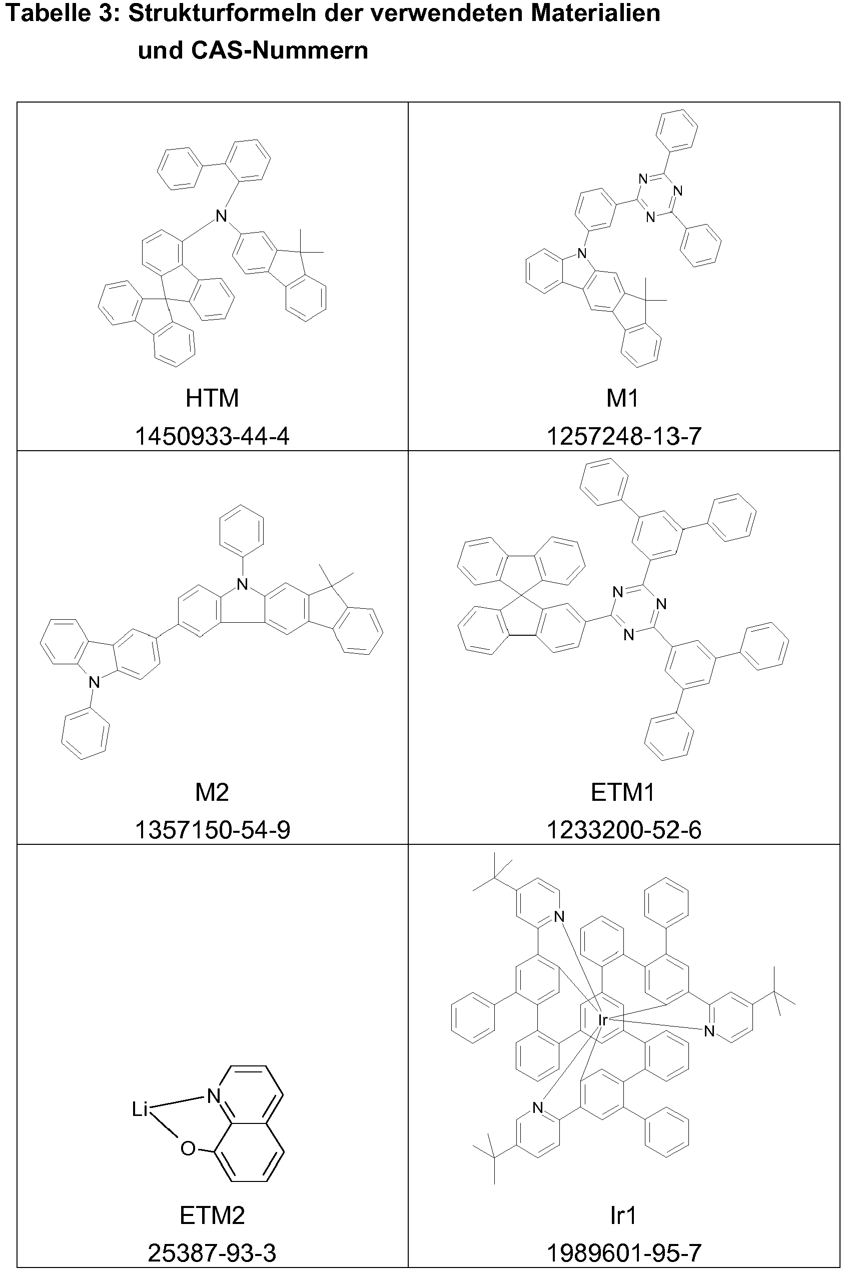

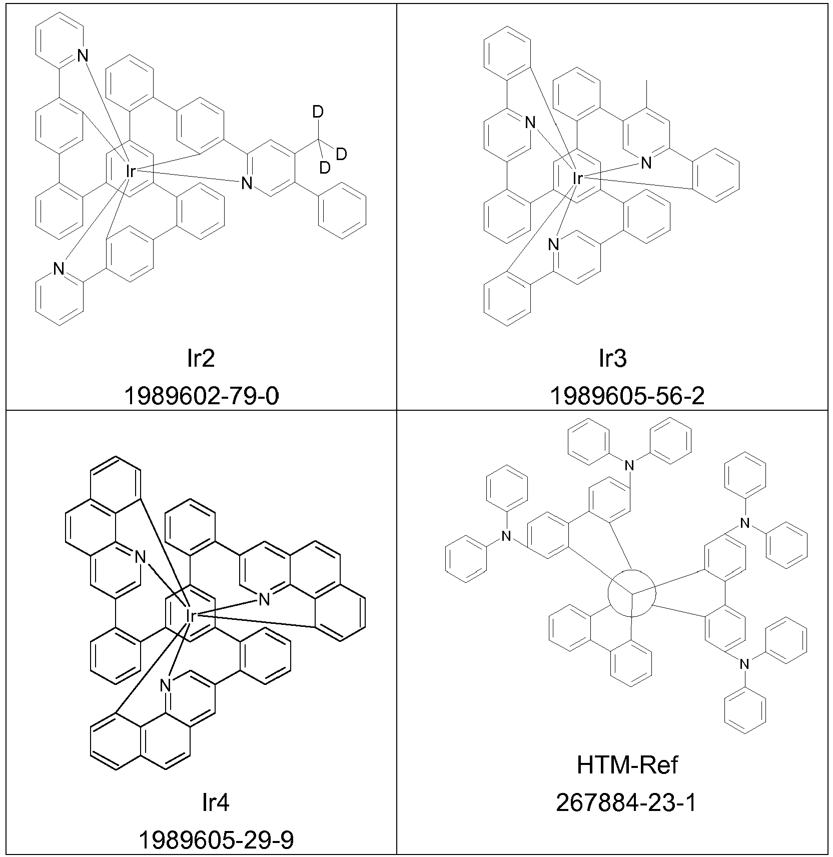

## Patentansprüche

1. Verbindung einer Formel (I), (II), (III) oder (IV), 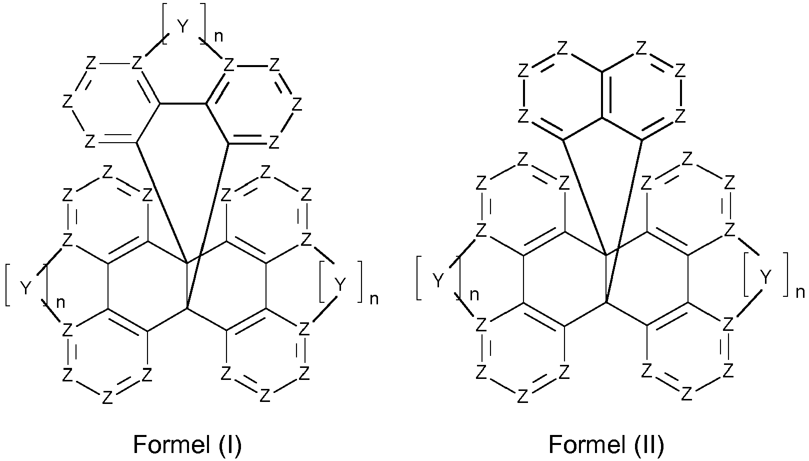 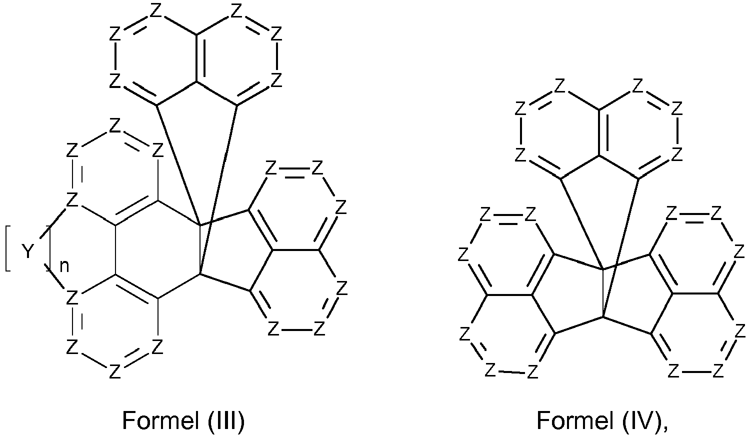 wobei für die auftretenden Variablen gilt:
Z ist bei jedem Auftreten gleich oder verschieden gewählt aus N und CR¹ oder C, wobei eine Gruppe Z genau dann gleich C ist, wenn eine Gruppe Y daran gebunden ist;
Y ist bei jedem Auftreten gleich oder verschieden gewählt aus BR², C(R²)₂, Si(R²)₂, NR², P(O)R², O, S, SO, SO₂;
n ist bei jedem Auftreten gleich oder verschieden 0 oder 1;
R¹, R² sind bei jedem Auftreten gleich oder verschieden gewählt aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste R¹ und/oder R² miteinander verknüpft sein können und einen Ring bilden können; wobei die genannten Alkyl-, Alkoxy-, Alkenyl- und Alkinylgruppen und die genannten aromatischen Ringsysteme jeweils mit einem oder mehreren Resten R³ substituiert sein können; wobei an einen oder mehrere der Sechsringe in Formel (I) bis (IV) jeweils ein Indolring ankondensiert sein kann, der wiederum mit Resten R³ substituiert sein kann;
R³ ist bei jedem Auftreten gleich oder verschieden gewählt aus H, D, F, CN, Si(R⁴)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme jeweils mit einem oder mehreren Resten R⁴ substituiert sein können;
R⁴ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen und die genannten aromatischen Ringsysteme mit F oder CN substituiert sein können;
**dadurch gekennzeichnet, dass** mindestens eine Gruppe Z je Formel gewählt aus den Formeln (I), (II), (III) und (IV) gleich CR¹ ist;
und weiterhin **dadurch gekennzeichnet, dass** genau eine Gruppe R¹ je Formel (I) durch eine Gruppe der Formel (A) oder eine Gruppe der Formel (H) ersetzt ist, 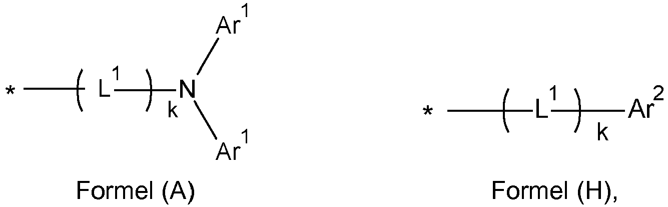
und weiterhin **dadurch gekennzeichnet, dass** genau eine Gruppe R¹ je Formel gewählt aus den Formeln (II), (III) und (IV) durch eine Gruppe der Formel (A) ersetzt ist,
wobei gilt:
L¹ ist bei jedem Auftreten gleich oder verschieden ein aromatisches Ringsystem mit 6 bis 24 aromatischen Ringatomen oder ein heteroaromatisches Ringsystem mit 5 bis 24 aromatischen Ringatomen, die jeweils durch einen oder mehrere Reste R⁵ substituiert sein können;
k ist gleich 0, 1, 2 oder 3;
Ar¹ ist bei jedem Auftreten gleich oder verschieden ein aromatisches Ringsystem mit 6 bis 24 aromatischen Ringatomen oder ein heteroaromatisches Ringsystem mit 5 bis 24 aromatischen Ringatomen, das mit einem oder mehreren Resten R⁵ substituiert sein kann;
Ar² ist ein heteroaromatisches Ringsystem mit 5 bis 24 aromatischen Ringatomen, das mit einem oder mehreren Resten R⁵ substituiert sein kann;
R⁵ ist bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
wobei die Gruppe der Formel (A) bzw. die Gruppe der Formel (H) über die mit * markierte Bindung gebunden ist.

2. Verbindung nach Anspruch 1, **dadurch gekennzeichnet, dass** Formel (I) einer der Formeln (I-H-1) bis (I-H-7) und (I-A-1) bis (I-A-7) entspricht
| | |
|---|---|
| 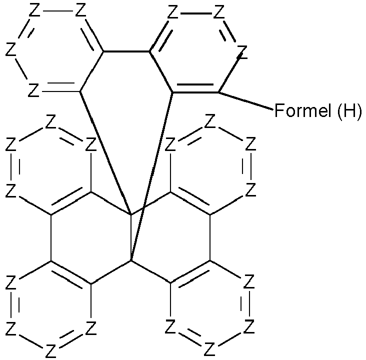 | 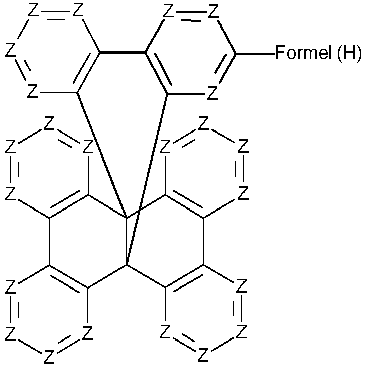 |
| Formel (I-H-1) | Formel (I-H-2) |
| 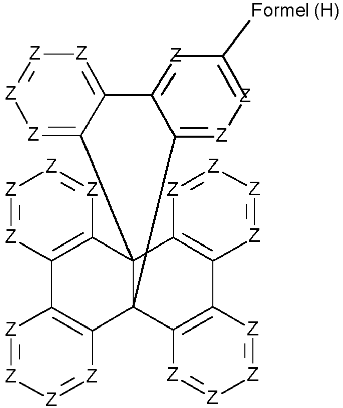 | 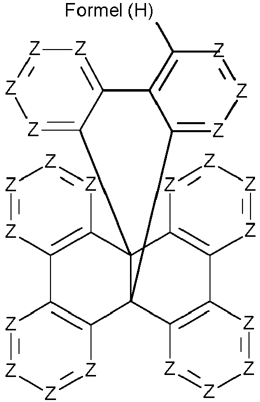 |
| Formel (I-H-3) | Formel (I-H-4) |
| 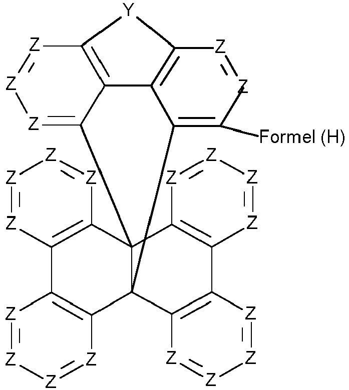 | 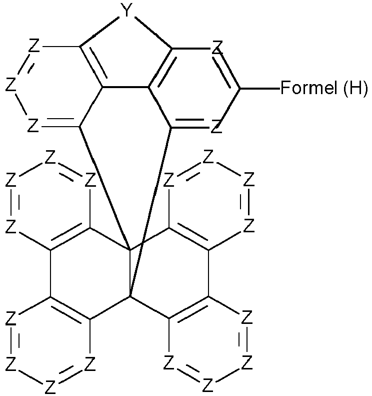 |
| Formel (I-H-5) | Formel (I-H-6) |
| 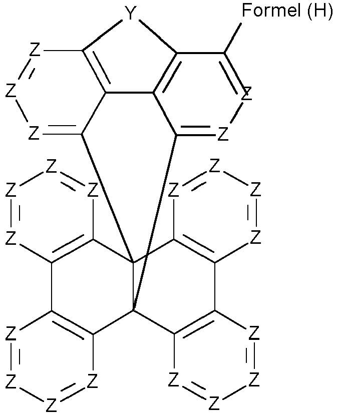 | 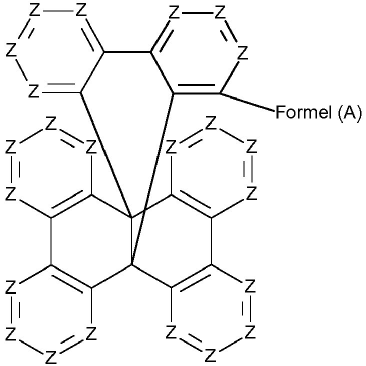 |
| Formel (I-H-7) | Formel (I-A-1) |
| 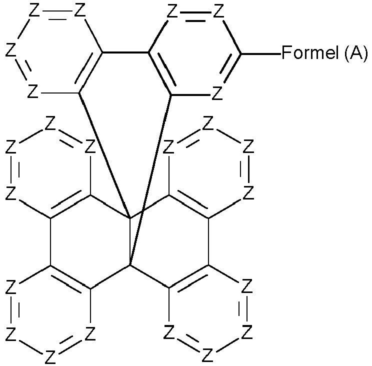 | 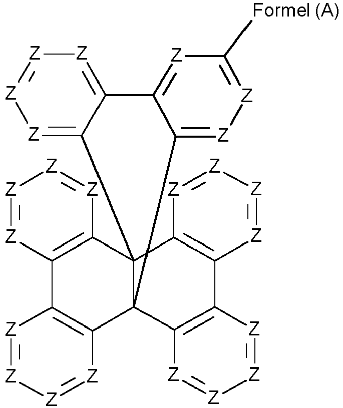 |
| Formel (I-A-2) | Formel (I-A-3) |
| 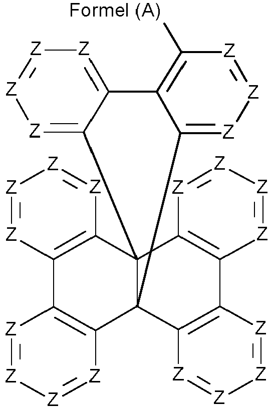 | 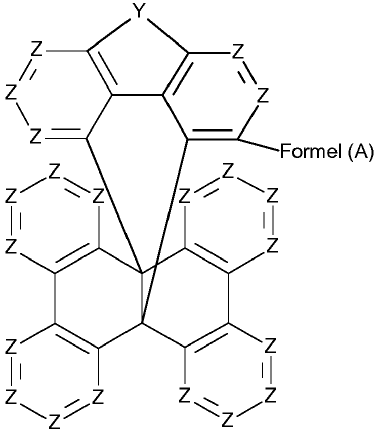 |
| Formel (I-A-4) | Formel (I-A-5) |
| 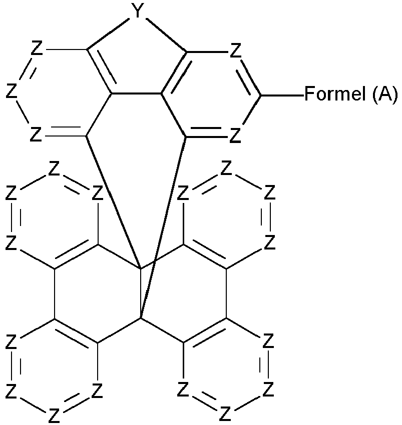 | 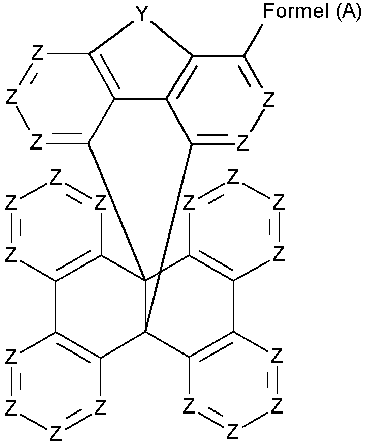 |
| Formel (I-A-6) | Formel (I-A-7), |
und/oder dass Formel (II) einer der Formeln (II-A-1) bis (II-A-3) entspricht
| | |
|---|---|
| 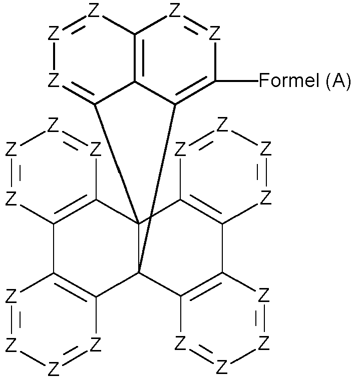 | 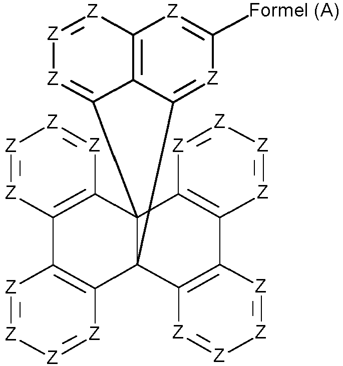 |
| Formel (II-A-1) | Formel (II-A-2) |
| 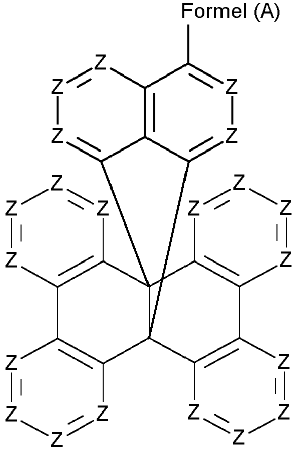 | |
| Formel (II-A-3), | |
und/oder dass Formel (III) einer der Formeln (III-A-1) bis (III-A-3) entspricht
| | |
|---|---|
| 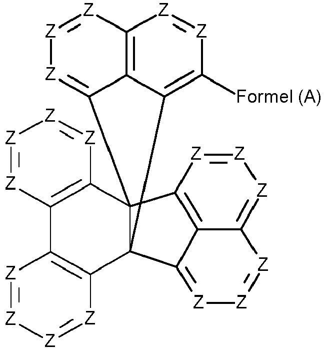 | 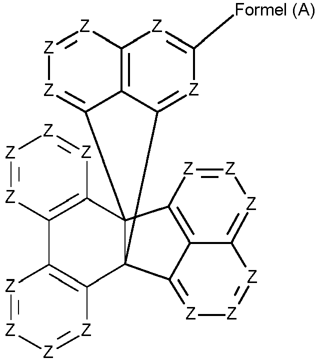 |
| Formel (III-A-1) | Formel (III-A-2) |
| 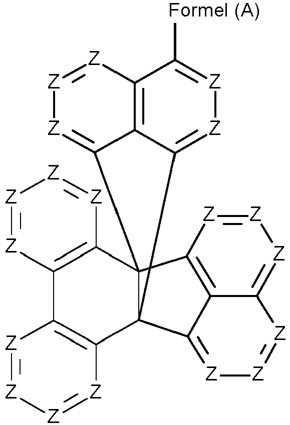 | |
| Formel (III-A-3), | |
und/oder dass Formel (IV) einer der Formeln (IV-A-1) bis (IV-A-3) entspricht
| | |
|---|---|
| 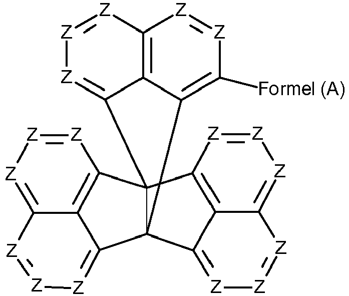 | 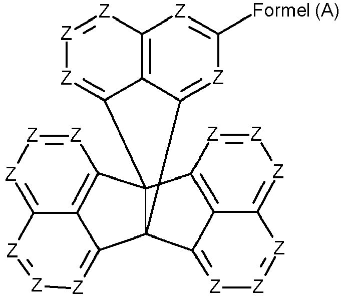 |
| Formel (IV-A-1) | Formel (IV-A-2) |
| 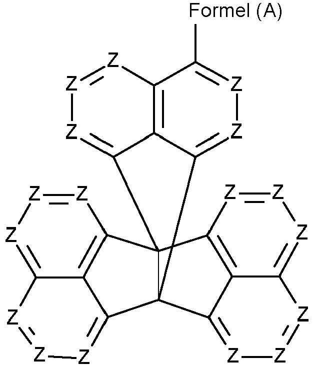 | |
| Formel (IV-A-3), | |
wobei die auftretenden Gruppen wie in Anspruch 1 definiert sind, und wobei "Formel (A)" eine Gruppe der Formel (A), wie in Anspruch 1 definiert, ist, und wobei "Formel (H)" eine Gruppe der Formel (H), wie in Anspruch 1 definiert, ist.

3. Verbindung nach Anspruch 1 oder 2, **dadurch gekennzeichnet, dass** Z gleich CR¹ oder C ist, wobei Z genau dann gleich C ist, wenn eine Gruppe Y daran gebunden ist.

4. Verbindung nach einem oder mehreren der Ansprüche 1 bis 3, **dadurch gekennzeichnet, dass** Y bei jedem Auftreten gleich oder verschieden gewählt ist aus O und NR².

5. Verbindung nach einem oder mehreren der Ansprüche 1 bis 4, **dadurch gekennzeichnet, dass** die Summe der Indices n in einer Verbindung der Formel (II) gleich 1 oder 0 ist, und/oder dass die Summe der Indices n in einer Verbindung der Formel (III) gleich 0 ist.

6. Verbindung nach einem oder mehreren der Ansprüche 1 bis 5, **dadurch gekennzeichnet, dass** der Index n gleich 0 ist.

7. Verbindung nach einem oder mehreren der Ansprüche 1 bis 6, **dadurch gekennzeichnet, dass** die Gruppe Y an denjenigen Sechsring gebunden ist, der eine Gruppe der Formel (A) oder (H) trägt.

8. Verbindung nach einem oder mehreren der Ansprüche 1 bis 7, **dadurch gekennzeichnet, dass** R¹ bei jedem Auftreten gleich oder verschieden gewählt ist aus H, D, geradkettigen oder verzweigten Alkylgruppen mit bis zu 12 C-Atomen, und aromatischen Ringsystemen mit 6 bis 24 aromatischen Ringatomen, wobei die genannten Alkylgruppen und die genannten aromatischen Ringsysteme jeweils mit einem oder mehreren Resten R³ substituiert sein können.

9. Verbindung nach einem oder mehreren der Ansprüche 1 bis 8, **dadurch gekennzeichnet, dass** R² gewählt ist aus geradkettigen oder verzweigten Alkylgruppen mit bis zu 12 C-Atomen, und aromatischen Ringsystemen mit 6 bis 24 aromatischen Ringatomen, wobei zwei Reste R², die an dasselbe Kohlenstoffatom einer Gruppe C(R²)₂ binden, und die Alkylgruppen oder Arylgruppen sind, miteinander zu einer cyclischen Alkylgruppe oder zu einer Fluoren-Gruppe verbunden sein können.

10. Verbindung nach einem oder mehreren der Ansprüche 1 bis 9, **dadurch gekennzeichnet, dass** R³ bei jedem Auftreten gleich oder verschieden gewählt ist aus H, D, F, CN, geradkettigen Alkylgruppen mit 1 bis 12 C-Atomen, verzweigten oder cyclischen Alkylgruppen mit 3 bis 12 C-Atomen, und aromatischen Ringsystemen mit 6 bis 24 aromatischen Ringatomen; wobei zwei oder mehr Reste R³ miteinander verknüpft sein können und einen Ring bilden können; und wobei die genannten Alkylgruppen und die genannten aromatischen Ringsysteme jeweils mit einem oder mehreren Resten R⁴ substituiert sein können.

11. Verbindung nach einem oder mehreren der Ansprüche 1 bis 10, **dadurch gekennzeichnet, dass** R⁴ bei jedem Auftreten gleich oder verschieden gewählt ist aus H, D, F, CN, geradkettigen Alkylgruppen mit 1 bis 12 C-Atomen, verzweigten oder cyclischen Alkylgruppen mit 3 bis 12 C-Atomen, und aromatischen Ringsystemen mit 6 bis 24 aromatischen Ringatomen; wobei zwei oder mehr Reste R⁴ miteinander verknüpft sein können und einen Ring bilden können; und wobei die genannten Alkylgruppen und die genannten aromatischen Ringsysteme mit F oder CN substituiert sein können.

12. Verbindung nach einem oder mehreren der Ansprüche 1 bis 11, **dadurch gekennzeichnet, dass** genau eine Gruppe R¹ je Formel (I) durch eine Gruppe der Formel (A) ersetzt ist.

13. Verbindung nach einem oder mehreren der Ansprüche 1 bis 12, **dadurch gekennzeichnet, dass** L¹ eine divalente Gruppe gewählt aus Phenylen, Biphenylen, Terphenylen, Naphthylen, Dibenzofuran, Dibenzothiophen, Carbazol, und Fluoren ist, wobei die divalente Gruppe mit einem oder mehreren Resten R⁵ substituiert sein kann.

14. Verbindung nach einem oder mehreren der Ansprüche 1 bis 13, **dadurch gekennzeichnet, dass** Ar¹ bei jedem Auftreten gleich oder verschieden gewählt ist aus Phenyl, Biphenyl, Terphenyl, Fluorenyl, Naphthyl, Spirobifluorenyl, Pyridyl, Pyrimidyl, Triazinyl, Dibenzofuranyl, benzo-kondensiertem Dibenzofuranyl, Dibenzothiophenyl, benzo-kondensiertem Dibenzothiophenyl, Carbazolyl, und benzo-kondensiertem Carbazolyl, und Kombinationen aus zwei, drei oder vier dieser Gruppen, wobei die genannten Gruppen jeweils mit einem oder mehreren Resten R⁵ substituiert sein können.

15. Verbindung nach einem oder mehreren der Ansprüche 1 bis 14, **dadurch gekennzeichnet, dass** L¹ eine divalente Gruppe gewählt aus Phenylen, Biphenylen, Terphenylen, Naphthylen, Dibenzofuran, Dibenzothiophen, Carbazol, und Fluoren ist, wobei die divalente Gruppe mit einem oder mehreren Resten R⁵ substituiert sein kann.

16. Verbindung nach einem oder mehreren der Ansprüche 1 bis 15, **dadurch gekennzeichnet, dass** Ar² bei jedem Auftreten gleich oder verschieden gewählt ist aus Gruppen der folgenden Formeln
| | |
|---|---|
| 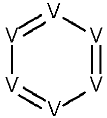 | 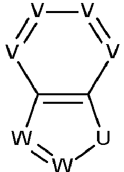 |
| (Ar²-A) | (Ar²-B) |
| 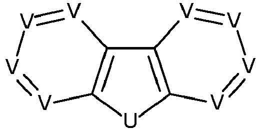 | 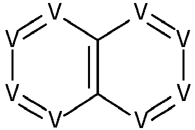 |
| (Ar²-C) | (Ar²-D) |
wobei die auftretenden Variablen wie folgt definiert sind:
V ist bei jedem Auftreten gleich oder verschieden N oder CR⁵, wobei in Formel (Ar²-A) und (Ar²-D) mindestens eine Gruppe V je Formel gleich N ist;
W ist bei jedem Auftreten gleich oder verschieden N oder CR⁵;
U ist gleich NR⁵;
wobei eine Gruppe R⁵ je Formel durch die Bindung an die Gruppe L¹ bzw. die Propellangruppe ersetzt ist.

17. Verbindung nach einem oder mehreren der Ansprüche 1 bis 16, **dadurch gekennzeichnet, dass** Ar² gewählt ist aus Pyridin, Pyrimidin, Pyridazin, Pyrazin, Triazin, Carbazol, Imidazol, Pyrazol, Triazol, Benzimidazol, Benzimidazoisochinolin, Imidazophenanthridin, Benzimidazophenanthridin Bathocuproin, Benzimidazobenzimidazol, Chinolin, Chinazolin, Phenanthrolin, Phenanthridin, Diazaphenanthren, und Acridin, die jeweils mit einem oder mehreren Resten R⁵ substituiert sein können.

18. Verbindung nach einem oder mehreren der Ansprüche 1 bis 17, **dadurch gekennzeichnet, dass** R⁵ gewählt ist aus H, D, F, CN, Si(R⁶)₃, N(R⁶)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁶ substituiert sein können; und wobei in den genannten Alkyl- oder Alkoxygruppen eine oder mehrere CH₂-Gruppen durch -C≡C-, -R⁶C=CR⁶-, Si(R⁶)₂, C=O, C=NR⁶, -NR⁶-, -O-, -S-, -C(=O)O- oder -C(=O)NR⁶- ersetzt sein können.

19. Verfahren zur Herstellung einer Verbindung nach einem oder mehreren der Ansprüche 1 bis 18, **dadurch gekennzeichnet, dass** zunächst das Alkoxy-substituierte Grundgerüst hergestellt wird, welches in einem weiteren Schritt in eine reaktive Verbindung überführt wird, bevorzugt in ein Triflat-Derivat, welches in einem weiteren Schritt durch Übergangsmetallkatalysierte Kupplungsreaktion, bevorzugt Hartwig-Buchwald-, Suzuki-, Stille-, oder Negishi-Kupplung, in die Verbindung nach einem oder mehreren der Ansprüche 1 bis 18 überführt wird.

20. Oligomer, Polymer oder Dendrimer, enthaltend eine oder mehrere Verbindungen nach einem oder mehreren der Ansprüche 1 bis 18, wobei die Bindung(en) zum Polymer, Oligomer oder Dendrimer an beliebigen mit R¹, R² oder R⁵ substituierten Positionen lokalisiert sein können.

21. Formulierung, enthaltend eine oder mehrere Verbindungen nach einem oder mehreren der Ansprüche 1 bis 18, sowie mindestens ein Lösungsmittel.

22. Elektronische Vorrichtung, gewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), enthaltend mindestens eine Verbindung nach einem oder mehreren der Ansprüche 1 bis 18.

23. Organische Elektrolumineszenzvorrichtung nach Anspruch 22, enthaltend Anode, Kathode und mindestens eine organische Schicht, wobei die Verbindung in einer lochtransportierenden Schicht, als Matrixmaterial in einer emittierenden Schicht, oder in einer elektronentransportierenden Schicht vorhanden ist.

24. Verwendung einer Verbindung nach einem oder mehreren der Ansprüche 1 bis 18 in einer elektronischen Vorrichtung, gewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs).

## Claims

1. Compound of a formula (I), (II), (III) or (IV), 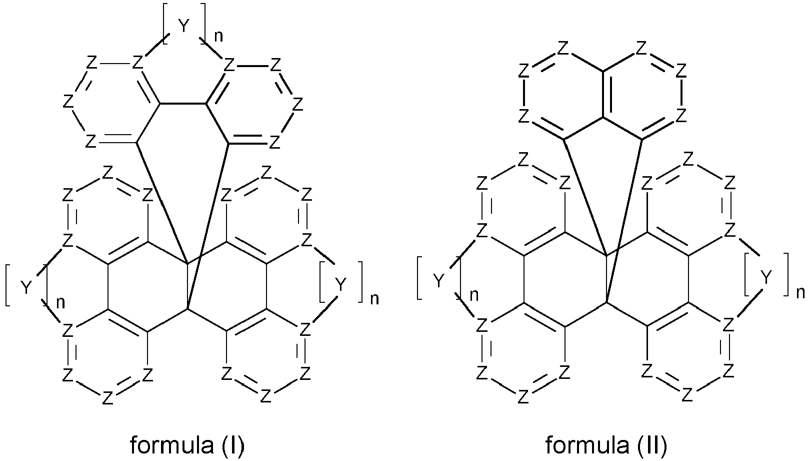 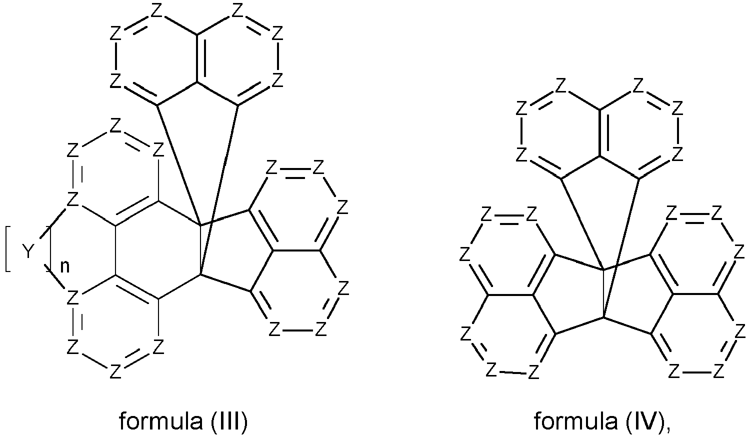 where the following applies to the variables appearing:
Z is selected on each occurrence, identically or differently, from N and CR¹, or is C, where a group Z is equal to C precisely when a group Y is bonded thereto;
Y is selected on each occurrence, identically or differently, from BR², C(R²)₂, Si(R²)₂, NR², P(O)R², O, S, SO, SO₂;
n is on each occurrence, identically or differently, 0 or 1;
R¹, R² are selected on each occurrence, identically or differently, from H, D, F, CN, Si(R³)₃, straight-chain alkyl or alkoxy groups having 1 to 20 C atoms, branched or cyclic alkyl or alkoxy groups having 3 to 20 C atoms, alkenyl or alkynyl groups having 2 to 20 C atoms, and aromatic ring systems having 6 to 40 aromatic ring atoms; where two or more radicals R¹ and/or R² may be linked to one another and may form a ring; where the said alkyl, alkoxy, alkenyl and alkynyl groups and the said aromatic ring systems may in each case be substituted by one or more radicals R³; where an indole ring, which may in turn be substituted by radicals R³, may in each case be condensed onto one or more of the six-membered rings in formulae (I) to (IV);
R³ is selected on each occurrence, identically or differently, from H, D, F, CN, Si(R⁴)₃, straight-chain alkyl or alkoxy groups having 1 to 20 C atoms, branched or cyclic alkyl or alkoxy groups having 3 to 20 C atoms, alkenyl or alkynyl groups having 2 to 20 C atoms, and aromatic ring systems having 6 to 40 aromatic ring atoms; where two or more radicals R³ may be linked to one another and may form a ring; where the said alkyl, alkoxy, alkenyl and alkynyl groups and the said aromatic ring systems may in each case be substituted by one or more radicals R⁴;
R⁴ is selected on each occurrence, identically or differently, from H, D, F, CN, alkyl or alkoxy groups having 1 to 20 C atoms, alkenyl or alkynyl groups having 2 to 20 C atoms, and aromatic ring systems having 6 to 40 aromatic ring atoms; where two or more radicals R⁴ may be linked to one another and may form a ring; and where the said alkyl, alkoxy, alkenyl and alkynyl groups and the said aromatic ring systems may be substituted by F or CN;
**characterised in that** at least one group Z per formula selected from the formulae (I), (II), (III) and (IV) is equal to CR¹;
and furthermore **characterised in that** precisely one group R¹ per formula (I) has been replaced by a group of the formula (A) or a group of the formula (H), 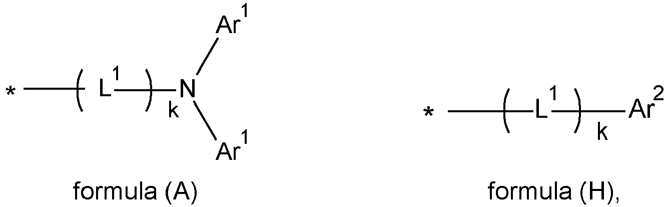 and furthermore **characterised in that** precisely one group R¹ per formula selected from the formulae (II), (III) and (IV) has been replaced by a group of the formula (A),
where:
L¹ is on each occurrence, identically or differently, an aromatic ring system having 6 to 24 aromatic ring atoms or a heteroaromatic ring system having 5 to 24 aromatic ring atoms, which may in each case be substituted by one or more radicals R⁵;
k is equal to 0, 1, 2 or 3;
Ar¹ is on each occurrence, identically or differently, an aromatic ring system having 6 to 24 aromatic ring atoms or a heteroaromatic ring system having 5 to 24 aromatic ring atoms, which may be substituted by one or more radicals R⁵;
Ar² ist a heteroaromatic ring system having 5 to 24 aromatic ring atoms, which may be substituted by one or more radicals R⁵;
R⁵ is selected on each occurrence, identically or differently, from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁵ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by-R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂;
R⁶ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁶ may be linked to one another and may form a ring; and where the said alkyl, alkoxy, alkenyl and alkynyl groups, aromatic ring systems and heteroaromatic ring systems may be substituted by F or CN;
where the group of the formula (A) or the group of the formula (H) is bonded via the bond marked with *.

2. Compound according to Claim 1, **characterised in that** formula (I) corresponds to one of the formulae (I-H-1) to (I-H-7) and (I-A-1) to (I-A-7)
| | |
|---|---|
| 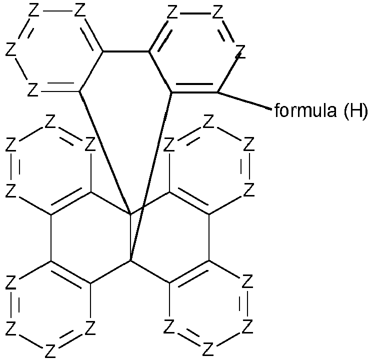 | 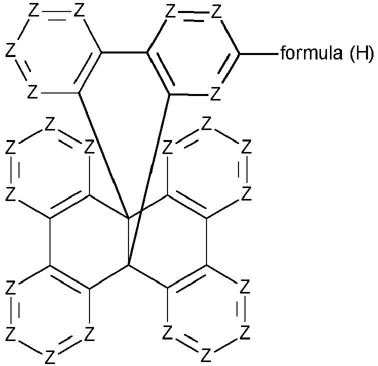 |
| formula (I-H-1) | formula (I-H-2) |
| 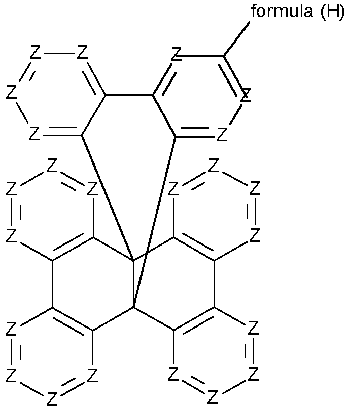 | 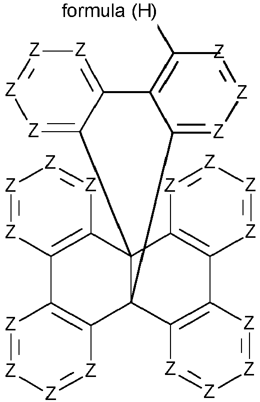 |
| formula (I-H-3) | formula (I-H-4) |
| 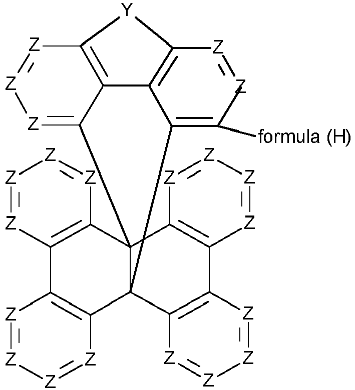 | 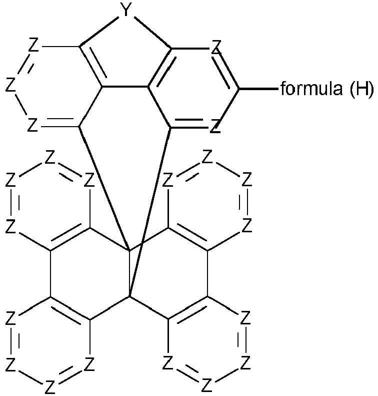 |
| formula (I-H-5) | formula (I-H-6) |
| 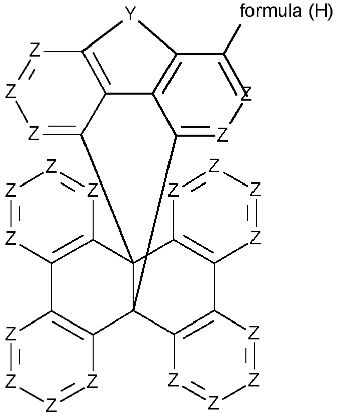 | 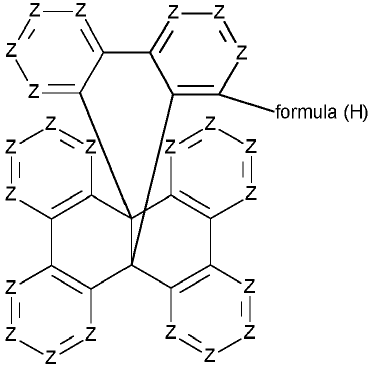 |
| formula (I-H-7) | formula (I-A-1) |
| 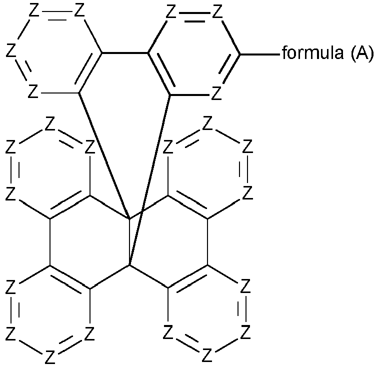 | 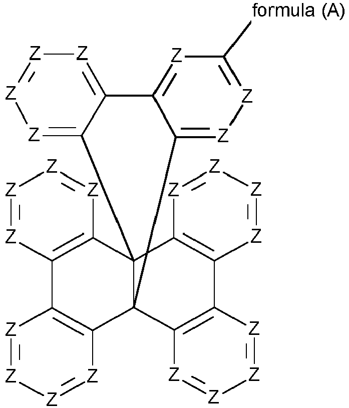 |
| formula (I-A-2) | formula (I-A-3) |
| 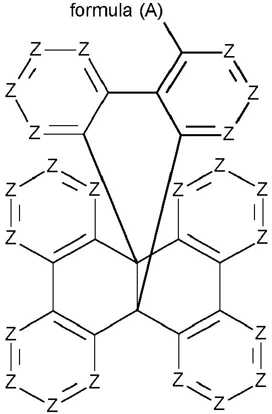 | 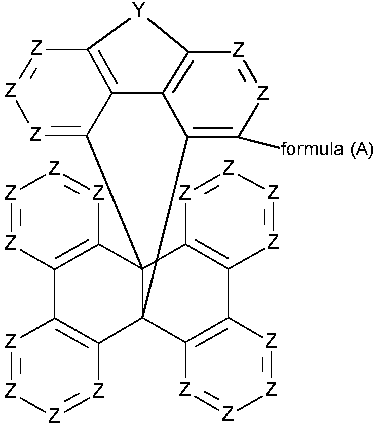 |
| formula (I-A-4) | formula (I-A-5) |
| 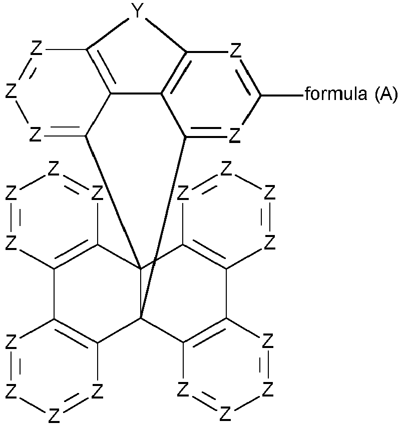 | 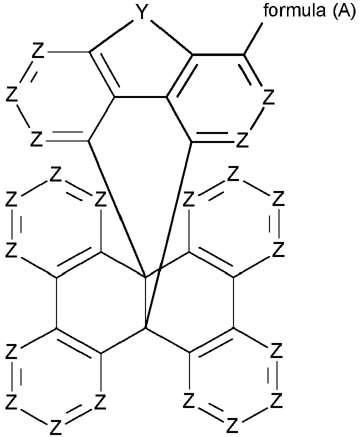 |
| formula (I-A-6) | formula (I-A-7), |
and/or **in that** formula (II) corresponds to one of the formulae (II-A-1) to (II-A-3)
| | |
|---|---|
| 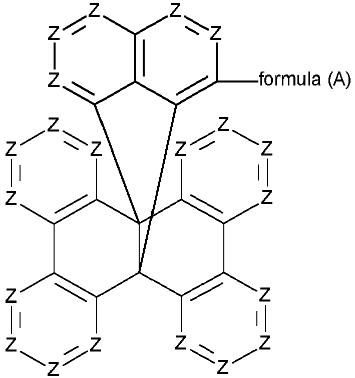 | 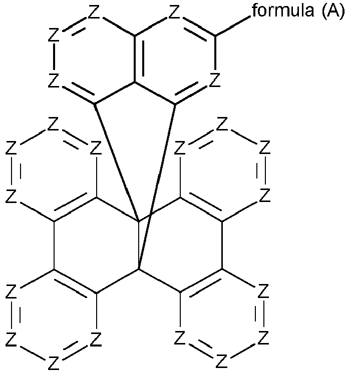 |
| formula (II-A-1) | formula (II-A-2) |
| 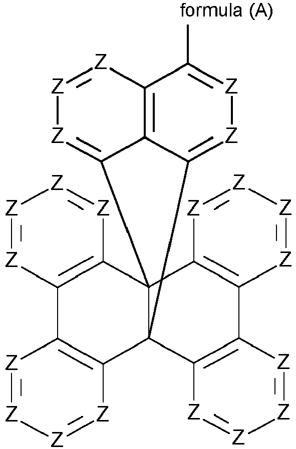 | |
| formula (II-A-3), | |
and/or **in that** formula (III) corresponds to one of the formulae (III-A-1) to (III-A-3)
| | |
|---|---|
| 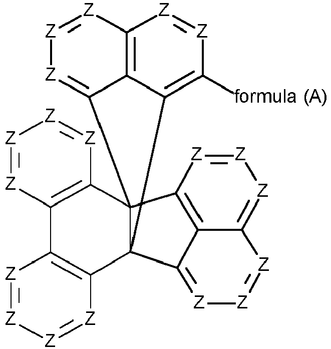 | 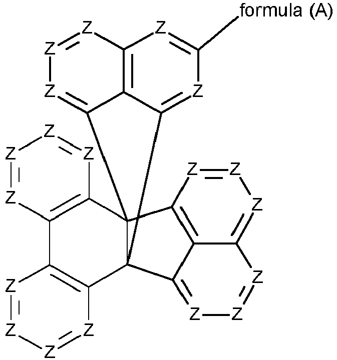 |
| formula (III-A-1) | formula (III-A-2) |
| 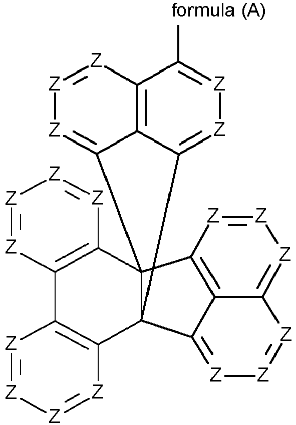 | |
| formula (III-A-3), | |
and/or **in that** formula (IV) corresponds to one of the formulae (IV-A-1) to (IV-A-3)
| | |
|---|---|
| 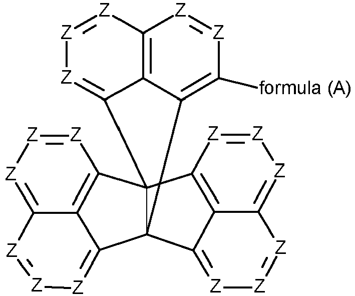 | 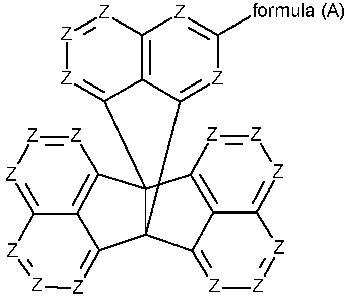 |
| formula (IV-A-1) | formula (IV-A-2) |
| 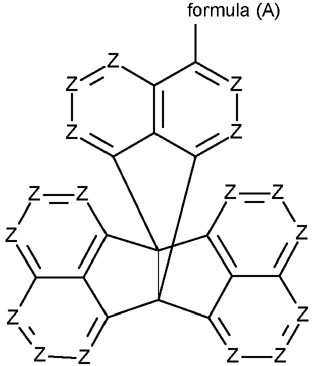 | |
| formula (IV-A-3), | |
where the groups appearing are defined as in Claim 1, and where "formula (A)" is a group of the formula (A) as defined in Claim 1, and where "formula (H)" is a group of the formula (H) as defined in Claim 1.

3. Compound according to Claim 1 or 2, **characterised in that** Z is equal to CR¹ or C, where Z is equal to C precisely when a group Y is bonded thereto.

4. Compound according to one or more of Claims 1 to 3, **characterised in that** Y is selected on each occurrence, identically or differently, from O and NR².

5. Compound according to one or more of Claims 1 to 4, **characterised in that** the sum of the indices n in a compound of the formula (II) is equal to 1 or 0, and/or **in that** the sum of the indices n in a compound of the formula (III) is equal to 0.

6. Compound according to one or more of Claims 1 to 5, **characterised in that** the index n is equal to 0.

7. Compound according to one or more of Claims 1 to 6, **characterised in that** the group Y is bonded to the six-membered ring that carries a group of the formula (A) or (H).

8. Compound according to one or more of Claims 1 to 7, **characterised in that** R¹ is selected on each occurrence, identically or differently, from H, D, straight-chain or branched alkyl groups having up to 12 C atoms, and aromatic ring systems having 6 to 24 aromatic ring atoms, where the said alkyl groups and the said aromatic ring systems may in each case be substituted by one or more radicals R³.

9. Compound according to one or more of Claims 1 to 8, **characterised in that** R² is selected from straight-chain or branched alkyl groups having up to 12 C atoms, and aromatic ring systems having 6 to 24 aromatic ring atoms, where two radicals R² that are bonded to the same carbon atom of a group C(R²)₂ and that are alkyl groups or aryl groups may be connected to one another to form a cyclic alkyl group or to form a fluorene group.

10. Compound according to one or more of Claims 1 to 9, **characterised in that** R³ is selected on each occurrence, identically or differently, from H, D, F, CN, straight-chain alkyl groups having 1 to 12 C atoms, branched or cyclic alkyl groups having 3 to 12 C atoms, and aromatic ring systems having 6 to 24 aromatic ring atoms; where two or more radicals R³ may be linked to one another and may form a ring; and where the said alkyl groups and the said aromatic ring systems may in each case be substituted by one or more radicals R⁴.

11. Compound according to one or more of Claims 1 to 10, **characterised in that** R⁴ is selected on each occurrence, identically or differently, from H, D, F, CN, straight-chain alkyl groups having 1 to 12 C atoms, branched or cyclic alkyl groups having 3 to 12 C atoms, and aromatic ring systems having 6 to 24 aromatic ring atoms; where two or more radicals R⁴ may be linked to one another and may form a ring; and where the said alkyl groups and the said aromatic ring systems may be substituted by F or CN.

12. Compound according to one or more of Claims 1 to 11, **characterised in that** precisely one group R¹ per formula (I) has been replaced by a group of the formula (A).

13. Compound according to one or more of Claims 1 to 12, **characterised in that** L¹ is a divalent group selected from phenylene, biphenylene, terphenylene, naphthylene, dibenzofuran, dibenzothiophene, carbazole and fluorene, where the divalent group may be substituted by one or more radicals R⁵.

14. Compound according to one or more of Claims 1 to 13, **characterised in that** Ar¹ is selected on each occurrence, identically or differently, from phenyl, biphenyl, terphenyl, fluorenyl, naphthyl, spirobifluorenyl, pyridyl, pyrimidyl, triazinyl, dibenzofuranyl, benzo-condensed dibenzofuranyl, dibenzothiophenyl, benzo-condensed dibenzothiophenyl, carbazolyl and benzo-condensed carbazolyl, and combinations of two, three or four of these groups, where the said groups may in each case be substituted by one or more radicals R⁵.

15. Compound according to one or more of Claims 1 to 14, **characterised in that** L¹ is a divalent group selected from phenylene, biphenylene, terphenylene, naphthylene, dibenzofuran, dibenzothiophene, carbazole and fluorene, where the divalent group may be substituted by one or more radicals R⁵.

16. Compound according to one or more of Claims 1 to 15, **characterised in that** Ar² is selected on each occurrence, identically or differently, from groups of the following formulae:
| | |
|---|---|
| 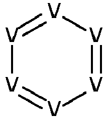 | 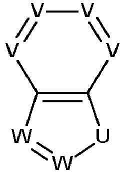 |
| (Ar²-A) | (Ar²-B) |
| 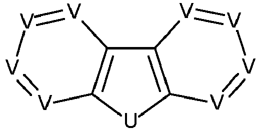 | 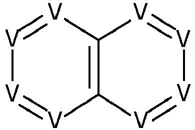 |
| (Ar²-C) | (Ar²-D) |
where the variables appearing are defined as follows:
V is on each occurrence, identically or differently, N or CR⁵, where in formulae (Ar²-A) and (Ar²-D) at least one group V per formula is equal to N;
W is on each occurrence, identically or differently, N or CR⁵;
U is equal to NR⁵;
where one group R⁵ per formula has been replaced by the bond to the group L¹ or the propellane group.

17. Compound according to one or more of Claims 1 to 16, **characterised in that** Ar² is selected from pyridine, pyrimidine, pyridazine, pyrazine, triazine, carbazole, imidazole, pyrazole, triazole, benzimidazole, benzimidazoisoquinoline, imidazophenanthridine, benzimidazophenanthridine, bathocuproine, benzimidazobenzimidazole, quinoline, quinazoline, phenanthroline, phenanthridine, diazaphenanthrene and acridine, which may in each case be substituted by one or more radicals R⁵.

18. Compound according to one or more of Claims 1 to 17, **characterised in that** R⁵ is selected from H, D, F, CN, Si(R⁶)₃, N(R⁶)₂, straight-chain alkyl or alkoxy groups having 1 to 20 C atoms, branched or cyclic alkyl or alkoxy groups having 3 to 20 C atoms, aromatic ring systems having 6 to 40 aromatic ring atoms, and heteroaromatic ring systems having 5 to 40 aromatic ring atoms; where the said alkyl and alkoxy groups, the said aromatic ring systems and the said heteroaromatic ring systems may in each case be substituted by one or more radicals R⁶; and where one or more CH₂ groups in the said alkyl or alkoxy groups may be replaced by -C≡C-, -R⁶C=CR⁶-, Si(R⁶)₂, C=O, C=NR⁶, -NR⁶-, -O-, -S-, -C(=O)O- or -C(=O)NR⁶-.

19. Process for the preparation of a compound according to one or more of Claims 1 to 18, **characterised in that** firstly the alkoxy-substituted skeleton is prepared, which is converted in a further step into a reactive compound, preferably into a triflate derivative, which is converted in a further step into the compound according to one or more of Claims 1 to 18 by a transition metal-catalysed coupling reaction, preferably Hartwig-Buchwald, Suzuki, Stille or Negishi coupling.

20. Oligomer, polymer or dendrimer containing one or more compounds according to one or more of Claims 1 to 18, where the bond(s) to the polymer, oligomer or dendrimer may be localised at any desired positions that are substituted by R¹, R² or R⁵.

21. Formulation comprising one or more compounds according to one or more of Claims 1 to 18, and at least one solvent.

22. Electronic device, selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), containing at least one compound according to one or more of Claims 1 to 18.

23. Organic electroluminescent device according to Claim 22, containing anode, cathode and at least one organic layer, where the compound is present in a hole-transporting layer, as matrix material in an emitting layer, or in an electron-transporting layer.

24. Use of a compound according to one or more of Claims 1 to 18 in an electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

## Revendications

1. Composé d'une formule (I), (II), (III) ou (IV), 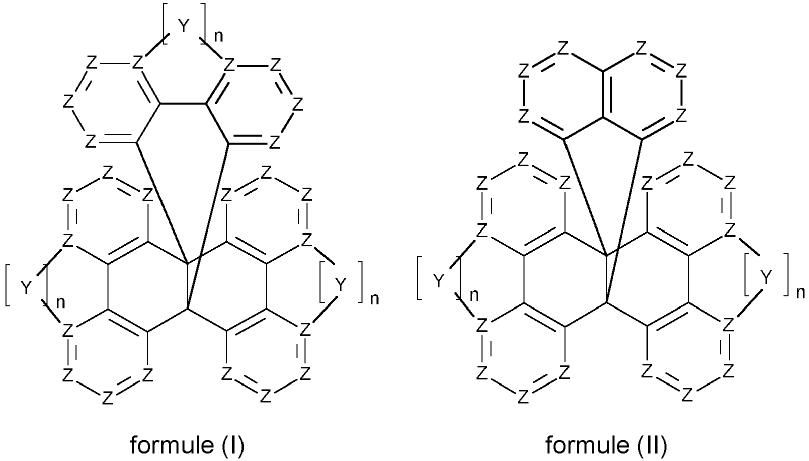 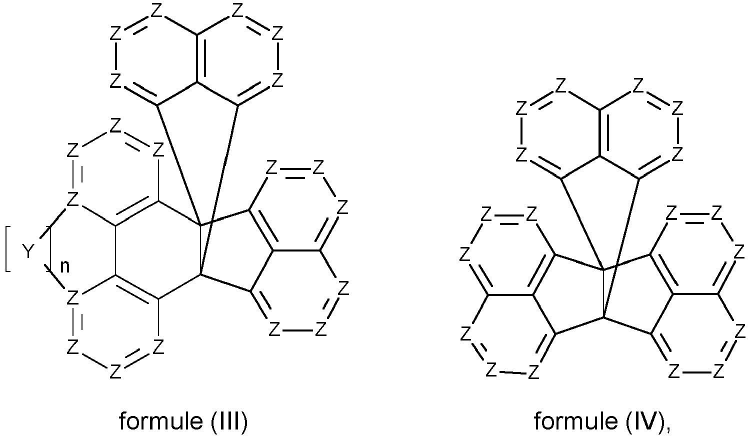 dans lesquelles ce qui suit s'applique aux variables rencontrées :
Z est sélectionné pour chaque occurrence, de manière identique ou différente, parmi N et CR¹, ou est C, où un groupe Z est égal C précisément lorsqu'un groupe Y lui est lié;
Y est sélectionné pour chaque occurrence, de manière identique ou différente, parmi BR², C(R²)₂, Si(R²)₂, NR², P(O)R², O, S, SO, SO₂;
n est pour chaque occurrence, de manière identique ou différente, 0 ou 1 ;
R¹, R² sont sélectionnés pour chaque occurrence, de manière identique ou différente, parmi H, D, F, CN, Si(R³)₃, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, et les systèmes de cycle aromatique qui comportent de 6 à 40 atomes de cycle aromatique ; où deux radicaux R¹ et/ou R² ou plus peuvent être liés l'un à l'autre ou les uns aux autres et peuvent former un cycle ; où lesdits groupes alkyle, alcoxy, alkényle et alkynyle et lesdits systèmes de cycle aromatique peuvent dans chaque cas être substitués par un radical ou par plusieurs radicaux R³; où un cycle indole, lequel peut à son tour être substitué par des radicaux R³, peut dans chaque cas être condensé sur un ou plusieurs des cycles à six éléments dans les formules (I) à (IV) ;
R³ est sélectionné pour chaque occurrence, de manière identique ou différente, parmi H, D, F, CN, Si(R⁴)₃, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, et les systèmes de cycle aromatique qui comportent de 6 à 40 atomes de cycle aromatique ; où deux radicaux R³ ou plus peuvent être liés l'un à l'autre ou les uns aux autres et peuvent former un cycle ; où lesdits groupes alkyle, alcoxy, alkényle et alkynyle et lesdits système de cycle aromatique peuvent dans chaque cas être substitués par un radical ou par plusieurs radicaux R⁴;
R⁴ est sélectionné pour chaque occurrence, de manière identique ou différente, parmi H, D, F, CN, les groupes alkyle ou alcoxy qui comportent de 1 à 20 atome(s) de C, les groupes alkényle ou alkynyle qui comportent de 2 à 20 atomes de C, et les systèmes de cycle aromatique qui comportent de 6 à 40 atomes de cycle aromatique ; où deux radicaux R⁴ ou plus peuvent être liés l'un à l'autre ou les uns aux autres et peuvent former un cycle ; et où lesdits groupes alkyle, alcoxy, alkényle et alkynyle et lesdits systèmes de cycle aromatique peuvent être substitués par F ou par CN ;
**caractérisé en ce qu'**au moins un groupe Z par formule sélectionnée parmi les formules (I), (II), (III) et (IV) est égal à CR¹;
et **caractérisé en outre en ce que** précisément un groupe R¹ par formule (I) a été remplacé par un groupe de la formule (A) ou par un groupe de la formule (H), 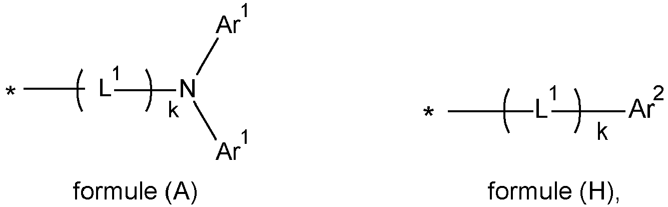
et **caractérisé en outre en ce que** précisément un groupe R¹ par formule sélectionnée parmi les formules (II), (III) et (IV) a été remplacé par un groupe de la formule (A),
où :
L¹ est pour chaque occurrence, de manière identique ou différente, un système de cycle aromatique qui comporte de 6 à 24 atomes de cycle aromatique ou un système de cycle hétéroaromatique qui comporte de 5 à 24 atomes de cycle aromatique, lequel peut dans chaque cas être substitué par un radical ou par plusieurs radicaux R⁵;
k est égal à 0, 1, 2 ou 3 ;
Ar¹ est pour chaque occurrence, de manière identique ou différente, un système de cycle aromatique qui comporte de 6 à 24 atomes de cycle aromatique ou un système de cycle hétéroaromatique qui comporte de 5 à 24 atomes de cycle aromatique, lequel peut être substitué par un radical ou par plusieurs radicaux R⁵;
Ar² est un système de cycle aromatique qui comporte de 5 à 24 atomes de cycle aromatique, lequel peut être substitué par un radical ou par plusieurs radicaux R⁵;
R⁵ est sélectionné pour chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les groupes alkényle ou alkynyle qui comportent de 2 à 20 atomes de C, les systèmes de cycle aromatique qui comportent de 6 à 40 atomes de cycle aromatique, et les systèmes de cycle hétéroaromatique qui comportent de 5 à 40 atomes de cycle aromatique ; où deux radicaux R⁵ ou plus peuvent être liés l'un à l'autre ou les uns aux autres et peuvent former un cycle ; où lesdits groupes alkyle, alcoxy, alkényle et alkynyle et lesdits systèmes de cycle aromatique et lesdits systèmes de cycle hétéroaromatique peuvent dans chaque cas être substitués par un radical ou par plusieurs radicaux R⁶; et où un ou plusieurs groupe(s) CH₂ dans lesdits groupes alkyle, alcoxy, alkényle et alkynyle peut/peuvent être remplacé(s) par -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO ou SO₂;
R⁶ est sélectionné pour chaque occurrence, de manière identique ou différente, parmi H, D, F, CN, les groupes alkyle ou alcoxy qui comportent de 1 à 20 atome(s) de C, les groupes alkényle ou alkynyle qui comportent de 2 à 20 atomes de C, et les systèmes de cycle aromatique qui comportent de 6 à 40 atomes de cycle aromatique et les systèmes de cycle hétéroaromatique qui comportent de 5 à 40 atomes de cycle aromatique ; où deux radicaux R⁶ ou plus peuvent être liés l'un à l'autre ou les uns aux autres et peuvent former un cycle ; et où lesdits groupes alkyle, alcoxy, alkényle et alkynyle, lesdits systèmes de cycle aromatique et lesdits systèmes de cycle hétéroaromatique peuvent être substitués par F ou par CN ;
où le groupe de la formule (A) ou le groupe de la formule (H) est lié via la liaison repérée au moyen de *.

2. Composé selon la revendication 1, **caractérisé en ce que** la formule (I) correspond à l'une des formules (I-H-1) à (I-H-7) et (I-A-1) à (I-A-7)
| | |
|---|---|
| 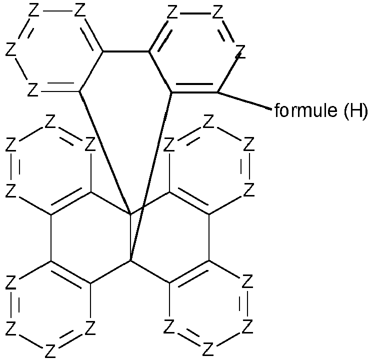 | 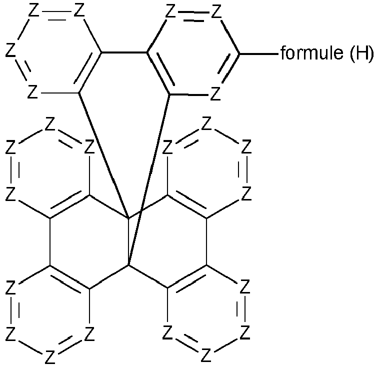 |
| formule (I-H-1) | formule (I-H-2) |
| 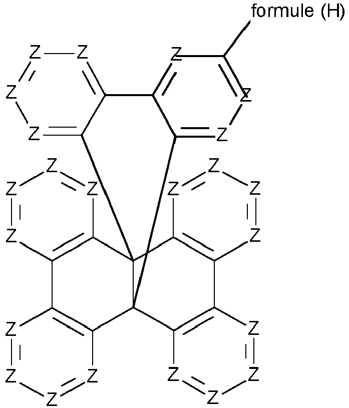 | 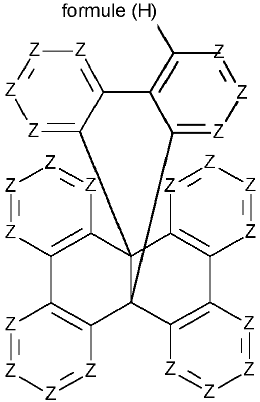 |
| formule (I-H-3) | formule (I-H-4) |
| 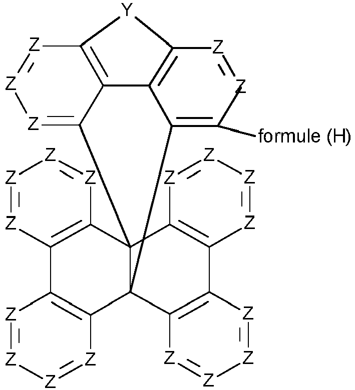 | 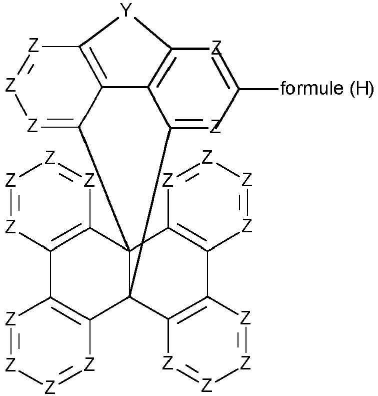 |
| formule (I-H-5) | formule (I-H-6) |
| 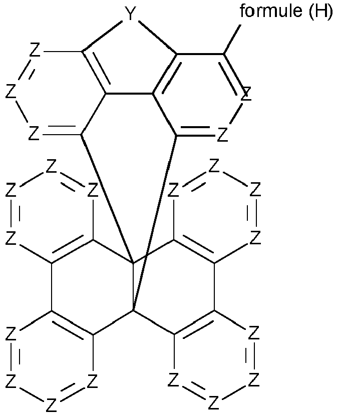 | 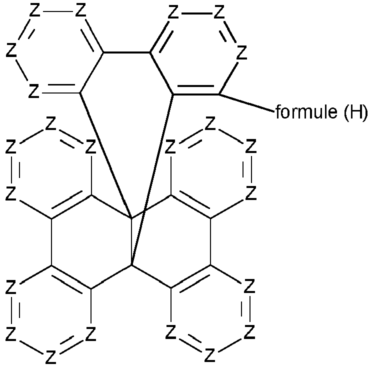 |
| formule (I-H-7) | formule (I-A-1) |
| 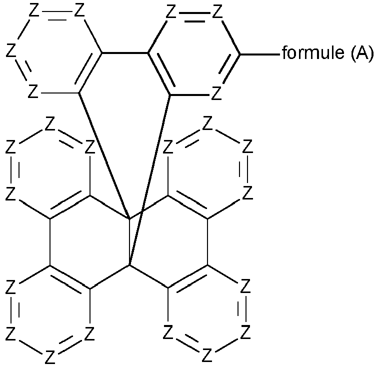 | 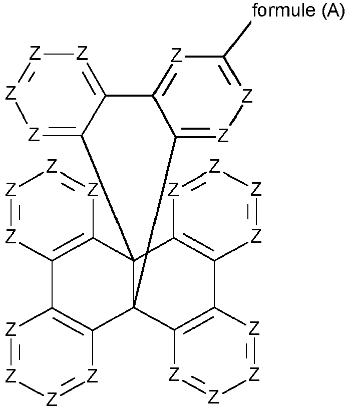 |
| formule (I-A-2) | formule (I-A-3) |
| 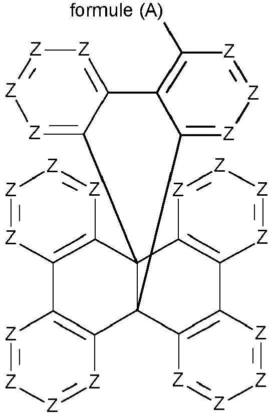 | 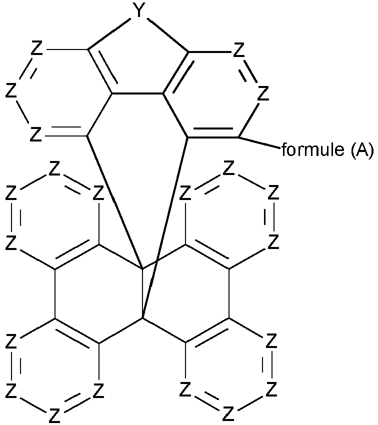 |
| formule (I-A-4) | formule (I-A-5) |
| 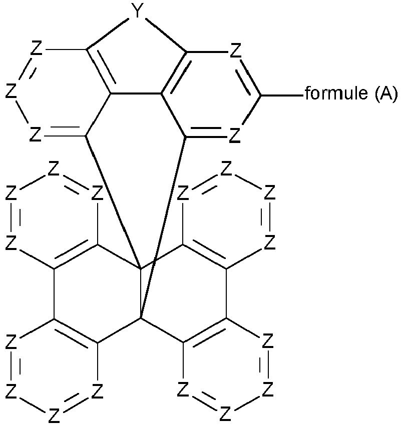 | 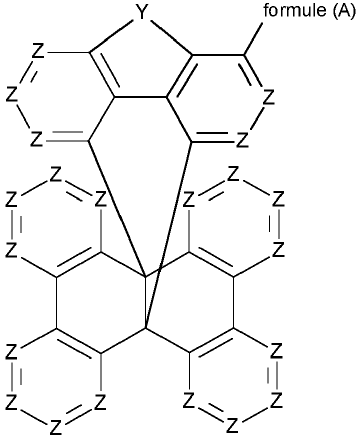 |
| formule (I-A-6) | formule (I-A-7), |
et/ou **en ce que** la formule (II) correspond à l'une des formules (II-A-1) à (II-A-3)
| | |
|---|---|
| 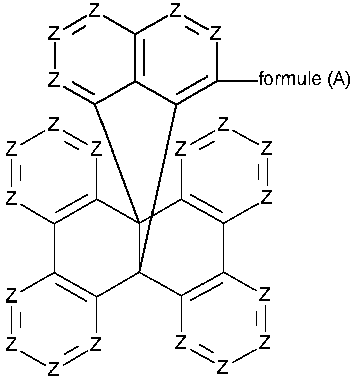 | 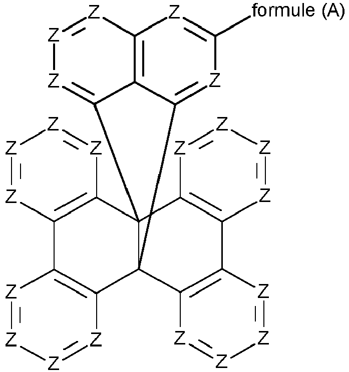 |
| formule (II-A-1) | formule (II-A-2) |
| 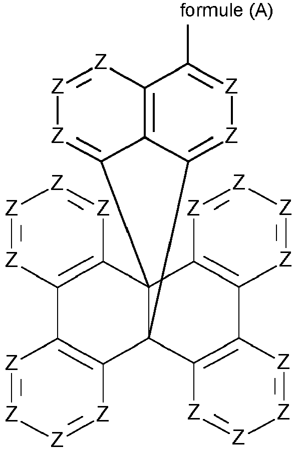 | |
| formule (II-A-3), | |
et/ou **en ce que** la formule (III) correspond à l'une des formules (III-A-1) à (III-A-3)
| | |
|---|---|
| 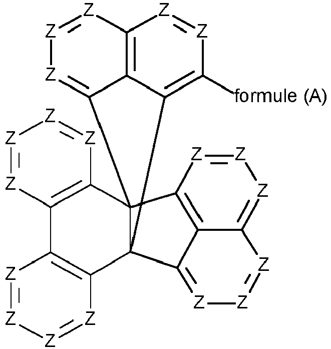 | 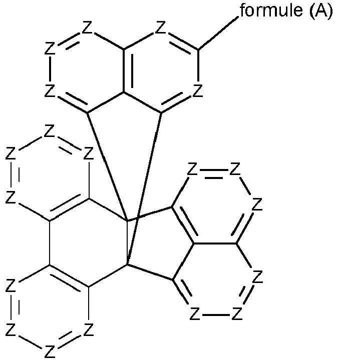 |
| formule (III-A-1) | formule (III-A-2) |
| 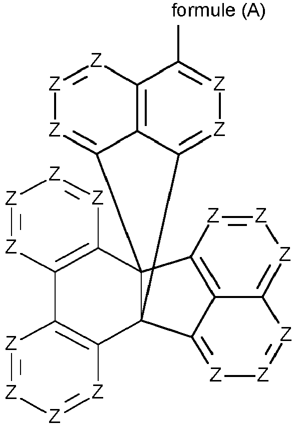 | |
| formule (III-A-3), | |
et/ou **en ce que** la formule (IV) correspond à l'une des formules (IV-A-1) à (IV-A-3)
| | |
|---|---|
| 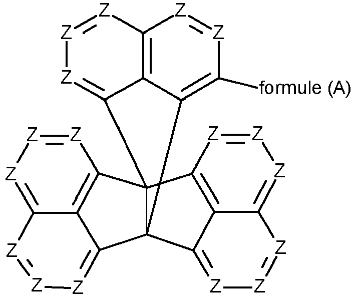 | 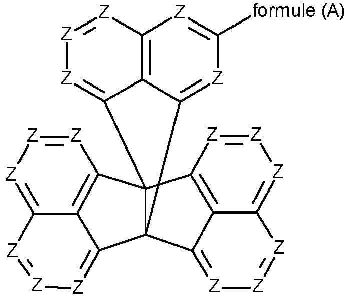 |
| formule (IV-A-1) | formule (IV-A-2) |
| 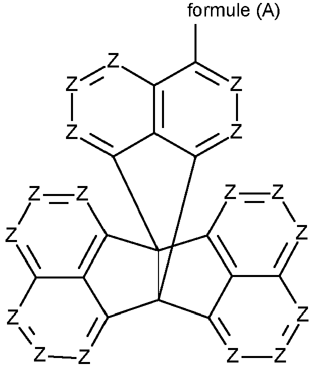 | |
| formule (IV-A-3), | |
dans lesquelles les groupes qui apparaissent sont définis tel que selon la revendication 1, et où "formule (A)" est un groupe de la formule (A) tel que défini selon la revendication 1, et où "formule (H)" est un groupe de la formule (H) tel que défini selon la revendication 1.

3. Composé selon la revendication 1 ou 2, **caractérisé en ce que** Z est égal à CR¹ ou à C, où Z est égal à C précisément lorsqu'un groupe Y lui est lié.

4. Composé selon une ou plusieurs des revendications 1 à 3, **caractérisé en ce que** Y est sélectionné pour chaque occurrence, de manière identique ou différente, parmi O et NR².

5. Composé selon une ou plusieurs des revendications 1 à 4, **caractérisé en ce que** la somme des indices n dans un composé de la formule (II) est égale à 1 ou à 0, et/ou **en ce que** la somme des indices n dans un composé de la formule (III) est égale à 0.

6. Composé selon une ou plusieurs des revendications 1 à 5, **caractérisé en ce que** l'indice n est égal à 0.

7. Composé selon une ou plusieurs des revendications 1 à 6, **caractérisé en ce que** le groupe Y est lié au cycle à six éléments qui est porteur d'un groupe de la formule (A) ou (H).

8. Composé selon une ou plusieurs des revendications 1 à 7, **caractérisé en ce que** R¹ est sélectionné pour chaque occurrence, de manière identique ou différente, parmi H, D, les groupes alkyle en chaîne droite ou ramifiés qui comportent jusqu'à 12 atomes de C, et les systèmes de cycle aromatique qui comportent de 6 à 24 atomes de cycle aromatique, où lesdits groupes alkyle et lesdits systèmes de cycle aromatique peuvent dans chaque cas être substitués par un radical ou par plusieurs radicaux R³.

9. Composé selon une ou plusieurs des revendications 1 à 8, **caractérisé en ce que** R² est sélectionné parmi les groupes alkyle en chaîne droite ou ramifiés qui comportent jusqu'à 12 atomes de C, et les systèmes de cycle aromatique qui comportent de 6 à 24 atomes de cycle aromatique, où deux radicaux R² qui sont liés au même atome de carbone d'un groupe C(R²)₂ et qui sont des groupes alkyle ou des groupes aryle peuvent être connectés l'un à l'autre pour former un groupe alkyle cyclique ou pour former un groupe fluorène.

10. Composé selon une ou plusieurs des revendications 1 à 9, **caractérisé en ce que** R³ est sélectionné pour chaque occurrence, de manière identique ou différente, parmi H, D, F, CN, les groupes alkyle en chaîne droite qui comportent de 1 à 12 atome(s) de C, les groupes alkyle ramifiés ou cycliques qui comportent de 3 à 12 atomes de C, et les systèmes de cycle aromatique qui comportent de 6 à 24 atomes de cycle aromatique ; où deux radicaux R³ ou plus peuvent être liés l'un à l'autre ou les uns aux autres et peuvent former un cycle ; et où lesdits groupes alkyle et lesdits systèmes de cycle aromatique peuvent dans chaque cas être substitués par un radical ou par plusieurs radicaux R⁴.

11. Composé selon une ou plusieurs des revendications 1 à 10, **caractérisé en ce que** R⁴ est sélectionné pour chaque occurrence, de manière identique ou différente, parmi H, D, F, CN, les groupes alkyle en chaîne droite qui comportent de 1 à 12 atome(s) de C, les groupes alkyle ramifiés ou cycliques qui comportent de 3 à 12 atomes de C, et les systèmes de cycle aromatique qui comportent de 6 à 24 atomes de cycle aromatique ; où deux radicaux R⁴ ou plus peuvent être liés l'un à l'autre ou les uns aux autres et peuvent former un cycle ; et où lesdits groupes alkyle et lesdits systèmes de cycle aromatique peuvent être substitués par F ou par CN.

12. Composé selon une ou plusieurs des revendications 1 à 11, **caractérisé en ce que** précisément un groupe R¹ par formule (I) a été remplacé par un groupe de la formule (A).

13. Composé selon une ou plusieurs des revendications 1 à 12, **caractérisé en ce que** L¹ est un groupe divalent qui est sélectionné parmi phénylène, biphénylène, terphénylène, naphtylène, dibenzofurane, dibenzothiophène, carbazole et fluorène, où le groupe divalent peut être substitué par un radical ou par plusieurs radicaux R⁵.

14. Composé selon une ou plusieurs des revendications 1 à 13, **caractérisé en ce que** Ar¹ est sélectionné pour chaque occurrence, de manière identique ou différente, parmi phényle, biphényle, terphényle, fluorényle, naphtyle, spirobifluorényle, pyridyle, pyrimidyle, triazinyle, dibenzofuranyle, dibenzofuranyle benzo-condensé, dibenzothiophényle, dibenzothiophényle benzo-condensé, carbazolyle et carbazolyle benzo-condensé, et des combinaisons de deux, trois ou quatre de ces groupes, où lesdits groupes peuvent dans chaque cas être substitués par un radical ou par plusieurs radicaux R⁵.

15. Composé selon une ou plusieurs des revendications 1 à 14, **caractérisé en ce que** L¹ est un groupe divalent qui est sélectionné parmi phénylène, biphénylène, terphénylène, naphtylène, dibenzofurane, dibenzothiophène, carbazole et fluorène, où le groupe divalent peut être substitué par un radical ou par plusieurs radicaux R⁵.

16. Composé selon une ou plusieurs des revendications 1 à 15, **caractérisé en ce que** Ar² est sélectionné pour chaque occurrence, de manière identique ou différente, parmi les groupes des formules qui suivent :
| | |
|---|---|
| 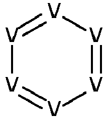 | 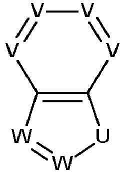 |
| (Ar²-A) | (Ar²-B) |
| 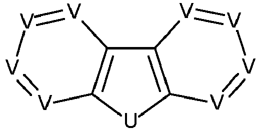 | 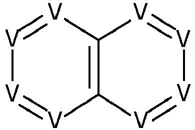 |
| (Ar²-C) | (Ar²-D) |
dans lesquelles les variables qui sont rencontrées sont définies comme suit :
V est pour chaque occurrence, de manière identique ou différente, N ou CR⁵, où, dans les formules (Ar²-A) et (Ar²-D), au moins un groupe V par formule est égal à N ;
W est pour chaque occurrence, de manière identique ou différente, N ou CR⁵;
U est égal à NR⁵;
où un groupe R⁵ par formule a été remplacé par la liaison sur le groupe L¹ ou sur le groupe propellane.

17. Composé selon une ou plusieurs des revendications 1 à 16, **caractérisé en ce que** Ar² est sélectionné parmi pyridine, pyrimidine, pyridazine, pyrazine, triazine, carbazole, imidazole, pyrazole, triazole, benzimidazole, benzimidazoisoquinoline, imidazophénanthridine, benzimidazophénanthridine, bathocuproine, benzimidazobenzimidazole, quinoline, quinazoline, phénanthroline, phénanthridine, diazaphénanthrène et acridine, lequel peut dans chaque cas être substitué par un radical ou par plusieurs radicaux R⁵.

18. Composé selon une ou plusieurs des revendications 1 à 17, **caractérisé en ce que** R⁵ est sélectionné parmi H, D, F, CN, Si(R⁶)₃, N(R⁶)₂, les groupes alkyle ou alcoxy en chaîne droite qui comportent de 1 à 20 atome(s) de C, les groupes alkyle ou alcoxy ramifiés ou cycliques qui comportent de 3 à 20 atomes de C, les systèmes de cycle aromatique qui comportent de 6 à 40 atomes de cycle aromatique et les systèmes de cycle hétéroaromatique qui comportent de 5 à 40 atomes de cycle aromatique ; où lesdits groupes alkyle et alcoxy, lesdits systèmes de cycle aromatique et lesdits systèmes de cycle hétéroaromatique peuvent dans chaque cas être substitués par un radical ou par plusieurs radicaux R⁶; et où un ou plusieurs groupe(s) CH₂ dans lesdits groupes alkyle ou alcoxy peut/ peuvent être remplacé(s) par -C=C-, -R⁶C=CR⁶-, Si(R⁶)₂, C=O, C=NR⁶, -NR⁶-, -O-, -S-, -C(=O)O- ou -C(=O)NR⁶-.

19. Procédé pour la préparation d'un composé selon une ou plusieurs des revendications 1 à 18, **caractérisé en ce que**, en premier lieu, le squelette substitué par alcoxy est préparé, lequel est converti lors d'une étape ultérieure selon un composé réactif, de préférence selon un dérivé de triflate, lequel est converti lors d'une étape ultérieure selon le composé selon une ou plusieurs des revendications 1 à 18 au moyen d'une réaction de couplage catalysée par métal/métaux de transition, de préférence un couplage de Hartwig-Buchwald, un couplage de Suzuki, un couplage de Stille ou un couplage de Negishi.

20. Oligomère, polymère ou dendrimère contenant un ou plusieurs composé(s) selon une ou plusieurs des revendications 1 à 18, où la/les liaison(s) sur le polymère, sur l'oligomère ou sur le dendrimère peut/peuvent être localisée(s) au niveau de n'importe quelles positions souhaitées qui sont substituées par R¹, par R² ou par R⁵.

21. Formulation comprenant un ou plusieurs composé(s) selon une ou plusieurs des revendications 1 à 18, et au moins un solvant.

22. Dispositif électronique, sélectionné parmi le groupe qui est constitué par les circuits intégrés organiques (OIC), les transistors à effet de champ organiques (OFET), les transistors à film mince organiques (OTFT), les transistors à émission de lumière ou électroluminescents organiques (OLET), les cellules solaires organiques (OSC), les détecteurs optiques organiques, les photorécepteurs organiques, les dispositifs à extinction de champ organiques (OFQD), les cellules électrochimiques à émission de lumières ou électroluminescentes organiques (OLEC), les diodes laser organiques (O-laser) et les dispositifs électroluminescents organiques (OLED), contenant au moins un composé selon une ou plusieurs des revendications 1 à 18.

23. Dispositif électroluminescent organique selon la revendication 22, contenant une anode, une cathode et au moins une couche organique, dans lequel le composé est présent dans une couche de transport de trous, en tant que matériau de matrice dans une couche d'émission, ou dans une couche de transport d'électrons.

24. Utilisation d'un composé selon une ou plusieurs des revendications 1 à 18 dans un dispositif électronique qui est sélectionné parmi le groupe qui est constitué par les circuits intégrés organiques (OIC), les transistors à effet de champ organiques (OFET), les transistors à film mince organiques (OTFT), les transistors à émission de lumière ou électroluminescents organiques (OLET), les cellules solaires organiques (OSC), les détecteurs optiques organiques, les photorécepteurs organiques, les dispositifs à extinction de champ organiques (OFQD), les cellules électrochimiques à émission de lumières ou électroluminescentes organiques (OLEC), les diodes laser organiques (O-laser) et les dispositifs électroluminescents organiques (OLED).
